# EUROPEAN PATENT APPLICATION

(11) **EP 4 566 599 A1**
(43) Date of publication of application: **11.06.2025**
(21) Application number: 23850082.1
(22) Date of filing: 01.08.2023
(51) Int. Cl.: A61K 31/352, A23L 33/10, A61P 11/00, A61P 11/02, A61P 11/06, A61P 17/00, A61P 17/04, A61P 37/08, A61P 43/00

(54) **PHARMACEUTICAL COMPOSITION FOR AMELIORATING ALLERGIC DISEASES**

(30) Priority: 02.08.2022 JP 2022123464; 12.12.2022 JP 2022198011
(71) Applicant: Kinki University, Higashiosaka-shi, Osaka 577-8502 (JP)
(72) Inventor: NISHIDA Shozo, Higashiosaka City, Osaka 5778502 (JP); TSUBAKI Masanobu, Higashiosaka City, Osaka 5778502 (JP); TAKEDA Tomoya, Higashiosaka City, Osaka 5778502 (JP); TANABE Genzoh, Higashiosaka City, Osaka 5778502 (JP); TAKASHIMA Katsuki, Higashiosaka City, Osaka 5778502 (JP); MORIKAWA Toshio, Higashiosaka City, Osaka 5778502 (JP)
(74) Representative: Grünecker Patent- und Rechtsanwälte PartG mbB
(86) International application number: PCT/JP2023/028116
(87) International publication number: WO 2024/029528

(57) **Abstract**

Antibody drugs have been proposed to treat allergic diseases, but the route of administration is limited to intravenous route, which is inconvenient for patients. In addition, novel agents that inhibit kinases such as NIK are always needed to develop effective drugs and other products to treat allergic diseases. At least one compound selected from mangiferin 8a (formula (1)), norathyriol (formula (2)), mangiferin (formula (3)), 1,3,5,6-tetrahydroxyxanthone (formula (4)), xanthohydrol (formula (5)), α-mangostin (formula (6)), and γ-mangostin (formula (7)) having a xanthone structure can improve allergic diseases and can be effective when taken orally.

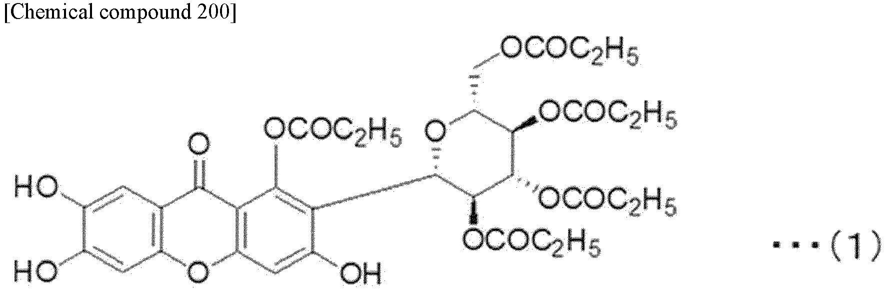

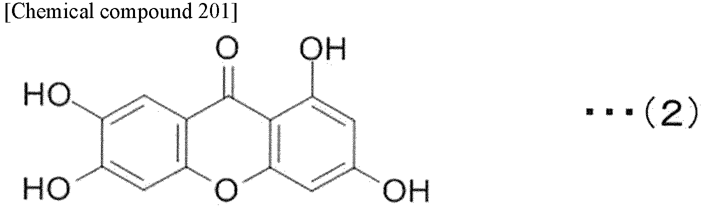

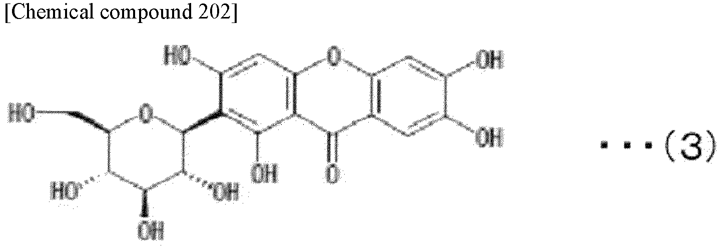

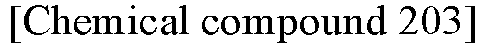

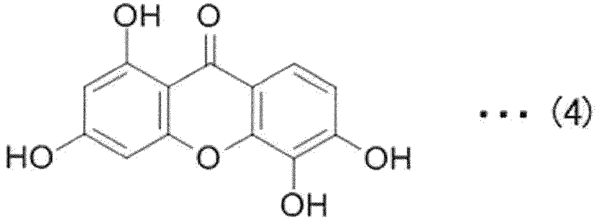

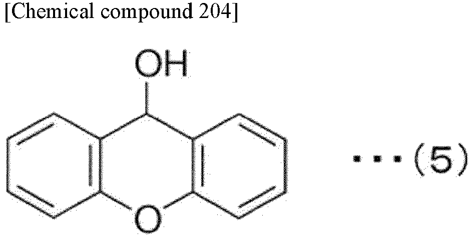

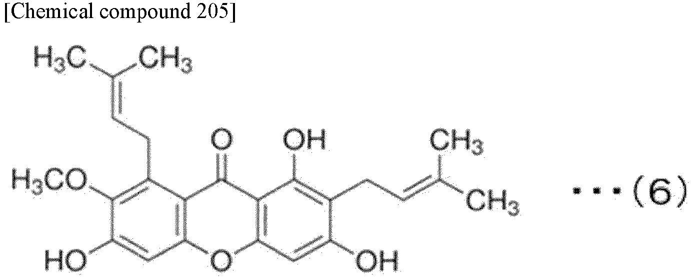

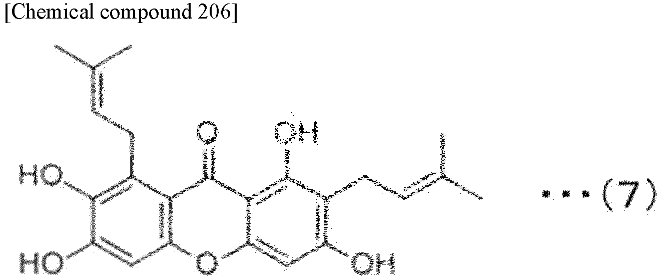

## Description

### [Technical Field]

This invention relates to compounds that inhibit NF-κB-induced kinase (also known as NIK-MAP3K14). These compounds are useful in the treatment and/or prevention of allergic diseases such as bronchial asthma, atopic dermatitis, allergic rhinitis such as pollinosis, allergic conjunctivitis, and food allergy.

The present invention also relates to compositions, pharmaceutical compositions, and processed foods for the prevention and treatment of allergic diseases associated with activation of immune cells that are B cells, Th1 cells, Th2 cells, Th17 cells, macrophage, and regulation of Treg cells, responsible for immune suppression, and that are overexpression of cytokines such as B cell activating factor (B AFF), interferon y (IFNy), interleukin 3 (IL-3), IL-4, IL-5, IL-6, IL-13, IL-17, tumor necrosis factor α (TNFα), and others.

NF-κB (Nuclear factor kappa B) is a transcription factor that regulates the expression of various genes involved in immune response, cell proliferation, apoptosis and carcinogenesis. The NF-κB is composed of five members: NF-κB p65 (p65), RelB, c-Rel, NF-κB1 (which are present in the form of both precursor p105 and cleavage type p50) and NF -kappaB2 (which are present in the form of both precursor p100 and cleavage type p52).

Mainly, NF-κB1 (cleavage type p50; NF-κB p50) and p65 form heterodimers, and NF-κB2 (cleavage type p52; NF-κB p52) and RelB form heterodimers. The activation of these NF-κB heterodimers is also performed by a signal transduction pathway that is strictly regulated by continuous events including phosphorylation reactions and proteolysis. These signaling pathway is classified into two pathways: the classical pathway (canonical pathway) and the non-classical pathway (non-canonical pathway).

NIK is a serine/threonine kinase and plays a role in both pathways; NIK is essential in non-canonical signaling pathways and phosphorylates IKKα to partially degrade NF-κB p100 and release NF-κB p52. NF-κB p52 forms a heterodimer with RelB, which then translocates into the nucleus and causes gene expression. In addition, the canonical pathway forms a heterodimer of p65 and NF-κB p50 by activating the IKKα, IKKβ, and IKKγ complexes. This heterodimer translocation into the nucleus regulates gene expression.

NIK is activated by ligands such as B cell activating factor (BAFF), CD40 ligand, and TNFα. It is known that NIK is important for the activation of signal transduction pathways by these ligands. Because of its important role, NIK expression is tightly regulated.

Under normal unstimulated conditions, the amount of NIK protein in cells is low because NIK is degraded by the interaction of NIK with the ubiquitin ligase, TNF receptor-associated factor (TRAF).

When the non-canonical pathway is stimulated by the ligand, the TRAF-NIK complex is thought to be dissociated by the activated receptor, thereby increasing NIK concentrations. (Non-Patent Literature 1).

BAFF is produced and secreted by T cells, monocytes/macrophages, dendritic cells, etc., and is known to regulate B cell differentiation, activation, survival, etc. via three types of receptors on B cells (Non-Patent Literature 2).

Known receptors for BAFF are BAFF-R (BAFF-Receptor), TACI (Transmembrane activator and calcium modulator and cyclophilin ligand interactor) and BCMA (B cell maturation antigen) are known as receptors for BAFF.

BAFF-R and BCMA are expressed mainly on B cells, while TACI is expressed on B cells and activated T cells. In addition, BAFF-R is expressed on T cells and macrophages. Interaction of BAFF with BAFF-R activates NIK-mediated signaling pathways essential for B cell formation and maintenance, which in turn produce immunoglobulin in response to invasion by xenobiotics. BAFF also induces T cell differentiation and activation via BAFF-R. Furthermore, BAFF promotes the maintenance and activation of macrophages.

The appropriate level of BAFF in a patient can help maintain normal levels of immunity, but low expression can result in immunodeficiency, and overexpression can produce abnormally high antibody production and an excessive immune response. Furthermore, when patients exhibit allergic disease, activation of B cells, Th1 cells, Th2 cells, Th17 cells, macrophages, suppression of Treg cells, and BAFF, IFNγ, IL-3, IL-4, IL-5, IL-6, IL-13, IL-17, TNFα, and other cytokines are overexpressed.

When a patient exhibits allergies, an exaggerated immune response to foreign antigens occurs, producing antibodies such as IgE. Allergic diseases in this description include bronchial asthma, atopic dermatitis, pollinosis, allergic rhinitis, food allergy, metal allergy, drug allergy, anaphylaxis, urticaria, allergic conjunctivitis, allergic bronchitis, contact dermatitis, and others.

Allergic diseases have been shown to overexpress BAFF in the bodies and increases in BAFF expression have been reported as the disease progresses. Increased BAFF activates intracellular NIK; NIK activation promotes nuclear translocation of NF-κB, leading to increased differentiation of B cells into plasma cells, which in turn promotes antibody production from these differentiated plasma cells, leading to allergic disease progression.

Therefore, it is important to inhibit B-cell differentiation by suppressing BAFF-induced NIK activation and NF-κB activation, and to reduce the expression or secretion of BAFF in order to treat patients with these diseases. The term "therapy or treatment" here includes not only the recovery of a disease state, but also the effect of controlling or delaying the progression of a disease state.

Furthermore, in allergic diseases, NF-κB activation by NIK and other activation causes activation of B cells, Th1 cells, Th2 cells, Th17 cells, macrophages, suppression of Treg cells, and overexpression of BAFF, IFNγ, IL-3, IL -4, IL-5, IL-6, IL-13, IL-17, TNFα, and other cytokines are overexpressed. Therefore, inhibition of NF-κB activation by activation of NIK, etc. is important in therapy because it inhibits activation of B cells, Th1 cells, Th2 cells, Th17 cells, and macrophages, as well as BAFF, IFNγ, IL-3, IL- 4, IL-5, IL-6, IL-13, IL-17, TNFα and other cytokine expression.

It is also important in therapy to promote Treg cell activation by inhibiting NF-κB activation through NIK activation or other means. Again, therapy includes not only the recovery of a disease state, but also the effect of inhibiting or delaying the progression of the disease state.

It has been shown that serum BAFF concentrations are increased in bronchial asthma patients compared to healthy controls (Non-Patent Literature 3). It has also been reported that in a mouse model of bronchial asthma, increased serum BAFF concentration increases inflammatory cells, and BAFF shRNA treatment improves bronchial asthma symptoms (Non-Patent Literature 4). Furthermore, it has been shown that activation of the non-canonical NF-κB pathway via NIK in lymphocytes at the site of disease is involved in pathogenesis in bronchial asthma patients and in mouse models of bronchial asthma (Non-Patent Literature 5).

Furthermore, in bronchial asthmatics, activation of Th2 cells results in the secretion of cytokines such as IL-4, IL-5, IL-9, and IL-13. IL-4 converts immunoglobulins secreted by B cells from IgG to IgE. IL-5 enhances proliferation and differentiation of eosinophils in the bone marrow and promotes their migration to tissues, activation, and survival; IL-9 promotes mast cell proliferation and induces inflammation; IL-13 induces production of nitric oxide synthase in airway epithelial cells, thereby inducing airway hyperresponsiveness and fibrosis by inducing production of nitric oxide synthase in airway epithelial cells. These have been shown to induce allergic inflammation.

It has also been shown that macrophages take up antigens and produce leukotriene B4, chemokine ligand 2, etc., thereby activating Th2 cells, etc., and that they are activated by IL-4, IL-5, etc. secreted by activated Th2 cells. It has also been reported that non-allergic bronchial asthma is caused by neutrophil activation via IFNy and IL-17 secreted by Th1 and Th17 cells, respectively (Non-Patent Literature 6, Non-Patent Literature 7).

Activation of NF-κB in Th2 cells and macrophages has also been shown to regulate the expression of cytokines such as IL-4, IL-5, and TNFα (Non-Patent Literature 8).

Furthermore, in a mouse model of bronchial asthma, BAFF shRNA has been shown to reduce Th2 cells, suppress the expression of cytokines such as IL-4, and increase Treg cells, thereby improving asthma symptoms (Non-Patent Literature 9).

It has also been shown that BAFF activates Th1 and Th17 cells, and in T cells, BAFF activates NIK via BAFF-R and activates non-canonical and canonical NF-κB pathways (Non-Patent Literature 10, Non-Patent Literature 11).

It has been reported that serum BAFF levels are elevated in patients with atopic dermatitis compared to healthy controls and correlate with disease progression and serum IgE antibody levels (Non-Patent Literature 12). Furthermore, in a mouse model of atopic dermatitis, NF-κB siRNA treatment has been shown to improve symptoms and suppress the expression of inflammatory cytokines such as TNFα (Non-Patent Literature 13).

In addition, activation of Th2 cells in patients with atopic dermatitis results in the secretion of cytokines such as IL-4, IL-5, IL-9, and IL-13. IL-4 converts immunoglobulins from IgG to IgE in B cells, and IL-5 enhances proliferation and differentiation of eosinophils in the bone marrow, their migration into tissues, activation, and survival. These have thus been shown to induce allergic inflammation (Non-Patent Literature 14).

It has also been shown that in a mouse model of atopic dermatitis, macrophages are activated and produce cytokines, etc., which activate Th2 cells, etc., and that macrophages are activated by IL-4, IL-5, etc. secreted by the activation of Th2 cells (Non-Patent Literature 15). Furthermore, activation of NF-κB in Th2 cells and macrophages has been shown to regulate the expression of cytokines such as IL-4, IL-5, and TNFα (Non-Patent Literature 8).

It has also been reported that in Th2 cell-mediated inflammatory diseases such as atopic dermatitis, cytokines such as IL-4 and IL-13 are secreted by Th2 cells due to increased BAFF in the serum, and are involved in disease development (Non-Patent Literature 16, Non-Patent Literature 17).

In patients with allergic diseases, sensitization to house dust such as pollen and mites increases BAFF concentration in bronchoalveolar lavage fluid, as well as increase lymphocyte, neutrophil, eosinophil numbers, and inflammatory cytokine production such as IL-6 and IL-13, and antibody production such as IgE and IgA antibody. Increases in these cell numbers, inflammatory cytokine production, and IgE and IgA antibody production have been reported to correlate with BAFF concentrations (Non-Patent Literature 18).

Furthermore, in a mouse model of pollinosis, increased numbers of Th17 cells, neutrophils, eosinophils, Treg cells, inflammatory cytokines such as TNFα and IL-17, and activated NF-κB in bronchoalveolar lavage fluid (Non-Patent Literature 19).

In patients with allergic rhinitis, BAFF production in serum and at diseased sites is elevated compared to healthy individuals, and has been shown to correlate with disease progression and severity (Non-Patent Literature 20). It has also been reported that in patients with allergic rhinitis, the NF-κB pathway is activated at the diseased site and plays an important role in the pathogenesis of the disease (Non-Patent Literature 21).

Furthermore, in patients with pollinosis and allergic rhinitis, dendritic cells and other cells that have taken up antigens activate Th2 cells via activation of natural killer cells. It has been shown that activated Th2 cells secrete cytokines such as IL-4 and IL-13, which cause B cells to produce IgE antibodies and develop rhinitis (Non-Patent Literature 22).

It has also been shown that the amount of BAFF produced in pollinosis and allergic rhinitis patients correlates with the activation of Th2 cells and the expression of cytokines such as IL-4, IL-5, and IL-13 produced by these cells (Non-Patent Literature 23). It has also been shown that BAFF activates Th17 cells, and in T cells, BAFF activates NIK via BAFF-R and activates the non-canonical NF-κB pathway and the canonical NF-κB pathway (Non-Patent Literature 10, Non-Patent Literature 11).

It has been shown that BAFF concentrations in serum and intestinal lavage fluid are higher in patients with food allergy compared to healthy individuals (Non-Patent Literature 24). In addition, activation of NF-κB at the diseased site was observed in a mouse model of food allergy, and the expression of IgE antibody and inflammatory cytokines such as IL-13, IL-25, and IL-33 increased (Non-Patent Literature 25).

It has also been shown that in food allergy, Th2 cells are increased by antigen presentation from dendritic cells, and that IL-4 and IL-13 expressed by these Th2 cells enhance differentiation into IgE-producing B cells and migration and activation of basophils, and IL 5 and IL-9 promote migration and activation of eosinophils (Non-Patent Literature 26). It has also been reported that BAFF production at the diseased site induces activation and differentiation of B cells, increased IgE production, and activation of Th2 cells and eosinophils (Non-Patent Literature 27).

It has been reported that BAFF production in serum is elevated in animal models of anaphylaxis and that BAFF inhibition prevents anaphylaxis (Non-Patent Literature 28). It has also been shown that activation of NF-κB is important for the expression of inflammatory cytokines and is a factor in causing anaphylaxis (Non-Patent Literature 29).

In anaphylaxis, the expression of cytokines such as IL-4 and IL-5 by Th2 cells leads to their differentiation into IgE-producing B cells. The IgE expressed from these B cells binds to high-affinity IgE receptors on mast cell and basophil cell membranes and promotes degranulation in mast cells and basophils. This degranulation releases histamine, heparin, tryptase, TNFα, cytokines, and other factors that have been shown to increase vascular permeability (Non-Patent Literature 30).

It has also been reported that BAFF production at the diseased site induces activation and differentiation of B cells, increased IgE production, and activation of Th2 cells and eosinophils (Non-Patent Literature 21).

It has been reported that urticaria patients have higher serum BAFF concentrations than healthy individuals and that BAFF concentrations correlate with disease progression (Non-Patent Literature 31). It has also been shown that NF-κB is activated in lymphocytes of urticaria patients (Non-Patent Literature 32).

It has been shown that Th2 and Th17 cells are increased at the diseased site in patients with urticaria, and that there is an increase in Th17 cells and a decrease in Treg cells in the peripheral blood (Non-Patent Literature 33, Non-Patent Literature 34).

It has also been reported that BAFF production at the diseased site induces activation and differentiation of B cells, increased IgE production, and activation of Th2 cells and eosinophils (Non-Patent Literature 21). Furthermore, BAFF has been shown to activate Th1 and Th17 cells, and in T cells, BAFF activates NIK via BAFF-R, activating both the non-canonical and canonical NF-κB pathways (Non-Patent Literature 10, Non-Patent Literature 11).

In patients with vernal catarrh, a severe case of allergic conjunctivitis, BAFF mRNA expression has been shown to be elevated at the diseased site and correlated with disease progression, compared to healthy individuals (Non-Patent Literature 35). It has also been reported that NF-κB is activated in the diseased areas of allergic conjunctivitis model mice, increasing the amount of IgE antibody and inflammatory cytokines such as IL-17 (Non-Patent Literature 36).

It has also been shown that IL-4, IL-5, and IL-17 expressed by Th2 and Th17 cells are increased in the tear fluid of patients with allergic conjunctivitis compared to healthy individuals. Furthermore, in mouse models of allergic conjunctivitis, IL-4, IL-5, IL-13, and IL-17 expression by Th2 and Th17 cells has been found to be increased (Non-Patent Literature 37, Non-Patent Literature 38).

It has also been reported that BAFF production at the diseased site induces activation and differentiation of B cells, increased IgE production, and activation of Th2 cells and eosinophils (Non-Patent Literature 21). Furthermore, BAFF has been shown to activate Th1 and Th17 cells, and in T cells, BAFF activates NIK via BAFF-R, activating both the non-canonical and canonical NF-κB pathways (Non-Patent Literature 10, Non-Patent Literature 11).

In a mouse model of allergic bronchitis, increased BAFF production in serum and at diseased sites has been shown to correlate with IgE antibody levels and the production of inflammatory cytokines such as TNFα and IL-6 (Non-Patent Literature 39). Furthermore, it has been reported that activation of NF-κB in bronchial epithelium increases lymphocyte and neutrophil numbers and inflammatory cytokine production such as IL-6, and is involved in the development of allergic bronchitis (Non-Patent Literature 40). In addition, in a mouse model of allergic bronchitis, Th2 and Th17 cells are increased, and specific IgE antibodies, IL-4, IL-6, IL-13, and IL-17 in serum have been shown to increase (Non-Patent Literature 41).

It has also been reported that BAFF production at the diseased site induces activation and differentiation of B cells, increased IgE production, and activation of Th2 cells and eosinophils (Non-Patent Literature 21). Furthermore, BAFF has been shown to activate Th1 and Th17 cells, and in T cells, BAFF activates NIK via BAFF-R, activating both the non-canonical and canonical NF-κB pathways (Non-Patent Literature 10, Non-Patent Literature 11).

Non-canonical and canonical NF-κB pathway activation via NIK activation has been shown to be important in the pathogenesis of contact dermatitis (Non-Patent Literature 42). In addition, increased expression of IL-4, IL-5, and IL-17 secreted by Th2 and Th17 cells at the diseased site has been observed in a mouse model of contact dermatitis (Non-Patent Literature 43).

It has also been reported that BAFF production at the diseased site induces activation and differentiation of B cells, increased IgE production, and activation of Th2 cells and eosinophils (Non-Patent Literature 21). Furthermore, BAFF has been shown to activate Th17 cells, and in T cells, BAFF activates NIK via BAFF-R, activating both the non-canonical and canonical NF-κB pathways (Non-Patent Literature 10, Non-Patent Literature 11). Increased IgE production is also observed for metal and drug allergies (Non-Patent Literature 44, Non-Patent Literature 45).

TNFα is secreted in inflammatory diseases and other inflammatory responses. In colon epithelial cells and mouse fibroblasts, TNFα stimulation promotes nuclear migration of NF-κB p52 and RelB heterodimers via activation of the non-canonical NF-κB pathway and induces inflammation. TNFα increases the cytoplasmic levels of NIK by enhancing the degradation of TRAF, thereby activating NIK (Non-Patent Literature 46).

As described above, it is already common scientific knowledge that allergic diseases are caused by overexpression of BAFF and activation of NF-κB by NIK, etc. Therefore, pharmaceuticals and other products that can inhibit NIK activation and NF-κB activation by BAFF have therapeutic effects on allergic diseases in which overexpression of BAFF and abnormal activation of NF-κB by NIK and other factors are identified.

Furthermore, studies have shown that NF-κB regulates the expression of many genes related to inflammation, and NF-κB signaling was found to be chronically activated in many inflammatory diseases, including inflammatory bowel disease and sepsis. Thus, compounds that can inhibit NIK and thereby attenuate the non-canonical NF-κB signaling pathway have therapeutic effects on diseases and disorders in which excessive activation of non-canonical NF-κB signaling is identified.

The following patent literature is cited with regard to inhibition of NF-κB activity. Patent Literature 1 discloses an antibody that specifically binds to NF-κB-induced kinase (NIK)/MAP3K14 or a specific portion thereof. This antibody is said to act as an immunomodulatory molecule.

Patent Literature 2 also discloses 3-(1H-pyrazol-4-yl)-1H-pyrrolo[2,3-c] pyridine derivatives as NIK inhibitors useful in the treatment of diseases such as cancer, inflammatory diseases, metabolic disorders, and autoimmune diseases.

In addition, Patent Literature 3 discloses a substance of formula (9) as a therapeutic agent for diseases caused by immune dysfunction.

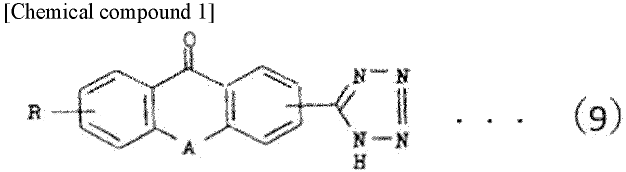

In the formula, R represents hydrogen or a lower alkoxy group, and A represents oxygen or a sulfonyl group. In addition, Patent Document 4 describes that mangiferin has anti-inflammatory effects against rheumatism and other diseases.

### [Citation List]

### [Patent Literatures]

[Patent Literature 1]=Japan Patent Application Laid-Open No. 2007-537708
[Patent Literature 2] Japan Patent Application Laid-Open No. 2016-531858
[Patent Literature 3] Japan Patent Application Laid-Open No. S57-16821
[Patent Literature 4] Japan Patent Application Laid-Open No. 2009-023935

### [Non-Patent Literature]

[Non-Patent Literature 1] Thu YM, Richmond A.: Cytokine Growth Factor Rev. 2010, 21, 213-226.
[Non-Patent Literature 2] Moore PA, Belvedere O, Orr A, Pieri K, LaFleur DW, Feng P, Soppet D, Charters M, Gentz R, Parmelee D, Li Y, Galperina O, Giri J, Roschke V, Nardelli B, Carrell J, Sosnovtseva S, Greenfield W. Carrell J, Sosnovtseva S, Greenfield W, Ruben SM, Olsen HS, Fikes J, Hilbert DM.: Science. 1999, 285, 260-263.
[Non-Patent Literature 3] Alturaiki W, Mubarak A, Mir SA, Afridi A, Premanathan M, Mickymaray S, Vijayakumar R, Alsagaby SA, Almalki SG, Alghofaili F, Alnemare AK, Flanagan BF.: Saudi J Biol Sci. 2021, 28, 7455-7459.
[Non-Patent Literature 4] Tang X, Tong X, An Y.: Life Sci. 2020, 241, 117172.
[Non-Patent Literature 5] Hurrell BP, Galle-Treger L, Jahani PS, Howard E, Helou DG, Banie H, Soroosh P, Akbari O.: Cell Rep. 2019, 29, 4509-4524.e5.
[Non-Patent Literature 6] Chiu CJ, Huang MT.: Int J Mol Sci. 2021, 22, 4528.
[Non-Patent Literature 7] Rodriguez-Coira J, Villaseñor A, Izquierdo E, Huang M, Barker-Tejeda TC, Radzikowska U, Sokolowska M, Barber D.: Front Immunol. 2021, 12, 692004.
[Non-Patent Literature 8] Panday A, Inda ME, Bagam P, Sahoo MK, Osorio D, Batra S.: Arch Immunol Ther Exp. 2016, 64, 463-483.
[Non-Patent Literature 9] Zhou B, Kermany MH, Glickstein J, Cai Q, Cai C, Zhou Y, Nair U, Kim JW, Kim P, Liu W, Kanangat S, Yoo TJ.: Clin Immunol. 2011, 138, 222-230.
[Non-Patent Literature 10] Lied GA, Berstad A.: Scand J Immunol. 2011, 73, 1-7.
[Non-Patent Literature 11] Gardam S, Brink R.: Front Immunol. 2014, 4, 509.
[Non-Patent Literature 12] Jee HM, Kim KW, Hong JY, Sohn MH, Kim KE.: Clin Exp Dermatol. 2010, 35, 593-598.
[Non-Patent Literature 13] Kanazawa T, Hamasaki T, Endo T, Tamano K, Sogabe K, Seta Y, Ohgi T, Okada H.: Int J Pharm. 2015, 489, 261-267.
[Non-Patent Literature 14] Berker M, Frank LJ, Geßner AL, Grassl N, Holtermann AV, Höppner S, Kraef C, Leclaire MD, Maier P, Messerer DA, Möhrmann L, Nieke JP, Schoch D, Soll D, Woopen CM.: Clin Immunol. 2017, 174, 73-83.
[Non-Patent Literature 15] Virgens AR, Goes HFO, de Carvalho GC, Pietrobon AJ, C C Branco AC, Ramos YAL, Pereira NV, Orfali RL, Aoki V, da Silva LFF, Sotto MN, Dos Reis VMS, Sato MN.: Exp Dermatol. 2021,doi: 10.1111/exd. 14442
[Non-Patent Literature 16] Wang W, Sung N, Gilman-Sachs A, Kwak-Kim J.: Front Immunol. 2020, 11, 2025.
[Non-Patent Literature 17] Kido M, Tanaka J, Aoki N, Iwamoto S, Nishiura H, Chiba T, Watanabe N.: Infect Immun. 2010, 78, 108-114.
[Non-Patent Literature 18] Kato A, Xiao H, Chustz RT, Liu MC, Schleimer RP.: J Allergy Clin Immunol. 2009, 123, 369-375.
[Non-Patent Literature 19] Chen Y, Han L, Zhou Y, Yang L, Guo YS.: Int Arch Allergy Immunol. 2020, 181, 342-352.
[Non-Patent Literature 20] Liu W, Sun C, Luo X, Han M, Zhou L, Li J, Wang J, Li Y, Luo R, Li H.: Int J Pediatr Otorhinolaryngol. 2014, 78, 2156-2160.
[Non-Patent Literature 21] Wang SZ, Ma FM, Zhao JD.: Eur Arch Otorhinolaryngol. 2013, 270, 1329-1334.
[Non-Patent Literature 22] Dahl Å.: Front Immunol. 2018, 9, 2816.
[Non-Patent Literature 23] Luo R, Liu W, Wang J, Chen Y, Sun C, Zhou L, Li Y, Deng L.: Eur J Pediatr. 2014, 173, 1033-1040.
[Non-Patent Literature 24] Lied GA, Lillestol K, Valeur J, Berstad A.: Aliment Pharmacol Ther. 2010, 32, 66-73.
[Non-Patent Literature 25] Yu Y, Li J, Liu C.: Biochem Pharmacol. 2022, 195, 114867.
[Non-Patent Literature 26] SaidovaA, Hershkop AM, Ponce M, Eiwegger T.: Arch Immunol Ther Exp. 2018, 66, 161-170.
[Non-Patent Literature 27] Wang G, Li M, Zheng J, Zhan J, Zheng H, Li R, Wei X.: Int Immunopharmacol. 2022, 104, 108515.
[Non-Patent Literature 28] Doerfler PA, Nayak S, Herzog RW, Morel L, Byrne BJ.: Clin Immunol. 2015, 158, 140-147.
[Non-Patent Literature 29] Alfano DN, Klei LR, Klei HB, Trotta M, Gough PJ, Foley KP, Bertin J, Sumpter TL, Lucas PC, McAllister-Lucas LM.: J Immunol. 2020, 204, 2337-2348.
[Non-Patent Literature 30] Nguyen SMT, Rupprecht CP, Haque A, Pattanaik D, Yusin J, Krishnaswamy G.: Int J Mol Sci. 2021, 22, 7785.
[Non-Patent Literature 31] Kessel A, Yaacoby-Bianu K, Vadasz Z, Peri R, Halazs K, Toubi E.: Hum Immunol. 2012, 73, 620-622.
[Non-Patent Literature 32] Kessel A, Bishara R, Amital A, Bamberger E, Sabo E, Grushko G, Toubi E.: Clin Exp Allergy. 2005, 35, 221-225.
[Non-Patent Literature 33] Moy AP, Murali M, Nazarian RM.: J Cutan Pathol. 2016, 43, 372-378.
[Non-Patent Literature 34] Yu Q, Lin W, Zhang J, Peng L, Kong Q, Zhong R, Lan Y, Xiao M, Chen M.: Medicine. 2020, 99, e22014.
[Non-Patent Literature 35] Yoshida K, Shoji J, Ishimori A, Nakashima M, Inada N, Sawa M.: Nippon Ganka Gakkai Zasshi. 2014, 118, 368-377.
[Non-Patent Literature 36] Bai MT, Li Y, Hu ZL.: Int J Ophthalmol. 2021, 14, 955-964.
[Non-Patent Literature 37] Yan A, Luo G, Zhou Z, Hang W, Qin D.: Allergol Immunopathol. 2018, 46, 144-148.
[Non-Patent Literature 38] Chen X, Deng R, Chi W, Hua X, Lu F, Bian F, Gao N, Li Z, Pflugfelder SC, de Paiva CS, Li DQ.: Allergy. 2019, 74, 910-921.
[Non-Patent Literature 39] Liang ZQ, Tu PC, Ji JJ, Xing QQ, Zhao X.: Biomed Pharmacother. 2020, 132, 110801.
[Non-Patent Literature 40] Pantano C, Ather JL, Alcorn JF, Poynter ME, Brown AL, Guala AS, Beuschel SL, Allen GB, Whittaker LA, Bevelander M, Irvin CG, Janssen-Heininger YM.: Am J Respir Crit Care Med. 2008, 177, 959-969.
[Non-Patent Literature 41] Cheng FL, An YF, Xue JM, Wang YJ, Ding XW, Zhang YT, Zhao CQ.: Int Forum Allergy Rhinol. 2021,doi: 10.1002/alr.22914.
[Non-Patent Literature 42] Piao W, Xiong Y, Famulski K, Brinkman CC, Li L, Toney N, Wagner C, Saxena V, Simon T, Bromberg JS.: Nat Commun. 2018, 9, 3020.
[Non-Patent Literature 43] Piao CH, Fan YJ, Nguyen TV, Song CH, Jeong HJ, Chai OH.: J Dermatolog Treat. 2020,1-8.=
[Non-Patent Literature 44] Katherine A,Aleksandr B,Jenny R.:J Immnotoxicology.2019,16,1, 87-124.
[Non-Patent Literature 45] Brian A.:Antibodies.2014,3,56-91.
[Non-Patent Literature 46] Bhattacharyya S, Dudeja PK, Tobacman JK.: J Biol Chem. 2010, 285, 39511-39522.
[Non-Patent Literature 47] Hu L, Hu H, Wu W, Chai X, Luo J, Wu Q.: Bioorg Med Chem Lett. 2011, 21, 4013-4015.

### [Summary of Invention]

### [Technical Problem]

As shown in the patent literature, antibody preparations are mainly used as agents that inhibit cytokines expressed by immune cells, but with the exception of mangiferin in Patent Literature 4, the dosing route is intravenous administration, which imposes a heavy burden on patients. Therefore, the purpose of this invention is to provide a therapeutic agent that inhibits NIK by oral administration and improves allergic diseases, as well as compositions for improvement.

In addition, although mangiferin of the patent literature 4 is effective by oral administration, the required intake is large, and even by oral administration, it could not be easily ingested. Therefore, a more effective or active therapeutic agent and composition for improvement were sought.

Furthermore, new or improved forms of existing drugs that inhibit kinases such as NIK are always needed to develop effective drugs and other products for the treatment of allergic diseases.

### [Solution to Problem]

The inventors searched for compounds that inhibit NIK to solve the above problem and found that a certain compound with a xanthone structure has NIK inhibitory activity, and actually cured mice that had developed allergic rhinitis such as bronchial asthma, atopic dermatitis, and pollinosis. Furthermore, we found that this compound inhibited BAFF-induced activation of NIK, NF-κBp52 and NF-κBp65, and also inhibited BAFF-induced differentiation of B cells into plasmablasts and plasma cells. The compound was also found to induce the dedifferentiation of plasma cells into immature B cells, leading to the completion of the present invention.

In other words, the pharmaceutical compositions for the improvement of allergic diseases are characterized in that they contain at least one compound selected from the following mangiferin, mangiferin 8a, norathyriol, 1,3,5,6-tetrahydroxyxanthone, xanthohydrol, α-mangostin, γ-mangostin, and at least one compound selected from the following.

### [Advantageous Effects of Invention]

The pharmaceutical compositions for improving allergic diseases can improve allergic diseases, for example, dramatically curing symptoms of allergic rhinitis such as bronchial asthma, atopic dermatitis, and pollinosis. Furthermore, the administration is effective orally, and the burden on the patient is very light when applied to humans.

It is also believed that allergic diseases are caused by overexpression of BAFF and activation of the NF-κB pathway, which leads to activation of B cells and T cells and production of antibodies such as IgE. In other words, allergic diseases have the same cause of pathogenesis, and many allergic diseases other than the allergic diseases listed above may share the same cause. Therefore, the pharmaceutical composition for improvement of allergic diseases according to the present invention that suppresses NIK activation is considered to have an improvement effect not only on allergic rhinitis such as bronchial asthma, atopic dermatitis, and pollinosis as shown in the examples, but also on all allergic diseases accompanied by BAFF overexpression and NF-κB pathway activation.

### [Brief Description of Drawing]

[Figure 1] The graph shows the time course of auricular swelling in mice with MC903 applied to the auricle and norathyriol, betamethasone ointment, and delgocitinib ointment applied to the auricle of mice with atopic dermatitis.
[Figure 2] The following photographs show the condition of mouse auricular skin on day 7 after the development of atopic dermatitis in Figure 1. (a) control, (b) vehicle (MC903 application), (c) MC903 + norathyriol, (d) MC903 + betamethasone ointment, and (e) MC903 + delgocitinib ointment results.
[Figure 3] The graph shows mouse serum collected after 7 days in Figure 1 and IgE antibody measured in the serum.
[Figure 4] The graph shows mouse auricular tissue collected after 7 days in Figure 1 and TSLP secretion measured in the tissue.
[Figure 5] The graph shows mouse auricular tissue collected after 7 days in Figure 1 and IL-4 secretion measured in the tissue.
[Figure 6] The graph shows mouse auricular tissue collected after 7 days in Figure 1 and IL-5 secretion measured in the tissue.
[Figure 7] The graph shows the number of nose-scratching behaviors measured in mice immunized with ovalbumin to develop allergic rhinitis and treated with norathyriol, mangiferin 8a, tofacitinib, dexamethasone, and olopatadine.
[Figure 8] The graph shows the number of sneezes measured in mice immunized with ovalbumin to develop allergic rhinitis and treated with norathyriol, mangiferin 8a, tofacitinib, dexamethasone, and olopatadine.
[Figure 9] The graph shows the OVA-IgE antibody measured by collecting mouse serum after administration of norathyriol, mangiferin 8a, tofacitinib, dexamethasone, and olopatadine to mice immunized with ovalbumin and suffering from allergic rhinitis.
[Figure 10] The graph shows the IgE antibody measured by collecting mouse serum after administration of norathyriol, mangiferin 8a, tofacitinib, dexamethasone, and olopatadine to mice immunized with ovalbumin and suffering from allergic rhinitis.
[Figure 11] The graph shows the BAFF measured by collecting mouse serum after administration of norathyriol, mangiferin 8a, tofacitinib, dexamethasone, and olopatadine to mice immunized with ovalbumin and suffering from allergic rhinitis.
[Figure 12] The graph shows the IL-4 measured by collecting mouse serum after administration of norathyriol, mangiferin 8a, tofacitinib, dexamethasone, and olopatadine to mice immunized with ovalbumin and suffering from allergic rhinitis.
[Figure 13] The graph shows the IL-5 measured by collecting mouse serum after administration of norathyriol, mangiferin 8a, tofacitinib, dexamethasone, and olopatadine to mice immunized with ovalbumin and suffering from allergic rhinitis.
[Figure 14] The graph shows the IL-17 measured by collecting mouse serum after administration of norathyriol, mangiferin 8a, tofacitinib, dexamethasone, and olopatadine to mice immunized with ovalbumin and suffering from allergic rhinitis.
[Figure 15] The graph shows the number of coughs measured in mice immunized with ovalbumin to develop bronchial asthma and treated with norathyriol, mangiferin 8a, tofacitinib, dexamethasone, and olopatadine.
[Figure 16] The graph shows the OVA-IgE antibody measured by collecting mouse serum after administration of norathyriol, mangiferin 8a, tofacitinib, dexamethasone, and olopatadine to mice immunized with ovalbumin and suffering from bronchial asthma.
[Figure 17] The graph shows the IgE antibody measured by collecting mouse serum after administration of norathyriol, mangiferin 8a, tofacitinib, dexamethasone, and olopatadine to mice immunized with ovalbumin and suffering from bronchial asthma.
[Figure 18] The graph shows the BAFF measured by collecting mouse serum after administration of norathyriol, mangiferin 8a, tofacitinib, dexamethasone, and olopatadine to mice immunized with ovalbumin and suffering from bronchial asthma.
[Figure 19] The graph shows the IL-4 measured by collecting mouse serum after administration of norathyriol, mangiferin 8a, tofacitinib, dexamethasone, and olopatadine to mice immunized with ovalbumin and suffering from bronchial asthma.
[Figure 20] The graph shows the IL-5 measured by collecting mouse serum after administration of norathyriol, mangiferin 8a, tofacitinib, dexamethasone, and olopatadine to mice immunized with ovalbumin and suffering from bronchial asthma.
[Figure 21] The graph shows the IL-17 measured by collecting mouse serum after administration of norathyriol, mangiferin 8a, tofacitinib, dexamethasone, and olopatadine to mice immunized with ovalbumin and suffering from bronchial asthma.
[Figure 22] The graph measures the frequency of diarrhea in mice immunized with ovalbumin to develop food allergy and treated with norathyriol, mangiferin 8a, tofacitinib, dexamethasone, and olopatadine.
[Figure 23] The graph shows the OVA-IgE antibody measured by collecting mouse serum after administration of norathyriol, mangiferin 8a, tofacitinib, dexamethasone, and olopatadine to mice immunized with ovalbumin and developing food allergy.
[Figure 24] The graph shows the IgE antibody measured by collecting mouse serum after administration of norathyriol, mangiferin 8a, tofacitinib, dexamethasone, and olopatadine to mice immunized with ovalbumin and developing food allergy.
[Figure 25] The graph shows the BAFF measured by collecting mouse serum after administration of norathyriol, mangiferin 8a, tofacitinib, dexamethasone, and olopatadine to mice immunized with ovalbumin and developing food allergy.
[Figure 26] The graph shows the IL-4 measured by collecting mouse serum after administration of norathyriol, mangiferin 8a, tofacitinib, dexamethasone, and olopatadine to mice immunized with ovalbumin and developing food allergy.
[Figure 27] The graph shows the IL-5 antibody measured by collecting mouse serum after administration of norathyriol, mangiferin 8a, tofacitinib, dexamethasone, and olopatadine to mice immunized with ovalbumin and developing food allergy.
[Figure 28] The graph shows the IL-17 measured by collecting mouse serum after administration of norathyriol, mangiferin 8a, tofacitinib, dexamethasone, and olopatadine to mice immunized with ovalbumin and developing food allergy.
[Figure 29] The graph shows the number of nose-scratching behaviors measured in mice immunized with cedar pollen to develop pollinosis and treated with norathyriol, mangiferin 8a, tofacitinib, dexamethasone, and olopatadine.
[Figure 30] The graph shows the number of sneezes measured in mice immunized with cedar pollen to develop pollinosis and treated with norathyriol, mangiferin 8a, tofacitinib, dexamethasone, and olopatadine.
[Figure 31] The graph shows the IgE antibody measured by collecting mouse serum after administration of norathyriol, mangiferin 8a, tofacitinib, dexamethasone, and olopatadine to mice immunized with cedar pollen and developing pollinosis.
[Figure 32] The graph shows the IL-4 measured by collecting mouse serum after administration of norathyriol, mangiferin 8a, tofacitinib, dexamethasone, and olopatadine to mice immunized with cedar pollen and developing pollinosis.
[Figure 33] The graph shows the IL-5 measured by collecting mouse serum after administration of norathyriol, mangiferin 8a, tofacitinib, dexamethasone, and olopatadine to mice immunized with cedar pollen and developing pollinosis.
[Figure 34] The graph shows the IL-17 measured by collecting mouse serum after administration of norathyriol, mangiferin 8a, tofacitinib, dexamethasone, and olopatadine to mice immunized with cedar pollen and developing pollinosis.
[Figure 35] This panel shows IgM and IgD expression of mouse B cells with the reagent (compound) and followed by BAFF stimulation, measured using flow cytometry. (a) represents the results of control, (b) BAFF only, and (c) BAFF and mangiferin.
[Figure 36] The panels show IgM and IgD expression of mouse B cells with the reagents (compounds) and followed by BAFF stimulation, as measured using flow cytometry. (d) represents the results of BAFF and mangiferin 8a, (e) BAFF and norathyriol, and (f) BAFF and 1,3,5,6-tetrahydroxyxanthone.
[Figure 37] This panel shows IgM and IgD expression of mouse B cells with the reagent (compound) and followed by BAFF stimulation, measured using flow cytometry. (g) represents results for BAFF and xanthohydrol, (h) BAFF and α-mangostin, and (i) BAFF and γ-mangostin.
[Figure 38] This panel shows CD138 expression of mouse B cells with the reagent (compound) and followed by BAFF stimulation, as measured using flow cytometry. (a) represents the results for mangiferin, (b) for mangiferin 8a, and (c) for norathyriol.
[Figure 39] This panel shows CD138 expression of mouse B cells with the reagent (compound) and followed by BAFF stimulation, as measured using flow cytometry. (d) represents the results for 1,3,5,6-tetrahydroxyxanthone, and (e) for xanthohydrol.
[Figure 40] This panel shows CD138 expression of mouse B cells with the reagent (compound) and followed by BAFF stimulation, as measured using flow cytometry. (f) represents the results for α-mangostin, and (g) for γ-mangostin.
[Figure 41] Mangiferin, mangiferin 8a, norathyriol, 1,3,5,6-tetrahydroxyxanthone, xanthohydrol, α-mangostin, γ-mangostin were added to pre-B cells collected from mice. Then, the expression of phospho-NIK, NIK, NF-κB p52 nuclear, NF-κB p65 nuclear and Lamin in these pre-B cells after BAFF stimulation was examined by Western blotting.
[Figure 42] The graph shows TNFα measured in the culture supernatant in which the reagent (compound) was added to pre-B cells collected from mice, followed by BAFF stimulation.
[Figure 43] The graph shows IL-6 measured in the culture supernatant in which the reagent (compound) was added to pre-B cells collected from mice, followed by BAFF stimulation.
[Figure 44] This panel shows IFNy expression of T cells collected from inflammation model mice treated with the reagent (compound), measured using flow cytometry. (a) represents the results for mangiferin 8a and (b) for norathyriol.
[Figure 45] This panel shows IL-4 expression of T cells collected from inflammation model mice treated with the reagent (compound), measured using flow cytometry. (a) represents the results for mangiferin 8a and (b) for norathyriol.
[Figure 46] This panel shows IL-17A expression of T cells collected from inflammation model mice treated with the reagent (compound), measured using flow cytometry. (a) represents the results for mangiferin 8a and (b) for norathyriol.
[Figure 47] This panel shows Foxp3 expression of T cells collected from inflammation model mice treated with the reagent (compound), measured using flow cytometry. (a) represents the results for mangiferin 8a and (b) for norathyriol.
[Figure 48] This graph examines the effect of each compound on cell proliferation when mouse B cells are stimulated with LPS.
[Figure 49] This graph examines the effect of each compound on cell proliferation when mouse macrophage is stimulated with LPS.
[Figure 50] This panel shows IgM and IgD expression of mouse B cells with the reagent (compound) followed by LPS stimulation, measured using flow cytometry. (a) represents the results for control, and (b) for LPS-only.
[Figure 51] This panel shows IgM and IgD expression of mouse B cells with the reagent (compound) followed by LPS stimulation, measured using flow cytometry. (c) represents the results for LPS and mangiferin 8a, and (d) for LPS and norathyriol.
[Figure 52] This panel shows CD138 expression of mouse B cells with the reagent (compound) and followed by LPS stimulation, measured using flow cytometry. (a) represents the results for mangiferin 8a and (b) for norathyriol.
[Figure 53] The panels show CD11b and F4/80 expression of cells in which each reagent (compound) was added to mouse macrophages followed by LPS stimulation, as measured using flow cytometry. (a) represents the results for control, and (b) LPS-only.
[Figure 54] The panels show CD11b and F4/80 expression of cells in which each reagent (compound) was added to mouse macrophages followed by LPS stimulation, as measured using flow cytometry. (c) represents the results for LPS and mangiferin 8a, and (d) for LPS and norathyriol.
[Figure 55] This panel shows CD80 expression of cells in which each reagent (compound) was added to mouse macrophages followed by LPS stimulation, measured using flow cytometry. (a) represents the results for mangiferin 8a and (b) for norathyriol.
[Figure 56] Mangiferin 8a and norathyriol were added to pre-B cells collected from mice. Then, the expression of phospho-NIK, NIK, NF-κB p52 nuclear, NF-κB p65 nuclear and Lamin in these pre-B cells after LPS stimulation was examined by Western blotting.
[Figure 57] The graph shows the results of the study of the inhibitory effect of each compound on the secretion of TNFα when mouse B cells are stimulated with LPS.
[Figure 58] The graph shows the results of the study of the inhibitory effect of each compound on the secretion of IL-6 when mouse B cells are stimulated with LPS.
[Figure 59] The graph shows the results of the study of the inhibitory effect of each compound on the secretion of TNFα when mouse macrophage is stimulated with LPS.
[Figure 60] The graph shows the results of the study of the inhibitory effect of each compound on the secretion of IL-6 when mouse macrophage is stimulated with LPS.

### [Description of Embodiments]

The following is a description of the pharmaceutical compositions for the improvement of allergic diseases. These are pharmaceutical compositions and NIK inhibitors. The following description is an example of an embodiment and an example of the invention, and the invention is not limited to the following description. The following embodiments may be modified insofar as they do not depart from the intent of the invention.

In addition, "improvement" may include reduction and suppression of symptoms as well as treatment of the disease or symptom being targeted as indicated in this description. In addition, "therapy or treatment" includes not only the recovery of a disease condition, but also the effect of inhibiting or delaying the progression of the disease condition.

The pharmaceutical compositions for the improvement of allergic diseases of the present invention include mangiferin with xanthone skeleton (formula (3)), mangiferin 8a (formula (1)), norathyriol (formula (2)), also 1,3,5,6-tetrahydroxyxanthone (formula (4)), xanthohydrol (formula (5)), α-mangostin (formula (6)), and γ-mangostin (formula (7)) as active ingredients. Formula (1) is 1,2',3',4',6-penta-O-propionyl mangiferin, hereinafter referred to as "mangiferin 8a". Mangiferin is shown in formula (3). Formula (2) is 1,3,6,7-tetrahydroxyxanthone, hereafter referred to as "norathyriol".

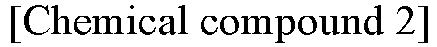

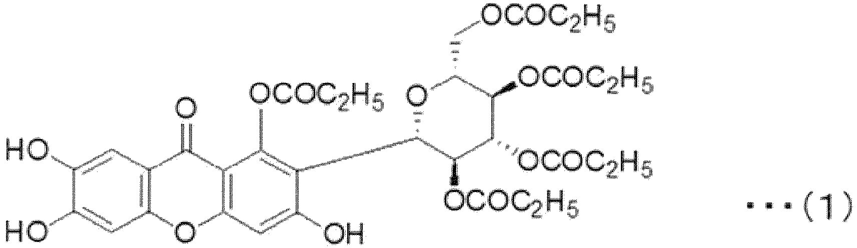

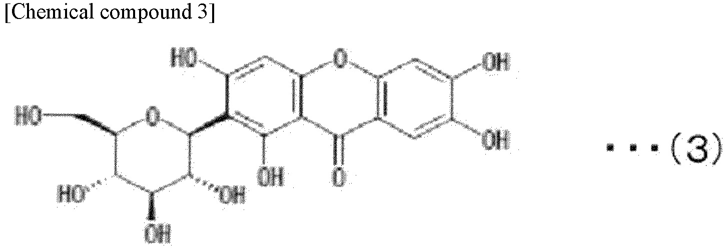

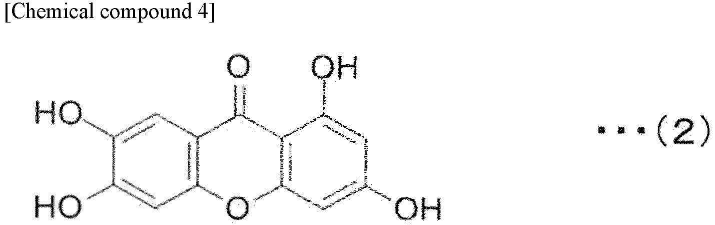

Mangiferin 8a is an ether bonded propionyl group to some hydroxy group positions of mangiferin (see formula (3)), as can be seen in formula (1). This compound shows better inhibition of NIK activity than mangiferin, as shown in the following examples, and is highly effective in preventing allergic diseases.

Norathyriol (CAS No.: 3542-72-1) is a xanthone (CAS No.: 90-47-1) with a hydroxy group attached to part of it, and like mangiferin 8a, has a xanthone structure. And, like mangiferin 8a, it shows more inhibition of NIK activity than mangiferin, indicating a high preventive effect against allergic diseases. Also, 1,3,5,6-tetrahydroxyxanthone (CAS No.: 5084-31-1: formula (4)), xanthohydrol (CAS No.: 90-46-0: formula (5)), α-mangostin (CAS No.: 6147-11-1: formula (6)), γ-mangostin (CAS No.: 31271-07 -5: formula (7)) are also compounds with a xanthone structure, and like norathyriol and mangiferin 8a, they show more inhibition of NIK activity than mangiferin, indicating a high preventive effect against allergic diseases.

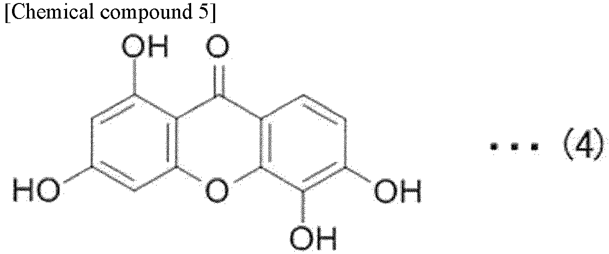

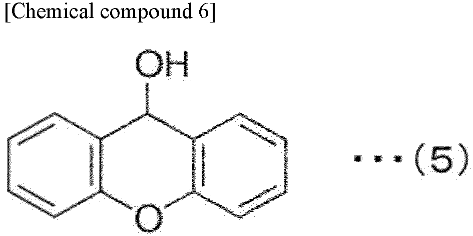

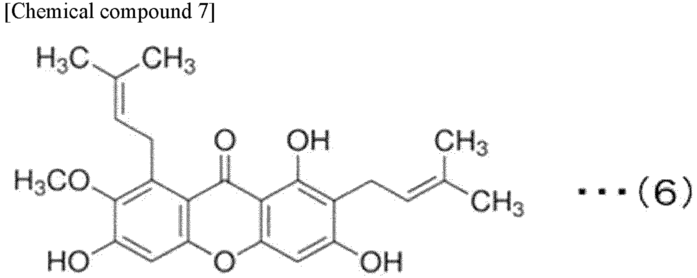

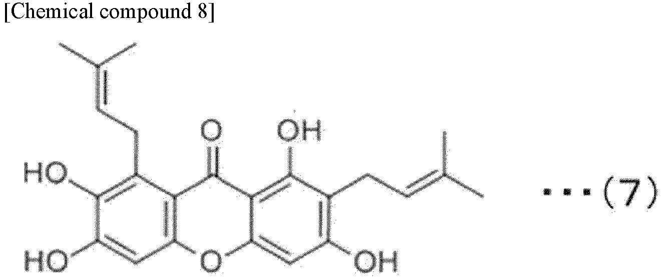

These compounds, like mangiferin, are low molecular weight compounds that dissolve well in water and can be taken orally. The pharmaceutical compositions for the improvement of allergic diseases of the present invention contain at least any of the above compounds as active ingredients. It may also contain other pharmaceutically acceptable ingredients. The pharmaceutical composition for improvement of allergic diseases may also contain more than one of these compounds.

The pharmaceutical composition for improvement of allergic diseases of the present invention can be provided as a therapeutic agent for allergic diseases (it may be called a pharmaceutical composition). These compounds can also be provided as NIK inhibitors. As a pharmaceutical composition, the compositions of the invention can be effective when administered orally as well as by intravenous, subcutaneous, or intramuscular injection. Therefore, it can be provided as an internal application. For example, powdered pharmaceutical compositions for improving allergic diseases can be provided in the form of capsules, granules, dispersions, tablets, etc. For oral dosage forms, additives such as binders, lubricants, disintegrants, coloring agents, flavoring substances, preservatives, antioxidants, and stabilizers are added, and capsules, granules, dispersions, and tablets can be manufactured according to conventional methods.

Furthermore, the pharmaceutical compositions for the improvement of allergic diseases may be formulated into topical formulations such as liquids, ointments, creams, gels, pastes, and aerosols for parenteral administration. Water, lower alcohols, dissolution aids, surfactants, emulsion stabilizers, gelling agents, adhesives, and other necessary base ingredients can be added to make the topical application. Additives such as vasodilators, corticosteroids, keratolytic agents, moisturizers, disinfectants, antioxidants, coolants, fragrances, and dyes may be added as needed.

The pharmaceutical compositions for improving allergic diseases can also be provided as processed foods. In other words, the pharmaceutical composition for improvement of allergic diseases of the present invention has the same effect as the pharmaceutical composition for improvement of allergic diseases of the present invention when taken as a processed food.

Processed foods include not only general processed foods such as candy, gum, jelly, biscuits, cookies, rice crackers, bread, noodles, fish and meat paste products, tea, soft drinks, coffee drinks, lactic drinks, lactic acid beverages, yogurt, ice cream, pudding, etc.. In addition, nutritional supplements, feed, food additives, etc. are also included in the processed food category.

The processed foods can be prepared by adding the pharmaceutical composition for improving allergic diseases in the ingredients of these processed foods. The addition of mangiferin, mangiferin 8a, norathyriol, 1,3,5,6-tetrahydroxyxanthone, xanthohydrol, α-mangostin, and γ-mangostin to these processed foods is excluded from application when materials containing these ingredients are used as raw materials in the first place. However, a processed food product that contains these ingredients in excess of the amount of these ingredients that the material contains is a processed food product of the present invention. The following examples describe the pharmaceutical compositions for the improvement of allergic diseases.

The diseases covered by the pharmaceutical compositions for improvement of allergic diseases of the present invention are, to restate, bronchial asthma, atopic dermatitis, pollinosis, allergic rhinitis, food allergy, metal allergy, drug allergy, anaphylaxis, urticaria, allergic conjunctivitis, allergic bronchitis, and contact dermatitis.

In addition, diseases other than those mentioned above that can be determined as allergic diseases due to overexpression of BAFF or activation of the NF-κB pathway can also be included in the list.

### [Example]

### <Synthesis of mangiferin 8a>

Mangiferin 8a was obtained in two steps: 1,3,2',3',4',6,6',7-octa-O-propionyl mangiferin was synthesized and further synthesized from it in two steps.

### (1) Synthesis of 1,3,2',3',4',6,6',7-octa-O-propionyl mangiferin

Mangiferin (2.1 g, 4.98 mmol), propionic anhydride (12.8 mL, 99.4 mmol) and dry pyridine (60 mL) were heated at 80°C for 5 h. The reaction solution was poured into ice water (400 mL) and extracted with ethyl acetate. The ethyl acetate layer was washed sequentially with ice-cold 10% sulfuric acid, saturated sodium bicarbonate solution and saturated brine, dried over anhydrous sodium sulfate, filtered and concentrated. The residue was purified using column chromatography (n-hexane/ethyl acetate = 1/1) to afford 1,3,2',3',4',6,6',7-Octa-O-propionyl mangiferin (3.71 g, 86%) of formula (8) was obtained as a colorless solid.

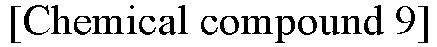

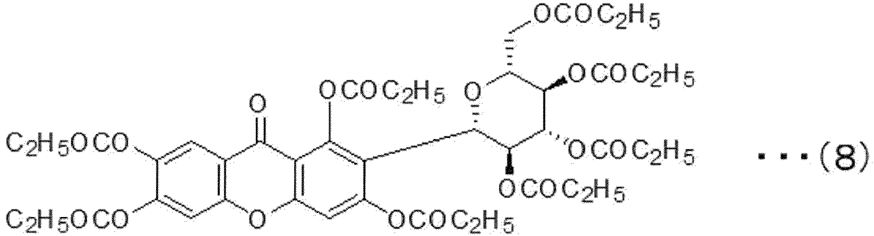

The NMR spectrum is shown below.
IR (KBr): 1774, 1755, 1667, 1620, 1458, 1354, 1273, 1157, 1114, 1083 cm⁻¹. ¹H NMR (800 MHz, DMSO-d₆, 25 °C) δ: 0.71-0.77/0.93-0.97 (each 3H, m, COCH₂CH₃), 0.98-1.02/1.12-1.16/1.19-1.28 (each 6H, m, COCH₂CH₃), 1.92-2.01 (2H, m, COCH₂CH₃), 2.17-2.32 (6H, m, COCH₂CH₃), 2.60-2.94 (8H, m, COCH₂CH₃), 3.86-3.92 (1H, m, H-6'a), 4.21-4.30 (2H, m, H-5' and H-6'b), 5.01-5.06 (1H, m, H-4'), 5.10-5.20 (1H, m, H-1'), 5.45-5.53 (2H, m, H-2' and H-3'), 7.51/7.53 (1H, each s, H-4), 7.68/7.69 (1H, each s, H-5), 7.97/7.98 (1H, each s, H-8). ¹³C NMR (200 MHz, DMSO-d₆ 25 °C)
δ: 8.64/8.75/8.77/8.91/8.94/8.96/9.00/9.13/9.16 (COCH₂CH₃), 26.58/26.63/26.7/26.8/26.89/ 26.94/27.0/27.1/27.32/27.34 (COCH2CH3), 62.1/62.2 (C6'), 68.18/68.20 (C4'), 69.6/70.0 (C2'), 70.8/71.3 (C1'), 73.5/73.6 (C3'), 75.0/75.1 (C5'), 110.2/111.8 (C4), 111.6/112.7 (C9a), 113.2/113.3 (C5), 118.8/118.9 (C2), 119.6/119.7 (C8a), 120.3/120.4 (C8), 139.5/139.6 (C7), 147.9 (C6), 149.1/150.9 (C1), 152.47/152.51 (C8b), 153.4/154.8 (C4a), 156.5/156.8 (C3), 170.9/171.04/171.06/ 171.09/171.67/171.72/171.9/172.4/172.6/172.7/173.18/173.22/173.51/173.54 (COCH₂CH₃), 173.1 (C9). HRMS (ESI) m/z: [M+Na]⁺ Calcd for C₄₃H₅₀O₁₉Na 893.2839; Found 893.2853.

### (2) Synthesis of mangiferin 8a (1,2',3',4',6-penta-O-propionyl mangiferin)

A mixture of 1,3,2',3',4',6,6',7-octa-O-propionyl mangiferin (3.7 g, 4.25 mmol), ammonium acetate (3.8 g, 49.4 mmol), methanol (80 mL) and water (40 mL) was stirred at room temperature for 5.5 h. After methanol was removed from the reaction solution by concentrating under reduced pressure, the residue was diluted with ethyl acetate (100 mL). The mixture was washed sequentially with water and saturated brine, dried over anhydrous sodium sulfate, filtered, and concentrated. The pale yellow solid (2.54 g) obtained by purifying the residue using column chromatography [CHCl₃/CH₃OH (20:1)] was dissolved in methanol (80 mL) and decolorized with activated carbon to afford mangiferin 8a (2.16 g, 73%) of formula (1) as a colorless solid.

The NMR spectrum is shown below.
IR (KBr): 3406, 1751, 1620, 1462, 1354, 1284, 1192, 1083 cm⁻¹. ¹H NMR (800 MHz, DMSO-d₆, 25 °C) δ: 0.74/0.94 (each 3H, t-like, J = 7.6, COCH₂CH₃), 0.97-1.02 (6H, m, COCH₂CH₃), 1.20/1.23 (each 1.5H, t-like, J = 7.6, COCH₂CH₃), 1.92-2.02 (2H, m, COCH₂CH₃), 2.15-2.33 (6H, m, COCH₂CH₃), 2.61-2.86 (2H, m, COCH₂CH₃), 3.85 (0.5H, dd-like, J = ca. 12.5, 1.9, H-6a'), 4.05 (0.5H, br d-like, J = ca. 12.5, H-6a'), 4.07 (0.5H, ddd, J = 9.8, 4.7, 1.9, H-5'), 4.09-4.14 (0.5H, m, H-6b'), 4.11 (0.5H, br d-like, J = ca. 9.5, H-5'), 424 (0.5H, dd, J = 12.5, 4.7, H-6b'), 4.96 (0.5H, d, J = 9.9, H-1'), 5.00 (0.5H, dd, J = 9.5, 9.5, H-4'), 5.02 (0.5H, dd, J = 9.8, 9.8, H-4'), 5.16 (0.5H, d, J = 10.0, H-1'), 5.35 (0.5H, dd, J = 9.5, 9.5, H-3'), 5.40 (0.5H, dd, J = 9.8, 9.5, H-3'), 5.49 (0.5H, dd, J = 10.0, 9.5, H-2'), 5.84 (0.5H, dd, J = 9.9, 9.5, H-2'), 6.72/6.75 (each 0.5H, s, H-4), 6.80/6.81 (each 0.5H, s, H-5), 7.29/7.30 (each 0.5H, s, H-8), 9.62/10.5 (each 1H, br s, OH), 11.2/11.4 (each 0.5H, br s, OH). ¹³C NMR (200 MHz, DMSO-d₆, 25 °C) δ: 8.81/8.86/8.96/9.02/9.06/9.11/9.12/9.20 (COCH₂CH₃), 26.6/26.89/26.91/26.94/27.00/27.04/27.1/27.4 (COCH₂CH₃), 62.1/62.3 (C-6'), 68.2/68.3 (C-4'), 68.7/70.4 (C-2'), 71.0/71.1 (C-1'), 73.8/74.1 (C-3'), 74.6/75.2 (C-5'), 99.5/100.8 (C-4), 102.5 (C-5), 106.6/107.8 (C-9a), 109.1 (C-8), 113.3 (C-2), 114.0/114.1 (C-8a), 143.8 (C-7), 149.77/149.82 (C-6, C-1), 151.3 (C-1), 153.3 (C-8b), 157.5/157.7 (C-4a), 160.7/162.2 (C-3), 171.1/171.9/172.2/172.5/172.8/173.1/173.4/173.6 (COCH₂CH₃), 172.68/172.71 (C-9). HRMS (ESI)
m/z: [M-H]⁻ Calcd for C₃₄H₃₇O₁₆ 701.2076; Found 701.2070.

### < Synthesis of norathyriol >

Norathyriol was synthesized according to the method of Non-Patent Literature 47.

Norathyriol was synthesized as follows. Namely, 2,4,5-trimethoxybenzoic acid (Compound II) was treated with thionyl chloride to obtain 2,4,5-trimethoxybenzoic acid chloride (Compound III) according to the method described in the literature (Non-Patent Literature 48). Next, the obtained compound (Compound III) was reacted with 1,3,5-trimethoxybenzene (Compound IV) by Friedel-Craft reaction to give 2-hydrodoxy-2',4,4',5,6'-pentamethoxybenzophenone (Compound V) was obtained. The compound (V) was further treated with tetrabutylammonium hydroxide to afford 1,3,6,7-tetramethoxyxanthone (compound VI), followed by demethylation to give norathyriol (compound I) in 39% yield.

The following is a detailed description of the synthetic route of this example.

### (1) Synthesis of 2,4,5-trimethoxybenzoic acid chloride (Compound III)

2,4,5-trimethoxybenzoic acid (Compound II, 8.49 g, 0.040 mol) was dissolved by gradually adding thionyl chloride (5 mL) at room temperature under an argon atmosphere, and then heated to reflux for 6 h. After completion of the reaction, the reaction mixture was distilled off under reduced pressure to obtain 2,4,5-trimethoxybenzoic acid chloride (compound III, 8.30 g, 90%). The obtained compound (III) was immediately used for the next reaction.

### (2) Synthesis of 2-hydrodoxy-2',4,4',5,6'-pentamethoxybenzophenone (Compound V)

To a mixed suspension of 2,4,5-trimethoxybenzoic acid chloride (Compound III, 8.07 g, 0.035 mol) obtained as described above, 1,3,5-trimethoxybenzene (Compound IV, 6.48 g, 0.0385 mol), and anhydrous diethyl ether (500 mL) was added aluminum chloride (16 g) gradually at room temperature under argon atmosphere, and the reaction mixture was stirred at room temperature for 48 h. After removing the solvent from the reaction mixture under reduced pressure, water was added to the residue and extracted with ethyl acetate. The extract was washed with saturated brine, dried over anhydrous sodium sulfate, filtered off the drying agent using folded filter paper, and the filtrate was removed under reduced pressure to give the crude product. The crude product was purified by silica gel column chromatography (n-hexane:ethyl acetate = 1:1, v/v) to give 2-hydrodoxy-2',4,4',5,6'-pentamethoxybenzophenone (Compound V, 8.43 g, 69%).

### (3) Synthesis of 1,3,6,7-tetramethoxyxanthone (Compound VI)

2-hydrodoxy-2',4,4',5,6'-pentamethoxybenzophenone (Compound V, 6.97 g, 0.020 mol) obtained as described above was dissolved in a mixture of pyridine (10 mL) and water (10 mL), and 40% tetrabutylammonium hydroxide solution (5 mL) was added and heated to reflux for 6 h. The resulting reaction mixture was poured into 5% hydrochloric acid and extracted with ethyl acetate. The extract was washed with saturated brine, dried over anhydrous sodium sulfate, filtered off the drying agent using folded filter paper, and the filtrate was removed under reduced pressure. The crude product was purified by silica gel column chromatography (n-hexane: ethyl acetate = 1:1, v/v) to afford 1,3,6,7-tetramethoxyxanthone (compound VI, 5.82 g, 92%).

### (4) Synthesis of norathyriol (Compound I)

A mixture of 1,3,6,7-tetramethoxyxanthone (Compound VI, 4.74 g, 0.015 mol) and pyridine hydrochloride (5.00 g) obtained as described above was heated and stirred at 200 °C for 6 h. The resulting reaction mixture was allowed to cool to room temperature, poured into 5% hydrochloric acid, and extracted with ethyl acetate. The extract was washed with saturated brine, dried over anhydrous sodium sulfate, filtered off the drying agent using folded filter paper, and the filtrate was removed under reduced pressure to obtain the crude product. The crude product was purified by silica gel column chromatography (chloroform:methanol = 7:1, v/v) to afford norathyriol of formula (2) (Compound I, 2.65 g, 68%). The norathyriol structure is represented by formula (2).

The following are the results of our investigation of the cell death inducing effects of the compounds of the present invention.

### < Example 1: Examination to evaluate the anti-inflammatory effect of a compound with a xanthone structure on atopic dermatitis >

MC903 at 2 nM was applied to the right auricle of a 6-week-old male Balb/c (Japan SLC Inc.). Starting 8 h later, 5% norathyriol aqueous solution (1 mL of ethanol containing 0.05 mg of norathyriol), betamethasone ointment, and delgocitinib ointment were applied to the auricle for 7 days. The measurement of auricular skin swelling was calculated by calipers. The day on which the first application of the compound was made was defined as day 0. Betamethasone and delgocitinib are currently in clinical use in atopic dermatitis and were used as control drugs in this example.

Those without MC903 or norathyriol are called the control group, those with MC903 are called the vehicle group, those with 5% norathyriol aqueous are called the 5% norathyriol-applied group, those with betamethasone ointment are called the betamethasone-applied group, and those with delgocitinib ointment is referred to as the delgocitinib-applied group.

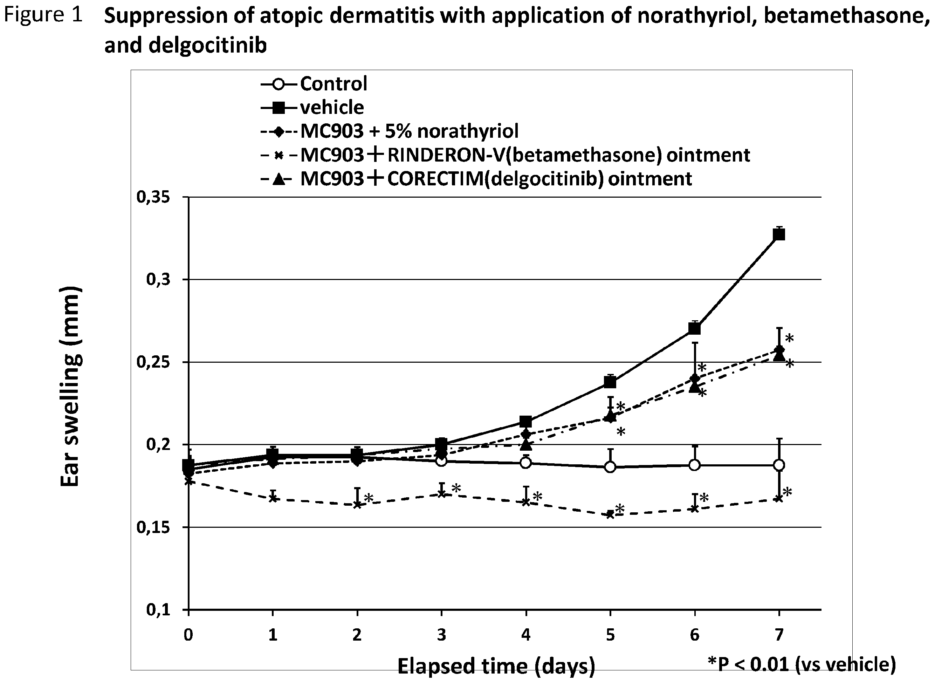

Figure 1 shows the results of auricular swelling. Referring to Figure 1, the horizontal axis shows the number of days elapsed and the vertical axis shows the auricular skin swelling. Solid white circles indicate the control group (labeled "control"). Solid black squares indicate the vehicle group (labeled "vehicle"), dotted black diamonds indicate the 5% norathyriol aqueous application group (labeled "MC903 + 5% norathyriol"), dashed black crosses indicate the betamethasone application group (labeled "MC903 + betamethasone ointment"), and single-dotted black dots indicate the delgocitinib application group (labeled "MC903 + delgocitinib ointment"). Those that were significantly different (P<0.01) from the target group are indicated with an "*".

In the vehicle group (black square mark "-■-"), a swelling of the auricle was observed from day 4, and then the swelling increased over time. In the 5% norathyriol aqueous application group (dotted black diamond mark "•••◆•••.") and the delgocitinib application group (dotted black triangle mark "•••**▲**•••"), swelling was significantly reduced, and in the betamethasone application group (dotted black triangle mark "•••×•••"), swelling of the auricle was almost completely absent.

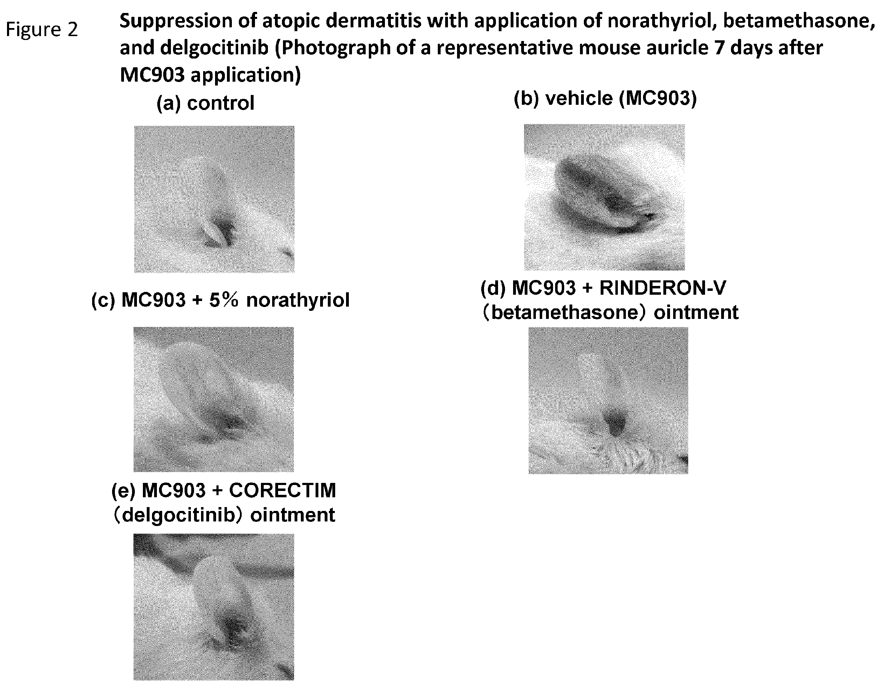

Figure 2 shows a photograph of the right auricle of the mouse at day 7 in Figure 1. Figure 2(a) shows a picture of the right auricle of one mouse in the control group, Figure 2(b) in the vehicle group, Figure 2(c) in the 5% norathyriol aqueous application group, Figure 2(d) in the betamethasone application group, and Figure 2(e) in the delgocitinib application group. In the control group in Figure 2(a), there is no swelling of the auricular skin at all because MC903 was not applied. In the vehicle group in Figure 2(b), swelling and redness of the auricular skin are prominent. On the other hand, the 5% norathyriol aqueous application group in Figure 2(c) and the delgocitinib application group in Figure 2(e) showed some redness, but not as much as in the target group, and the betamethasone application group in Figure 2(d) showed no swelling or redness, indicating clear anti-inflammatory effects.

Namely, the 5% norathyriol aqueous application group significantly reduced inflammation caused by atopic dermatitis more than the vehicle group. In other words, compositions with norathyriol as an active ingredient can inhibit inflammation caused by atopic dermatitis.

### < Example 2: Inhibition of IgE antibody production >

Serum was collected from mice on day 7 in Example 1, and IgE antibody production was measured by enzyme-linked immunosorbent assay (ELISA). This assay was performed using a mouse IgE antibody ELISA kit (R&D).

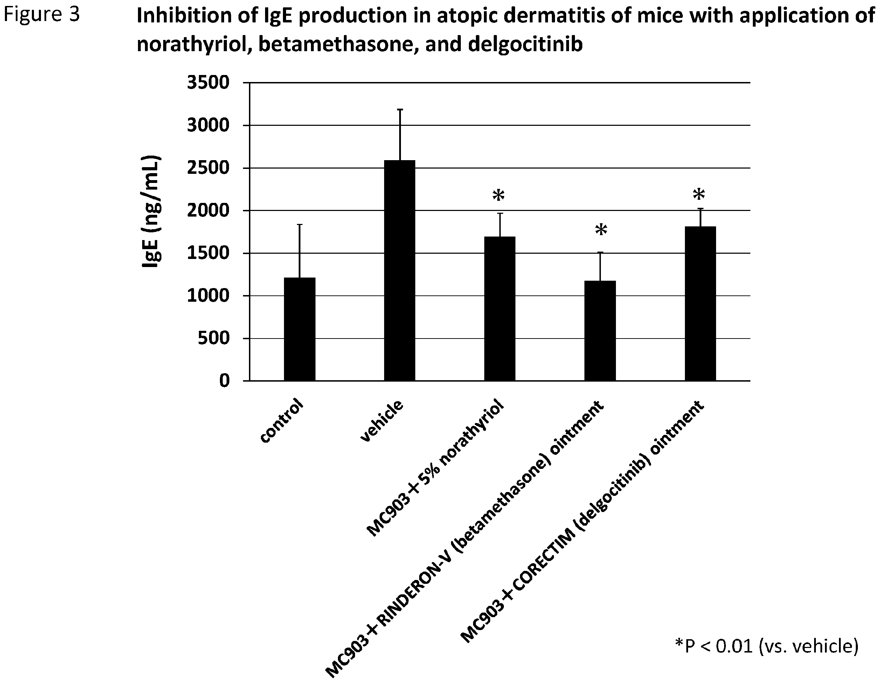

Results are shown in Figure 3. The horizontal axis represents the sample group and the vertical axis represents the amount of IgE antibody. In the control without MC903, the amount of IgE antibody was 1214 ng/mL, whereas in the subject group (labeled "vehicle") to which MC903 was applied, the amount of IgE antibody was 2588 ng/mL, a marked increase, indicating that the subject developed atopic dermatitis. Compared to the vehicle group, IgE antibody production was significantly lower in the norathyriol-, betamethasone-, and delgocitinib-applied groups: 1692 ng/mL in the norathyriol-applied group, 1175 ng/mL in the betamethasone-applied group, and 1812 ng/mL in the delgocitinib-applied group.

As described above, the norathyriol-applied group was found to markedly suppress IgE antibody production caused by atopic dermatitis. In other words, compositions with norathyriol as an active ingredient can inhibit IgE antibody production by atopic dermatitis.

### < Example 3: Inhibition of TSLP secretion >

Tissues were collected from day 7 mice in Example 1 and TSLP secretion was measured by enzyme-linked immunosorbent assay (ELISA). The mouse TSLP ELISA kit (R&D) was used for this measurement.

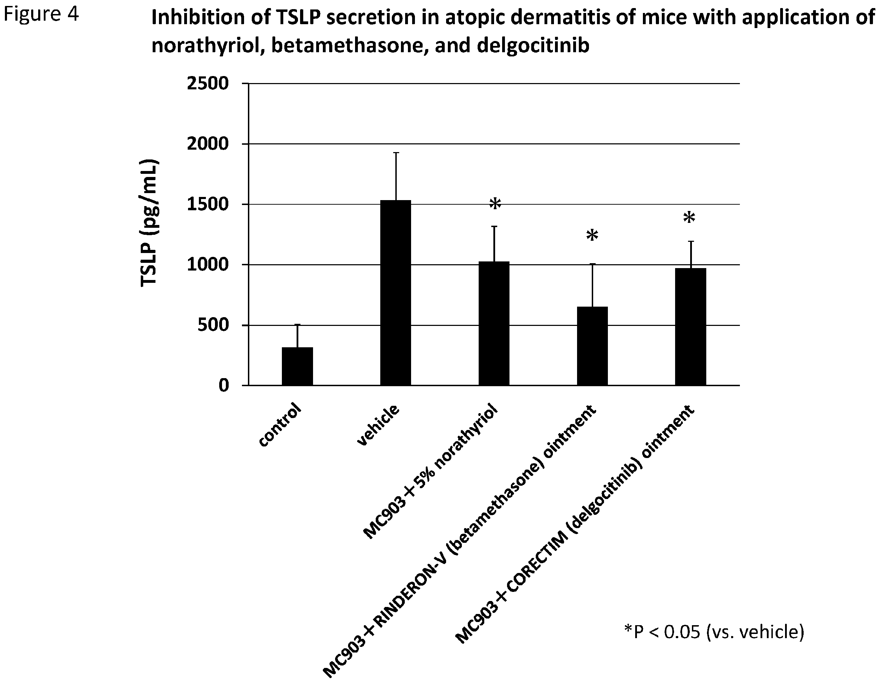

Results are shown in Figure 4. The horizontal axis represents the sample group and the vertical axis represents TSLP secretion. The amount of TSLP secreted by the MC903-applied subject group (labeled "vehicle") was markedly increased to 1533 pg/mL, compared to 317 pg/mL in the MC903-less control, indicating that the subject group developed atopic dermatitis. TSLP secretion was 1026 pg/mL in the norathyriol group, 651 pg/mL in the betamethasone group, and 971 pg/mL in the delgocitinib group, and was significantly lower in the norathyriol group, betamethasone group, and delgocitinib group compared to the vehicle group.

As described above, the norathyriol-applied group was found to markedly inhibit TSLP secretion due to atopic dermatitis. In other words, compositions containing norathyriol as an active ingredient can inhibit TSLP secretion caused by atopic dermatitis.

### < Example 4: Inhibition of IL-4 secretion >

Tissues were collected from day 7 mice in Example 1 and IL-4 secretion was measured by enzyme-linked immunosorbent assay (ELISA). The mouse IL-4 ELISA kit (R&D) was used for this measurement.

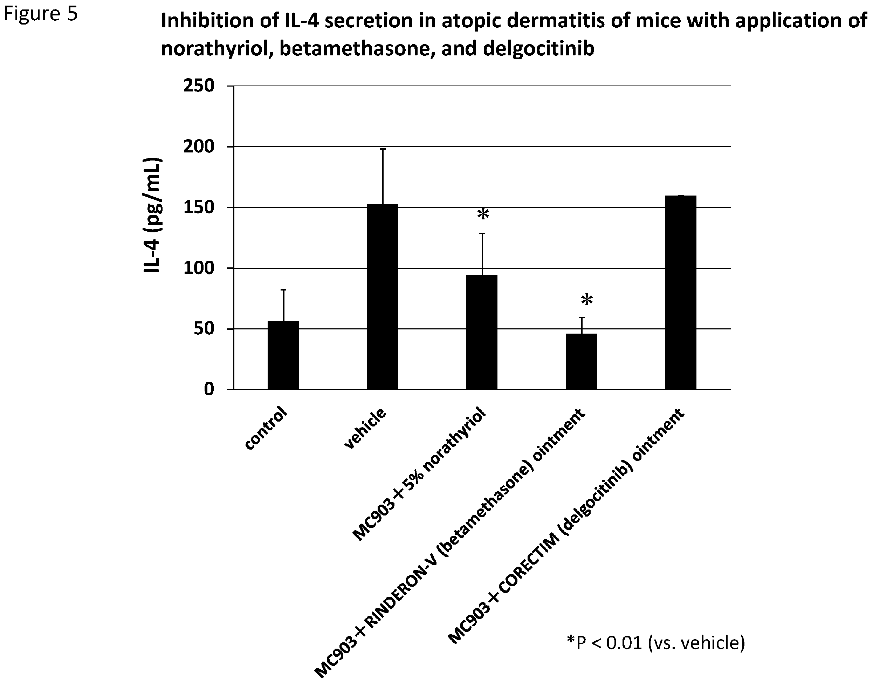

Results are shown in Figure 5. The horizontal axis represents the sample group and the vertical axis represents IL-4 secretion. The amount of IL-4 secreted by the MC903-applied subject group (labeled "vehicle") was markedly increased to 152 pg/mL, compared to 56 pg/mL in the MC903-less control, indicating that the subject group developed atopic dermatitis. IL-4 secretion was 94 pg/mL in the norathyriol group, 45 pg/mL in the betamethasone group, and 159 pg/mL in the delgocitinib group, and was significantly lower in the norathyriol group, betamethasone group, and delgocitinib group compared to the vehicle group.

As described above, the norathyriol-applied group was found to markedly inhibit IL-4 secretion due to atopic dermatitis. In other words, compositions containing norathyriol as an active ingredient can inhibit IL-4 secretion caused by atopic dermatitis.

### < Example 5: Inhibition of IL-5 secretion >

Tissues were collected from day 7 mice in Example 1 and IL-5 secretion was measured by enzyme-linked immunosorbent assay (ELISA). The mouse IL-5 ELISA kit (R&D) was used for this measurement.

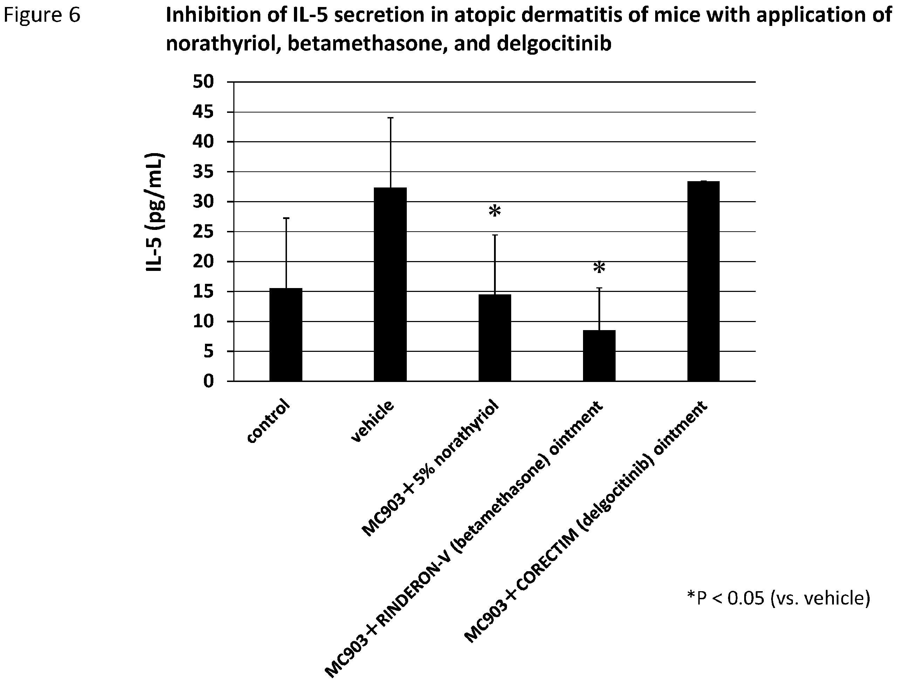

Results are shown in Figure 6. The horizontal axis represents the sample group and the vertical axis represents IL-5 secretion. The amount of IL-4 secreted by the MC903-applied subject group (labeled "vehicle") was markedly increased to 32 pg/mL, compared to 15 pg/mL in the MC903-less control, indicating that the subject group developed atopic dermatitis. IL-5 secretion was 14 pg/mL in the norathyriol group, 8 pg/mL in the betamethasone group, and 33 pg/mL in the delgocitinib group, and was significantly lower in the norathyriol group, betamethasone group, and delgocitinib group compared to the vehicle group.

As described above, the norathyriol-applied group was found to markedly inhibit IL-5 secretion due to atopic dermatitis. In other words, compositions containing norathyriol as an active ingredient can inhibit IL-5 secretion caused by atopic dermatitis.

### < Example 6: Suppression of rhinitis (nose scratching behavior) in a mouse model of ovalbumin-induced allergic rhinitis >

A 0.2 mL dose of 50 µg ovalbumin (OVA) and 2 mg Alum mixture was administered to 6-week-old female Balb/c mice (Japan SLC Inc.) (day 0 of the example). Alum was used as an adjuvant. Seven days later and 14 days later, the Balb/c mice were immunized again with 0.2 mL injections of the above mixture. Starting on day 20, mice were orally treated with 10 mg/kg of norathyriol, 10 mg/kg of mangiferin 8a, 10 mg/kg of tofacitinib (JAK inhibitor), 2 mg/kg of dexamethasone (steroid) and 10 mg/kg of olopatadine (antihistamine) daily for 8 consecutive days. Starting on day 21, 400 µg OVA was administered intranasally, and nose-scratching behavior was measured on day 27. Note that tofacitinib, dexamethasone, and olopatadine are currently in clinical use in allergic rhinitis and were used as control drugs in this example.

The group that was just immunized with OVA is called the subject group, the one that received norathyriol is called the norathyriol group, the one that received mangiferin 8a is called the mangiferin 8a group, the one that received tofacitinib is called the tofacitinib group, the one that received dexamethasone is called the dexamethasone group, and the one that received olopatadine is called the olopatadine group. Each group consisted of five animals.

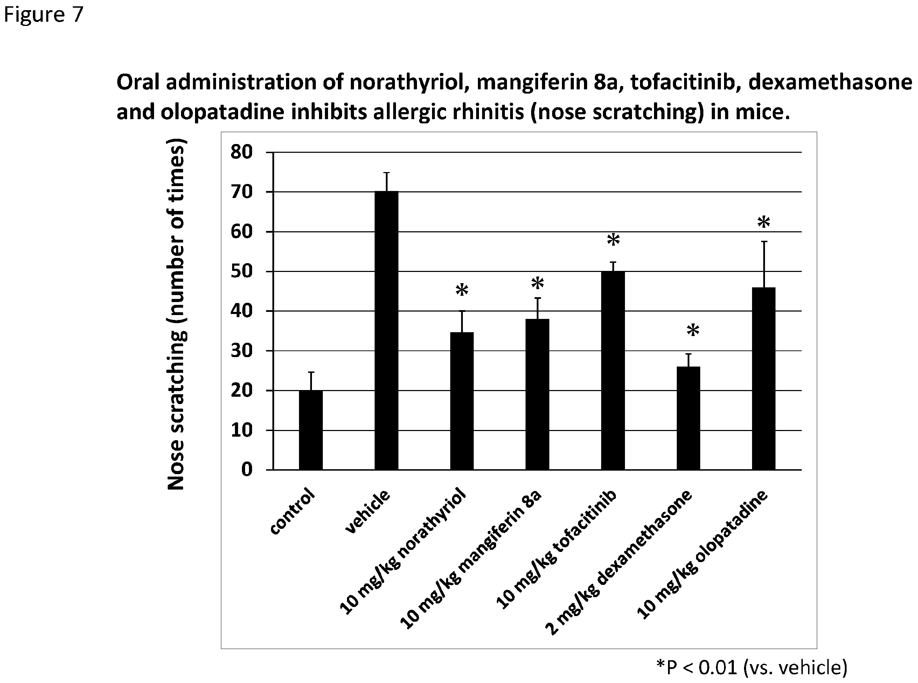

The results are shown in Figure 7. The horizontal axis represents the sample group and the vertical axis represents the number of nose scratches. Compared to the OVA-immunized control (labeled "control"), the OVA-immunized subject group (labeled "vehicle") showed a marked increase in nose-scratching behavior, indicating the development of allergic rhinitis. The norathyriol group ("10 mg/kg norathyriol"), mangiferin 8a group ("10 mg/kg mangiferin 8a"), tofacitinib group ("10 mg/kg tofacitinib"), dexamethasone group ("2 mg/kg dexamethasone"), and olopatadine group ("10 mg/kg olopatadine") significantly decreased the number of nose scratches compared to the vehicle group. The norathyriol and mangiferin 8a groups significantly suppressed nose-scratching behavior compared to the tofacitinib and olopatadine groups.

As described above, it was found that the norathyriol and mangiferin 8a treatment groups significantly suppressed nose-scratching behavior caused by allergic rhinitis. In other words, compositions containing norathyriol or mangiferin 8a as active ingredients can suppress nose-scratching behavior caused by allergic rhinitis.

### < Example 7: Inhibition of rhinitis ("sneezing") in a mouse model of ovalbumin-induced allergic rhinitis >

A 0.2 mL dose of 50 µg ovalbumin (OVA) and 2 mg Alum mixture was administered to 6-week-old female Balb/c mice (Japan SLC Inc.) (day 0 of the example). Alum was used as an adjuvant. Seven days later and 14 days later, the Balb/c mice were immunized again with 0.2 mL injections of the above mixture. Starting on day 20, mice were orally treated with 10 mg/kg of norathyriol, 10 mg/kg of mangiferin 8a, 10 mg/kg of tofacitinib (JAK inhibitor), 2 mg/kg of dexamethasone (steroid) and 10 mg/kg of olopatadine (antihistamine) daily for 8 consecutive days. Starting on day 21, 400 µg OVA was administered intranasally, and sneezing was measured on day 27. Note that tofacitinib, dexamethasone, and olopatadine are currently in clinical use in allergic rhinitis and were used as control drugs in this example.

The group that was just immunized with OVA is called the subject group, the one that received norathyriol is called the norathyriol group, the one that received mangiferin 8a is called the mangiferin 8a group, the one that received tofacitinib is called the tofacitinib group, the one that received dexamethasone is called the dexamethasone group, and the one that received olopatadine is called the olopatadine group. Each group consisted of five animals.

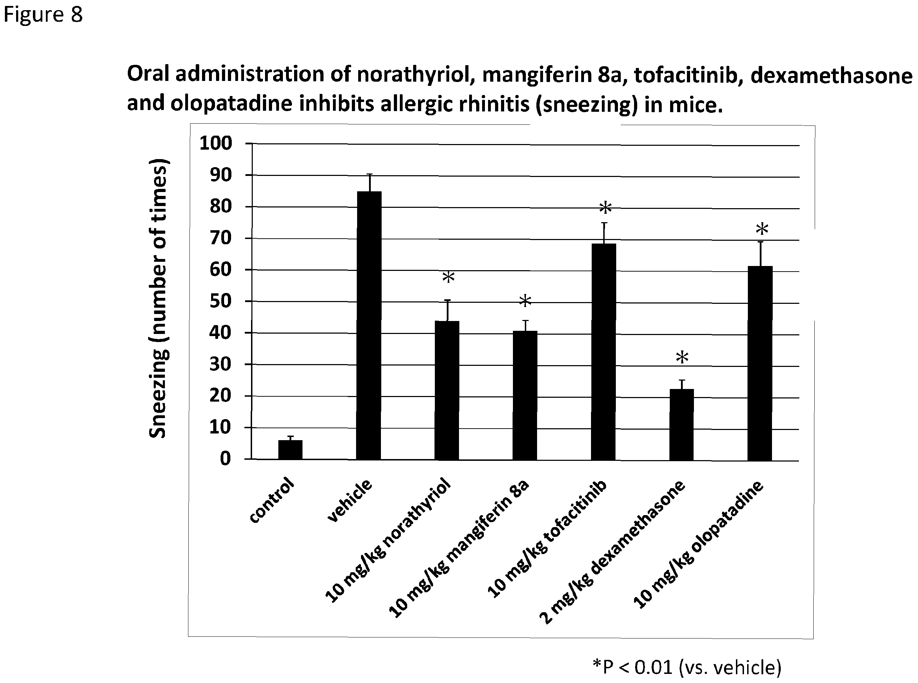

The results are shown in Figure 8. The horizontal axis represents the sample group and the vertical axis represents the number of sneezing. Compared to the OVA-immunized control (labeled "control"), the OVA-immunized subject group (labeled "vehicle") showed a marked increase in sneezing, indicating the development of allergic rhinitis. The norathyriol group ("10 mg/kg norathyriol"), mangiferin 8a group ("10 mg/kg mangiferin 8a"), tofacitinib group ("10 mg/kg tofacitinib"), dexamethasone group ("2 mg/kg dexamethasone"), and olopatadine group ("10 mg/kg olopatadine") significantly decreased the number of sneezing compared to the vehicle group. The norathyriol and mangiferin 8a groups significantly suppressed sneezing compared to the tofacitinib and olopatadine groups.

As described above, it was found that the norathyriol and mangiferin 8a treatment groups significantly suppressed sneezing caused by allergic rhinitis. In other words, compositions containing norathyriol or mangiferin 8a as active ingredients can suppress sneezing caused by allergic rhinitis.

### < Example 8: Inhibition of OVA-IgE antibody production >

Serum was collected from day 28 mice in Examples 6 and 7, and OVA-IgE antibody production was measured by enzyme-linked immunosorbent assay (ELISA). This assay was performed using a mouse OVA-IgE antibody ELISA kit (FUJIFILM Wako).

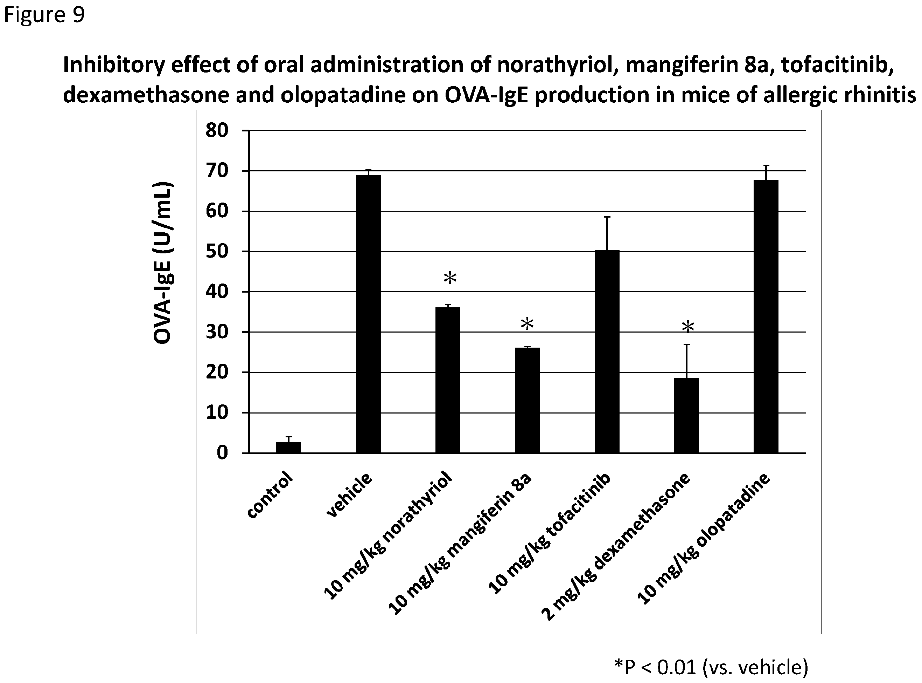

Results are shown in Figure 9. The horizontal axis represents the sample group and the vertical axis represents the amount of OVA-IgE antibody. The amount of OVA-IgE antibody in the OVA-immunized subject group (labeled "vehicle") was 69 U/mL, compared to 2.7 U/mL in the OVA-immunized control (labeled "control"), indicating that the subjects developed allergic rhinitis. The OVA-IgE antibody production was 36 U/mL in the norathyriol group ("10 mg/kg norathyriol"), 26 U/mL in the mangiferin 8a group ("10 mg/kg mangiferin 8a"), 50 U/mL in the tofacitinib group ("10 mg/kg tofacitinib"), 18 U/mL in the dexamethasone group ("2 mg/kg dexamethasone"), and 67 U/mL in the olopatadine group ("10 mg/kg olopatadine"), indicating that norathyriol group, mangiferin 8a group and dexamethasone groups significantly decreased OVA-IgE antibody production compared to the vehicle group. The norathyriol and mangiferin 8a groups significantly suppressed OVA-IgE antibody production compared to the tofacitinib and olopatadine groups.

As described above, it was found that the norathyriol and mangiferin 8a treatment groups markedly suppressed OVA-IgE antibody production caused by allergic rhinitis. In other words, compositions containing norathyriol or mangiferin 8a as active ingredients can suppress OVA-IgE antibody production caused by allergic rhinitis.

### < Example 9: Inhibition of IgE antibody production >

Serum was collected from day 28 mice in Examples 6 and 7, and IgE antibody production was measured by enzyme-linked immunosorbent assay (ELISA). This assay was performed using a mouse IgE antibody ELISA kit (R&D).

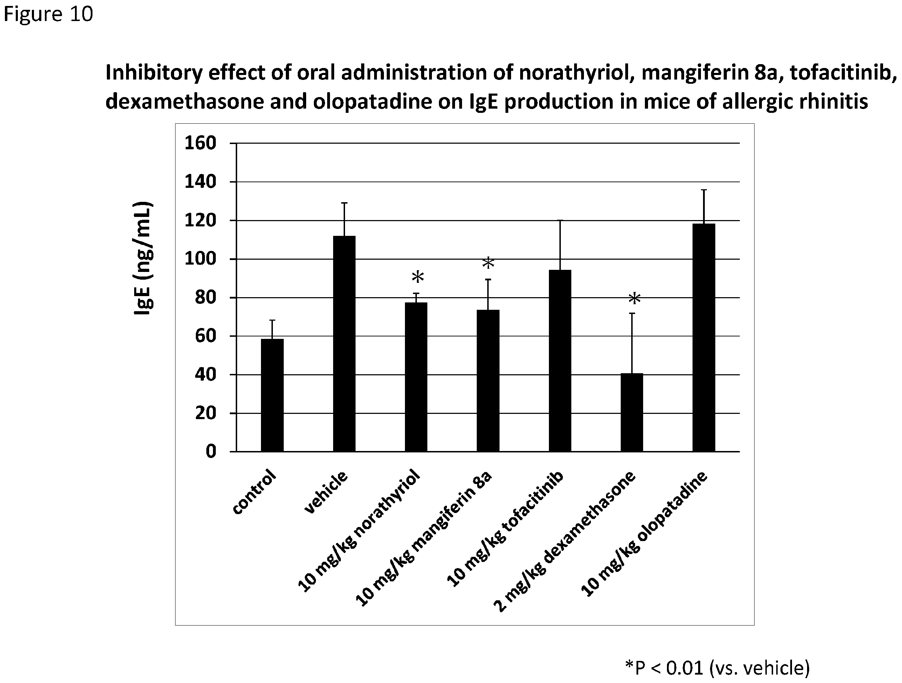

Results are shown in Figure 10. The horizontal axis represents the sample group and the vertical axis represents the amount of IgE antibody. The amount of IgE antibody in the OVA-immunized subject group (labeled "vehicle") was 111 ng/mL, compared to 58 ng/mL in the OVA-immunized control (labeled "control"), indicating that the subjects developed allergic rhinitis. The IgE antibody production was 77 ng/mL in the norathyriol group ("10 mg/kg norathyriol"), 73 ng/mL in the mangiferin 8a group ("10 mg/kg mangiferin 8a"), 94 ng/mL in the tofacitinib group ("10 mg/kg tofacitinib"), 40 ng/mL in the dexamethasone group ("2 mg/kg dexamethasone"), and 118 ng/mL in the olopatadine group ("10 mg/kg olopatadine"), indicating that norathyriol group, mangiferin 8a group and dexamethasone groups significantly decreased IgE antibody production compared to the vehicle group. The norathyriol and mangiferin 8a groups significantly suppressed IgE antibody production compared to the tofacitinib and olopatadine groups.

As described above, it was found that the norathyriol and mangiferin 8a treatment groups markedly suppressed IgE antibody production caused by allergic rhinitis. In other words, compositions containing norathyriol or mangiferin 8a as active ingredients can suppress IgE antibody production caused by allergic rhinitis.

### < Example 10: Inhibition of BAFF production >

Serum was collected from day 28 mice in Examples 6 and 7, and BAFF production was measured by enzyme-linked immunosorbent assay (ELISA). This assay was performed using a mouse BAFF ELISA kit (R&D).

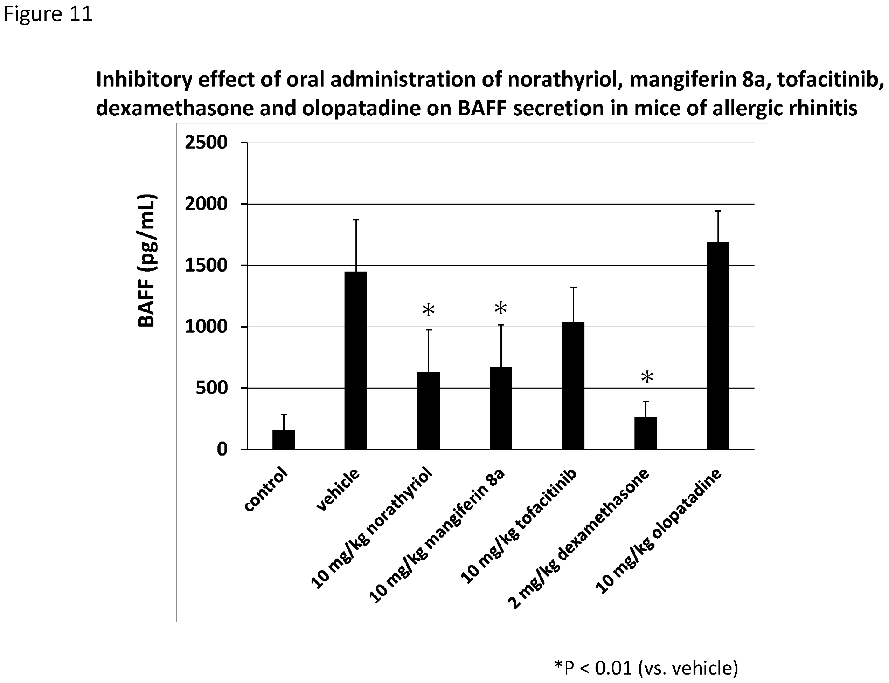

Results are shown in Figure 11. The horizontal axis represents the sample group and the vertical axis represents the amount of BAFF. The amount of BAFF in the OVA-immunized subject group (labeled "vehicle") was 1450 pg/mL, compared to 157 pg/mL in the OVA-immunized control (labeled "control"), indicating that the subjects developed allergic rhinitis. The BAFF production was 629 pg/mL in the norathyriol group ("10 mg/kg norathyriol"), 668 pg/mL in the mangiferin 8a group ("10 mg/kg mangiferin 8a"), 1041 pg/mL in the tofacitinib group ("10 mg/kg tofacitinib"), 266 pg/mL in the dexamethasone group ("2 mg/kg dexamethasone"), and 1691 pg/mL in the olopatadine group ("10 mg/kg olopatadine"), indicating that norathyriol group, mangiferin 8a group and dexamethasone groups significantly decreased BAFF production compared to the vehicle group. The norathyriol and mangiferin 8a groups significantly suppressed BAFF production compared to the tofacitinib and olopatadine groups.

As described above, it was found that the norathyriol and mangiferin 8a treatment groups markedly suppressed BAFF production caused by allergic rhinitis. In other words, compositions containing norathyriol or mangiferin 8a as active ingredients can suppress BAFF production caused by allergic rhinitis.

### < Example 11: Inhibition of IL-4 production >

Serum was collected from day 28 mice in Examples 6 and 7, and IL-4 production was measured by enzyme-linked immunosorbent assay (ELISA). This assay was performed using a mouse IL-4 ELISA kit (R&D).

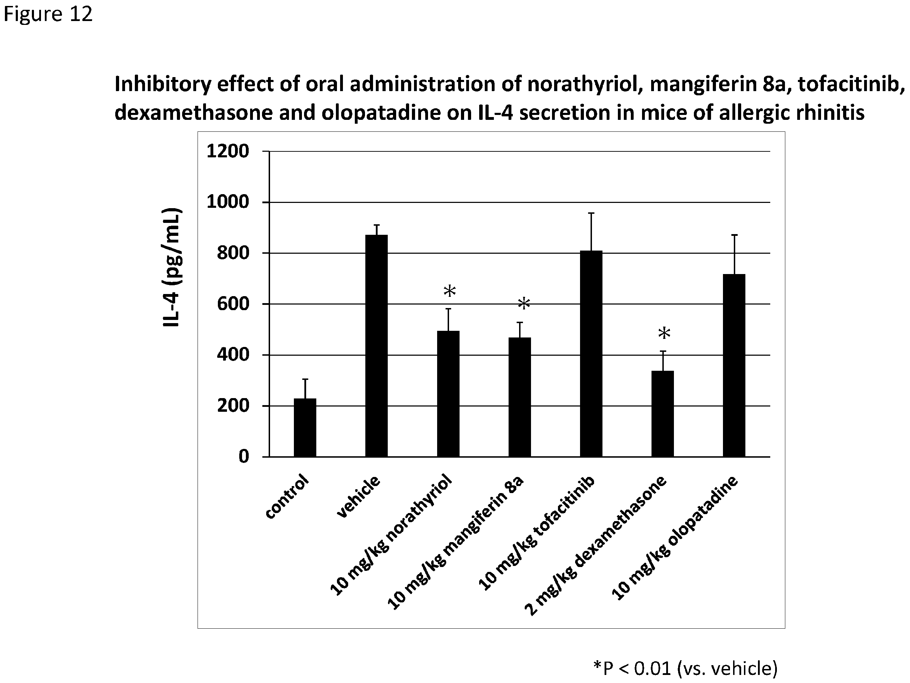

Results are shown in Figure 12. The horizontal axis represents the sample group and the vertical axis represents the amount of IL-4. The amount of IL-4 in the OVA-immunized subject group (labeled "vehicle") was 872 pg/mL, compared to 227 pg/mL in the OVA-immunized control (labeled "control"), indicating that the subjects developed allergic rhinitis. The IL-4 production was 495 pg/mL in the norathyriol group ("10 mg/kg norathyriol"), 468 pg/mL in the mangiferin 8a group ("10 mg/kg mangiferin 8a"), 808 pg/mL in the tofacitinib group ("10 mg/kg tofacitinib"), 336 pg/mL in the dexamethasone group ("2 mg/kg dexamethasone"), and 717 pg/mL in the olopatadine group ("10 mg/kg olopatadine"), indicating that norathyriol group, mangiferin 8a group and dexamethasone groups significantly decreased IL-4 production compared to the vehicle group. The norathyriol and mangiferin 8a groups significantly suppressed IL-4 production compared to the tofacitinib and olopatadine groups.

As described above, it was found that the norathyriol and mangiferin 8a treatment groups markedly suppressed IL-4 production caused by allergic rhinitis. In other words, compositions containing norathyriol or mangiferin 8a as active ingredients can suppress IL-4 production caused by allergic rhinitis.

### < Example 12: Inhibition of IL-5 production >

Serum was collected from day 28 mice in Examples 6 and 7, and IL-5 production was measured by enzyme-linked immunosorbent assay (ELISA). This assay was performed using a mouse IL-5 ELISA kit (R&D).

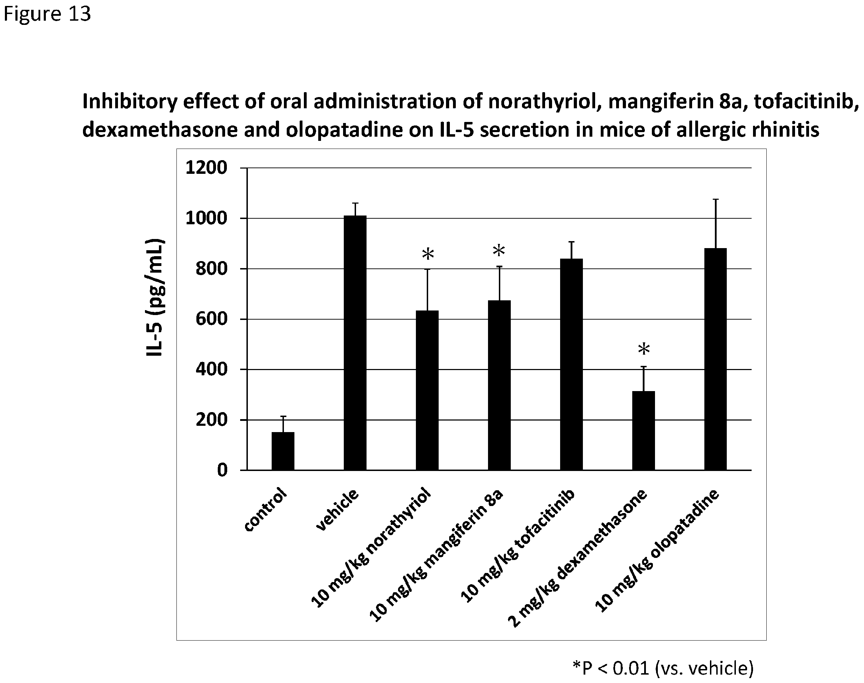

Results are shown in Figure 13. The horizontal axis represents the sample group and the vertical axis represents the amount of IL-5. The amount of IL-5 in the OVA-immunized subject group (labeled "vehicle") was 1010 pg/mL, compared to 151 pg/mL in the OVA-immunized control (labeled "control"), indicating that the subjects developed allergic rhinitis. The IL-5 production was 633 pg/mL in the norathyriol group ("10 mg/kg norathyriol"), 675 pg/mL in the mangiferin 8a group ("10 mg/kg mangiferin 8a"), 839 pg/mL in the tofacitinib group ("10 mg/kg tofacitinib"), 314 pg/mL in the dexamethasone group ("2 mg/kg dexamethasone"), and 880 pg/mL in the olopatadine group ("10 mg/kg olopatadine"), indicating that norathyriol group, mangiferin 8a group and dexamethasone groups significantly decreased IL-5 production compared to the vehicle group. The norathyriol and mangiferin 8a groups significantly suppressed IL-5 production compared to the tofacitinib and olopatadine groups.

As described above, it was found that the norathyriol and mangiferin 8a treatment groups markedly suppressed IL-5 production caused by allergic rhinitis. In other words, compositions containing norathyriol or mangiferin 8a as active ingredients can suppress IL-5 production caused by allergic rhinitis.

### < Example 13: Inhibition of IL-17 production >

Serum was collected from day 28 mice in Examples 6 and 7, and IL-17 production was measured by enzyme-linked immunosorbent assay (ELISA). This assay was performed using a mouse IL-17 ELISA kit (R&D).

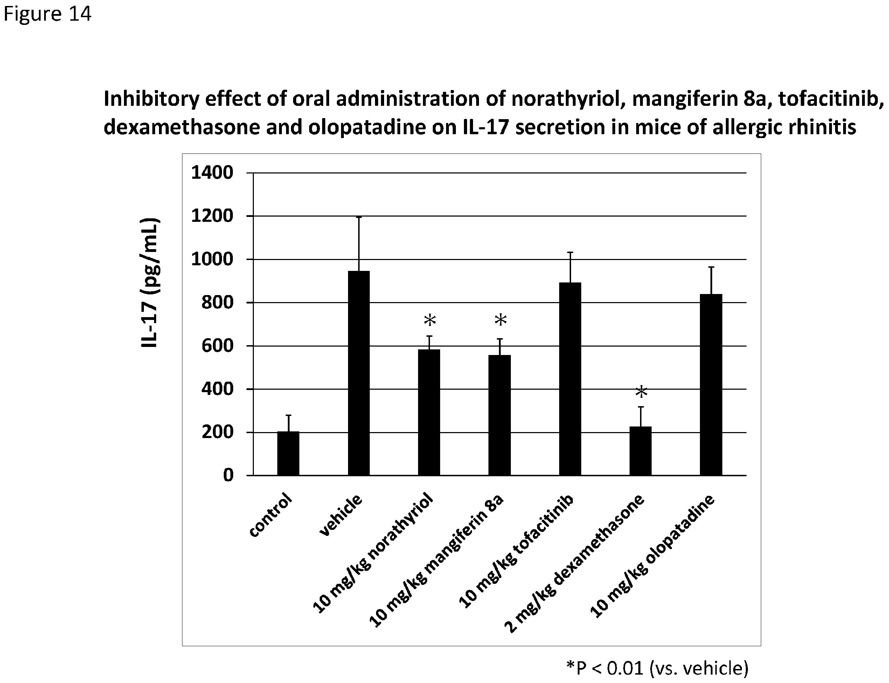

Results are shown in Figure 14. The horizontal axis represents the sample group and the vertical axis represents the amount of IL-17. The amount of IL-17 in the OVA-immunized subject group (labeled "vehicle") was 945 pg/mL, compared to 204 pg/mL in the OVA-immunized control (labeled "control"), indicating that the subjects developed allergic rhinitis. The IL-5 production was 583 pg/mL in the norathyriol group ("10 mg/kg norathyriol"), 557 pg/mL in the mangiferin 8a group ("10 mg/kg mangiferin 8a"), 892 pg/mL in the tofacitinib group ("10 mg/kg tofacitinib"), 227 pg/mL in the dexamethasone group ("2 mg/kg dexamethasone"), and 839 pg/mL in the olopatadine group ("10 mg/kg olopatadine"), indicating that norathyriol group, mangiferin 8a group and dexamethasone groups significantly decreased IL-17 production compared to the vehicle group. The norathyriol and mangiferin 8a groups significantly suppressed IL-17 production compared to the tofacitinib and olopatadine groups.

As described above, it was found that the norathyriol and mangiferin 8a treatment groups markedly suppressed IL-17 production caused by allergic rhinitis. In other words, compositions containing norathyriol or mangiferin 8a as active ingredients can suppress IL-17 production caused by allergic rhinitis.

In other words, the results of Examples 6-13 show that the subject group developed allergic rhinitis, while the norathyriol- and mangiferin 8a-treated groups suppressed allergic rhinitis. In other words, compositions with norathyriol or mangiferin 8a as active ingredients can inhibit allergic rhinitis.

### < Example 14: Cough suppression in a mouse model of ovalbumin-induced bronchial asthma>

A 0.2 mL dose of 50 µg ovalbumin (OVA) and 2 mg Alum mixture was administered to 6-week-old female Balb/c mice (Japan SLC Inc.) (day 0 of the example). Alum was used as an adjuvant. Seven days later and 14 days later, the Balb/c mice were immunized again with 0.2 mL injections of the above mixture. Starting on day 20, mice were orally treated with 10 mg/kg of norathyriol, 10 mg/kg of mangiferin 8a, 10 mg/kg of tofacitinib (JAK inhibitor), 2 mg/kg of dexamethasone (steroid) and 10 mg/kg of olopatadine (antihistamine) daily for 8 consecutive days. Starting on day 21, 400 µg OVA was administered intranasally, and cough was measured on day 27. Note that tofacitinib, dexamethasone, and olopatadine are currently in clinical use in allergic rhinitis and were used as control drugs in this example.

The group that was just immunized with OVA is called the subject group, the one that received norathyriol is called the norathyriol group, the one that received mangiferin 8a is called the mangiferin 8a group, the one that received tofacitinib is called the tofacitinib group, the one that received dexamethasone is called the dexamethasone group, and the one that received olopatadine is called the olopatadine group. Each group consisted of five animals.

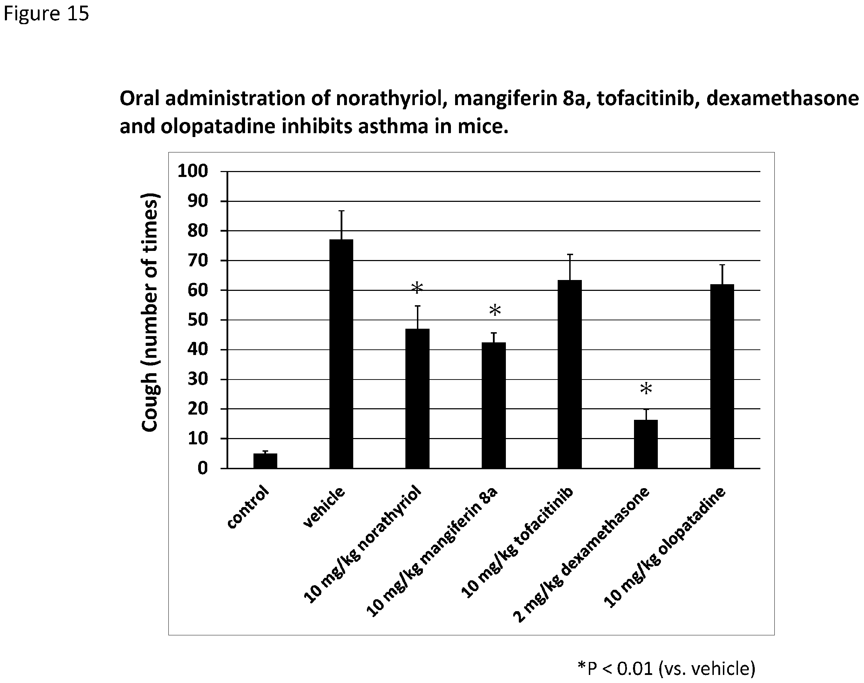

The results are shown in Figure 15. The horizontal axis represents the sample group and the vertical axis represents the number of cough. Compared to the OVA-immunized control (labeled "control"), the OVA-immunized subject group (labeled "vehicle") showed a marked increase in cough, indicating the development of bronchial asthma. The norathyriol group ("10 mg/kg norathyriol"), mangiferin 8a group ("10 mg/kg mangiferin 8a"), tofacitinib group ("10 mg/kg tofacitinib"), dexamethasone group ("2 mg/kg dexamethasone"), and olopatadine group ("10 mg/kg olopatadine") significantly decreased the number of coughs compared to the vehicle group. The norathyriol and mangiferin 8a groups significantly suppressed cough compared to the tofacitinib and olopatadine groups.

As described above, it was found that the norathyriol and mangiferin 8a treatment groups significantly suppressed cough caused by bronchial asthma. In other words, compositions containing norathyriol or mangiferin 8a as active ingredients can suppress cough caused by bronchial asthma.

### < Example 15: Inhibition of OVA-IgE antibody production >

Serum was collected from day 28 mice in Examples 14, and OVA-IgE antibody production was measured by enzyme-linked immunosorbent assay (ELISA). This assay was performed using a mouse OVA-IgE antibody ELISA kit (FUJIFILM Wako).

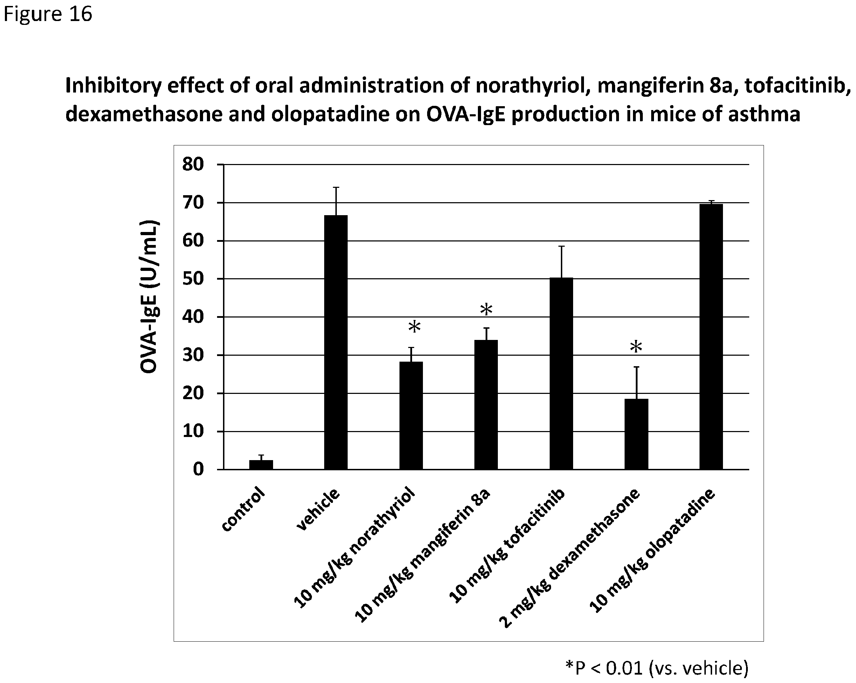

Results are shown in Figure 16. The horizontal axis represents the sample group and the vertical axis represents the amount of OVA-IgE antibody. The amount of OVA-IgE antibody in the OVA-immunized subject group (labeled "vehicle") was 66 U/mL, compared to 2.4 U/mL in the OVA-immunized control (labeled "control"), indicating that the subjects developed bronchial asthma. The OVA-IgE antibody production was 28 U/mL in the norathyriol group ("10 mg/kg norathyriol"), 34 U/mL in the mangiferin 8a group ("10 mg/kg mangiferin 8a"), 50 U/mL in the tofacitinib group ("10 mg/kg tofacitinib"), 18 U/mL in the dexamethasone group ("2 mg/kg dexamethasone"), and 69 U/mL in the olopatadine group ("10 mg/kg olopatadine"), indicating that norathyriol group, mangiferin 8a group and dexamethasone groups significantly decreased OVA-IgE antibody production compared to the vehicle group. The norathyriol and mangiferin 8a groups significantly suppressed OVA-IgE antibody production compared to the tofacitinib and olopatadine groups.

As described above, it was found that the norathyriol and mangiferin 8a treatment groups markedly suppressed OVA-IgE antibody production caused by bronchial asthma. In other words, compositions containing norathyriol or mangiferin 8a as active ingredients can suppress OVA-IgE antibody production caused by bronchial asthma.

### < Example 16: Inhibition of IgE antibody production >

Serum was collected from day 28 mice in Examples 14, and IgE antibody production was measured by enzyme-linked immunosorbent assay (ELISA). This assay was performed using a mouse IgE antibody ELISA kit (R&D).

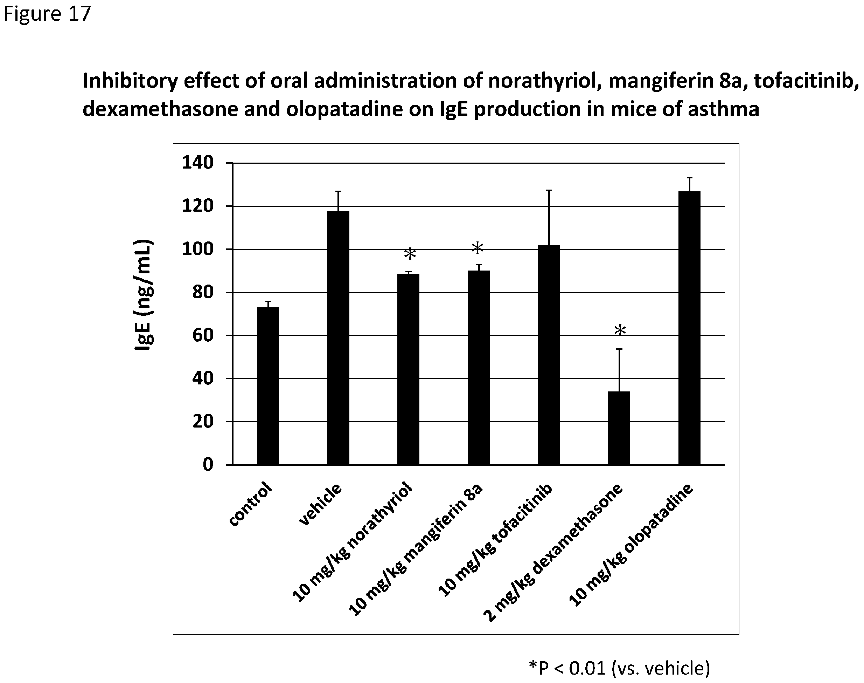

Results are shown in Figure 17. The horizontal axis represents the sample group and the vertical axis represents the amount of IgE antibody. The amount of IgE antibody in the OVA-immunized subject group (labeled "vehicle") was 117 ng/mL, compared to 73 ng/mL in the OVA-immunized control (labeled "control"), indicating that the subjects developed bronchial asthma. The IgE antibody production was 88 ng/mL in the norathyriol group ("10 mg/kg norathyriol"), 90 ng/mL in the mangiferin 8a group ("10 mg/kg mangiferin 8a"), 101 ng/mL in the tofacitinib group ("10 mg/kg tofacitinib"), 34 ng/mL in the dexamethasone group ("2 mg/kg dexamethasone"), and 126 ng/mL in the olopatadine group ("10 mg/kg olopatadine"), indicating that norathyriol group, mangiferin 8a group and dexamethasone groups significantly decreased IgE antibody production compared to the vehicle group. The norathyriol and mangiferin 8a groups significantly suppressed IgE antibody production compared to the tofacitinib and olopatadine groups.

As described above, it was found that the norathyriol and mangiferin 8a treatment groups markedly suppressed IgE antibody production caused by bronchial asthma. In other words, compositions containing norathyriol or mangiferin 8a as active ingredients can suppress IgE antibody production caused by bronchial asthma.

### < Example 17: Inhibition of BAFF production >

Serum was collected from day 28 mice in Examples 14, and BAFF production was measured by enzyme-linked immunosorbent assay (ELISA). This assay was performed using a mouse BAFF ELISA kit (R&D).

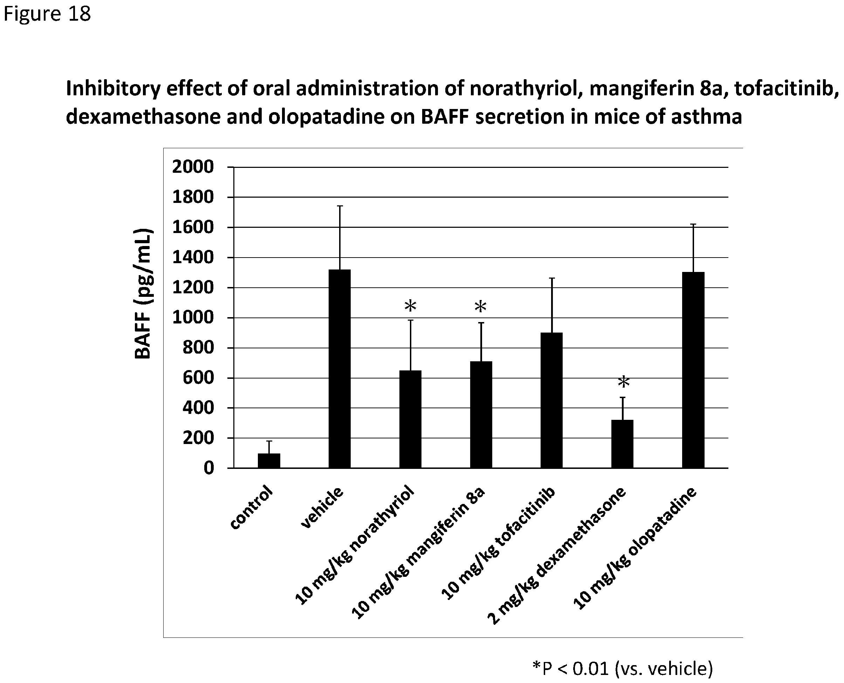

Results are shown in Figure 18. The horizontal axis represents the sample group and the vertical axis represents the amount of BAFF. The amount of BAFF in the OVA-immunized subject group (labeled "vehicle") was 1319 pg/mL, compared to 97 pg/mL in the OVA-immunized control (labeled "control"), indicating that the subjects developed bronchial asthma. The BAFF production was 649 pg/mL in the norathyriol group ("10 mg/kg norathyriol"), 708 pg/mL in the mangiferin 8a group ("10 mg/kg mangiferin 8a"), 902 pg/mL in the tofacitinib group ("10 mg/kg tofacitinib"), 321 pg/mL in the dexamethasone group ("2 mg/kg dexamethasone"), and 1304 pg/mL in the olopatadine group ("10 mg/kg olopatadine"), indicating that norathyriol group, mangiferin 8a group and dexamethasone groups significantly decreased BAFF production compared to the vehicle group. The norathyriol and mangiferin 8a groups significantly suppressed BAFF production compared to the tofacitinib and olopatadine groups.

As described above, it was found that the norathyriol and mangiferin 8a treatment groups markedly suppressed BAFF production caused by bronchial asthma. In other words, compositions containing norathyriol or mangiferin 8a as active ingredients can suppress BAFF production caused by bronchial asthma.

### < Example 18: Inhibition of IL-4 production >

Serum was collected from day 28 mice in Examples 14, and IL-4 production was measured by enzyme-linked immunosorbent assay (ELISA). This assay was performed using a mouse IL-4 ELISA kit (R&D).

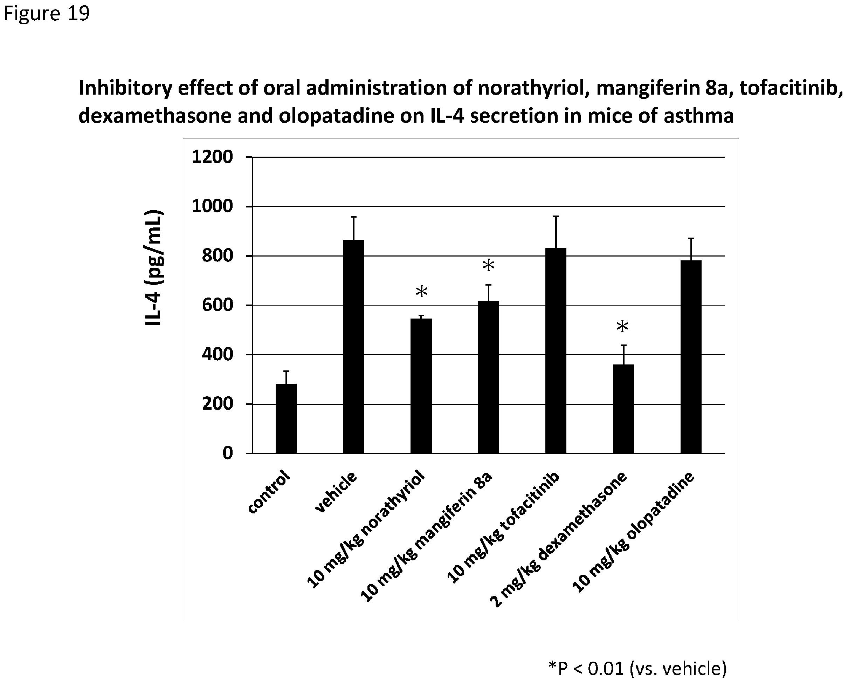

Results are shown in Figure 19. The horizontal axis represents the sample group and the vertical axis represents the amount of IL-4. The amount of IL-4 in the OVA-immunized subject group (labeled "vehicle") was 863 pg/mL, compared to 282 pg/mL in the OVA-immunized control (labeled "control"), indicating that the subjects developed bronchial asthma. The IL-4 production was 545 pg/mL in the norathyriol group ("10 mg/kg norathyriol"), 617 pg/mL in the mangiferin 8a group ("10 mg/kg mangiferin 8a"), 831 pg/mL in the tofacitinib group ("10 mg/kg tofacitinib"), 359 pg/mL in the dexamethasone group ("2 mg/kg dexamethasone"), and 781 pg/mL in the olopatadine group ("10 mg/kg olopatadine"), indicating that norathyriol group, mangiferin 8a group and dexamethasone groups significantly decreased IL-4 production compared to the vehicle group. The norathyriol and mangiferin 8a groups significantly suppressed IL-4 production compared to the tofacitinib and olopatadine groups.

As described above, it was found that the norathyriol and mangiferin 8a treatment groups markedly suppressed IL-4 production caused by bronchial asthma. In other words, compositions containing norathyriol or mangiferin 8a as active ingredients can suppress IL-4 production caused by bronchial asthma.

### < Example 19: Inhibition of IL-5 production >

Serum was collected from day 28 mice in Examples 14, and IL-5 production was measured by enzyme-linked immunosorbent assay (ELISA). This assay was performed using a mouse IL-5 ELISA kit (R&D).

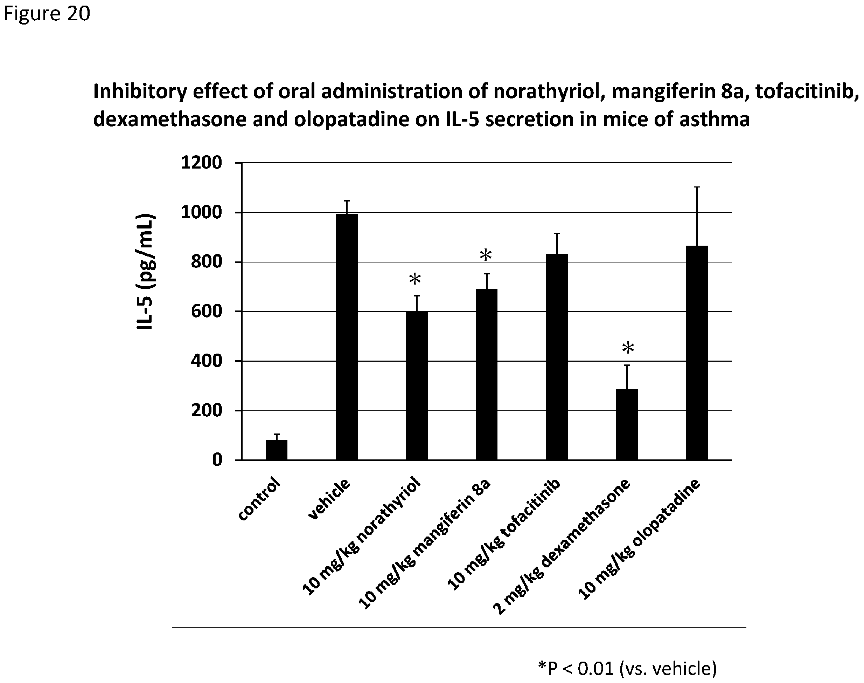

Results are shown in Figure 20. The horizontal axis represents the sample group and the vertical axis represents the amount of IL-5. The amount of IL-5 in the OVA-immunized subject group (labeled "vehicle") was 992 pg/mL, compared to 80 pg/mL in the OVA-immunized control (labeled "control"), indicating that the subjects developed bronchial asthma. The IL-5 production was 601 pg/mL in the norathyriol group ("10 mg/kg norathyriol"), 689 pg/mL in the mangiferin 8a group ("10 mg/kg mangiferin 8a"), 833 pg/mL in the tofacitinib group ("10 mg/kg tofacitinib"), 286 pg/mL in the dexamethasone group ("2 mg/kg dexamethasone"), and 866 pg/mL in the olopatadine group ("10 mg/kg olopatadine"), indicating that norathyriol group, mangiferin 8a group and dexamethasone groups significantly decreased IL-5 production compared to the vehicle group. The norathyriol and mangiferin 8a groups significantly suppressed IL-5 production compared to the tofacitinib and olopatadine groups.

As described above, it was found that the norathyriol and mangiferin 8a treatment groups markedly suppressed IL-5 production caused by bronchial asthma. In other words, compositions containing norathyriol or mangiferin 8a as active ingredients can suppress IL-5 production caused by bronchial asthma.

### < Example 20: Inhibition of IL-17 production >

Serum was collected from day 28 mice in Examples 14, and IL-17 production was measured by enzyme-linked immunosorbent assay (ELISA). This assay was performed using a mouse IL-17 ELISA kit (R&D).

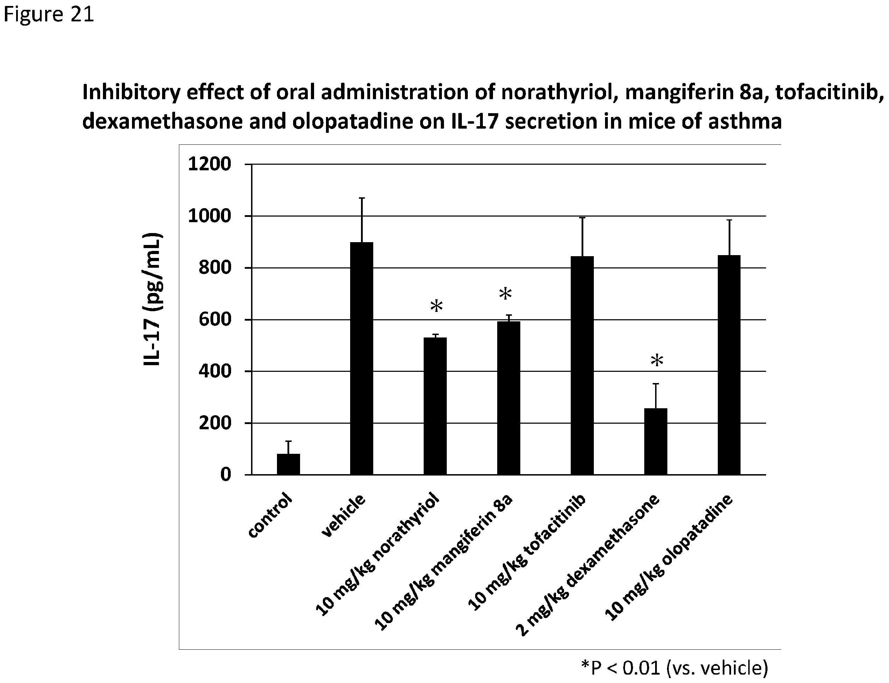

Results are shown in Figure 21. The horizontal axis represents the sample group and the vertical axis represents the amount of IL-17. The amount of IL-17 in the OVA-immunized subject group (labeled "vehicle") was 898 pg/mL, compared to 80 pg/mL in the OVA-immunized control (labeled "control"), indicating that the subjects developed bronchial asthma. The IL-5 production was 530 pg/mL in the norathyriol group ("10 mg/kg norathyriol"), 592 pg/mL in the mangiferin 8a group ("10 mg/kg mangiferin 8a"), 844 pg/mL in the tofacitinib group ("10 mg/kg tofacitinib"), 257 pg/mL in the dexamethasone group ("2 mg/kg dexamethasone"), and 848 pg/mL in the olopatadine group ("10 mg/kg olopatadine"), indicating that norathyriol group, mangiferin 8a group and dexamethasone groups significantly decreased IL-17 production compared to the vehicle group. The norathyriol and mangiferin 8a groups significantly suppressed IL-17 production compared to the tofacitinib and olopatadine groups.

As described above, it was found that the norathyriol and mangiferin 8a treatment groups markedly suppressed IL-17 production caused by bronchial asthma. In other words, compositions containing norathyriol or mangiferin 8a as active ingredients can suppress IL-17 production caused by bronchial asthma.

In other words, the results of Examples 14-20 show that the subject group developed bronchial asthma, while the norathyriol- and mangiferin 8a-treated groups suppressed bronchial asthma. In other words, compositions with norathyriol or mangiferin 8a as active ingredients can inhibit bronchial asthma.

### < Example 21: Suppression of diarrhea in a mouse model of ovalbumin-induced food allergy >

A 0.2 mL dose of 10 µg ovalbumin (OVA) and 1 mg Alum mixture was administered to 6-week-old female Balb/c mice (Japan SLC Inc.) (day 0 of the example). Alum was used as an adjuvant. Seven days later, the Balb/c mice were immunized again with a 0.2 mL injection of the above mixture. Starting on day 14, mice were orally treated with 10 mg/kg of norathyriol, 10 mg/kg of mangiferin 8a, 10 mg/kg of tofacitinib (JAK inhibitor), 2 mg/kg of dexamethasone (steroid) and 10 mg/kg of olopatadine (antihistamine) daily for 14 consecutive days. Starting on day 14, 10 mg OVA was administered orally every other day until day 21, and the frequency of diarrhea was measured after the final administration on day 28. Note that tofacitinib, dexamethasone, and olopatadine are currently in clinical use in food allergy and were used as control drugs in this example.

The group that was just immunized with OVA is called the subject group, the one that received norathyriol is called the norathyriol group, the one that received mangiferin 8a is called the mangiferin 8a group, the one that received tofacitinib is called the tofacitinib group, the one that received dexamethasone is called the dexamethasone group, and the one that received olopatadine is called the olopatadine group. Each group consisted of five animals.

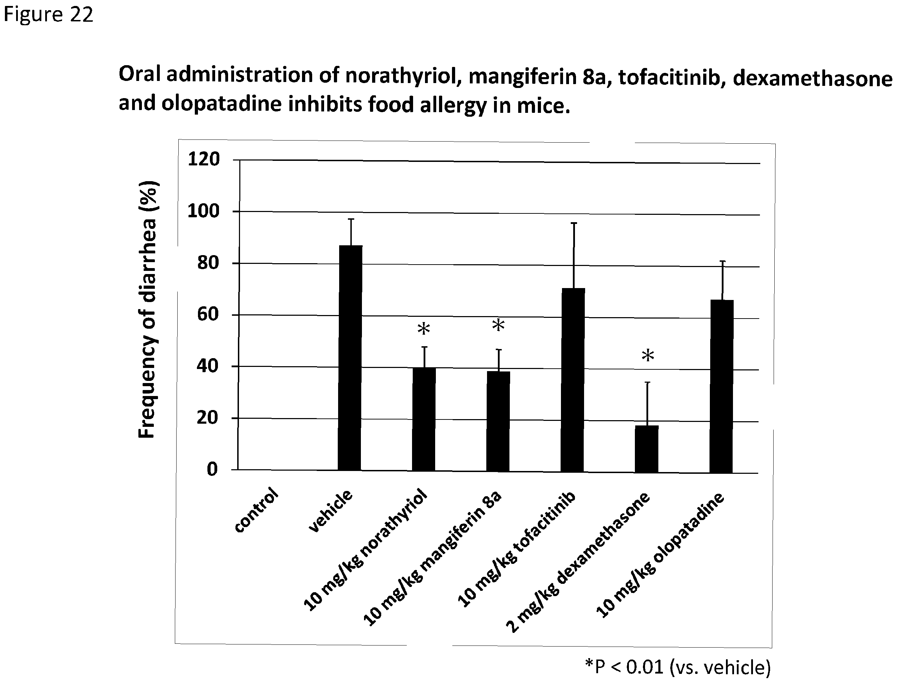

The results are shown in Figure 22. The horizontal axis represents the sample group and the vertical axis represents the frequency (%) of diarrhea. The frequency of diarrhea is markedly increased in the subject group immunized with OVA (labeled "vehicle"), indicating that they have developed food allergy. The norathyriol group ("10 mg/kg norathyriol"), the mangiferin 8a group ("10 mg/kg mangiferin 8a"), and the dexamethasone group ("2 mg/kg dexamethasone") showed a marked decrease in the frequency of diarrhea compared to the vehicle group. The norathyriol and mangiferin 8a groups were also significantly decreased in diarrhea compared to the tofacitinib group ("10 mg/kg tofacitinib") and olopatadine group ("10 mg/kg olopatadine").

As described above, it was found that norathyriol and mangiferin 8a significantly suppressed diarrhea caused by food allergy. In other words, compositions containing norathyriol or mangiferin 8a as active ingredients can suppress diarrhea caused by food allergy.

### < Example 22: Inhibition of OVA-IgE antibody production >

Serum was collected from day 28 mice in Examples 21, and OVA-IgE antibody production was measured by enzyme-linked immunosorbent assay (ELISA). This assay was performed using a mouse OVA-IgE antibody ELISA kit (FUJIFILM Wako).

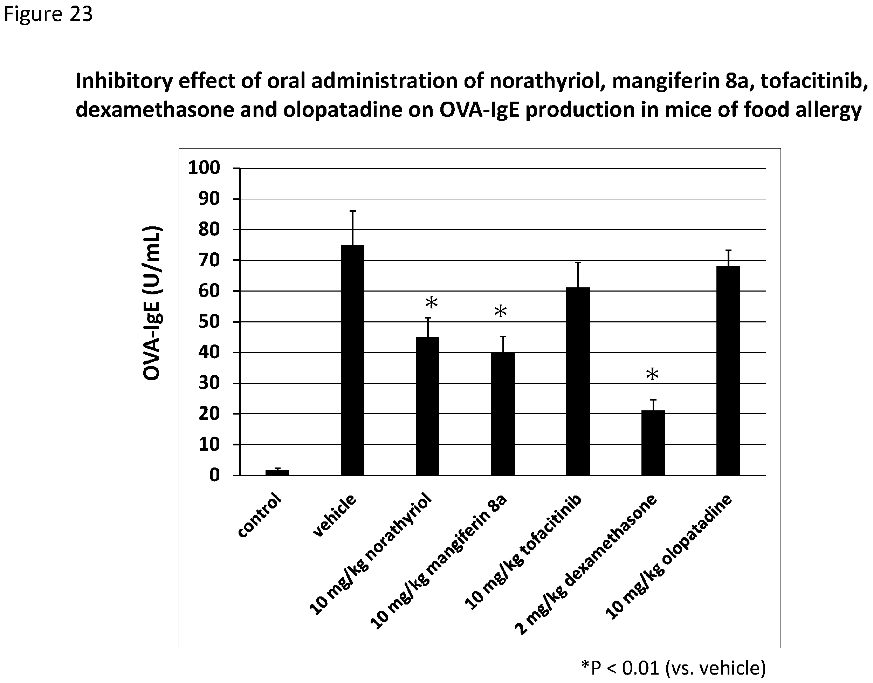

Results are shown in Figure 23. The horizontal axis represents the sample group and the vertical axis represents the amount of OVA-IgE antibody. The amount of OVA-IgE antibody in the OVA-immunized subject group (labeled "vehicle") was 74 U/mL, compared to 1.5 U/mL in the OVA-immunized control (labeled "control"), indicating that the subjects developed food allergy. The OVA-IgE antibody production was 45 U/mL in the norathyriol group ("10 mg/kg norathyriol"), 40 U/mL in the mangiferin 8a group ("10 mg/kg mangiferin 8a"), 61 U/mL in the tofacitinib group ("10 mg/kg tofacitinib"), 21 U/mL in the dexamethasone group ("2 mg/kg dexamethasone"), and 68 U/mL in the olopatadine group ("10 mg/kg olopatadine"), indicating that norathyriol group, mangiferin 8a group and dexamethasone groups significantly decreased OVA-IgE antibody production compared to the vehicle group. The norathyriol and mangiferin 8a groups significantly suppressed OVA-IgE antibody production compared to the tofacitinib and olopatadine groups.

As described above, it was found that the norathyriol and mangiferin 8a treatment groups markedly suppressed OVA-IgE antibody production caused by food allergy. In other words, compositions containing norathyriol or mangiferin 8a as active ingredients can suppress OVA-IgE antibody production caused by food allergy.

### < Example 23: Inhibition of IgE antibody production >

Serum was collected from day 28 mice in Examples 21, and IgE antibody production was measured by enzyme-linked immunosorbent assay (ELISA). This assay was performed using a mouse IgE antibody ELISA kit (R&D).

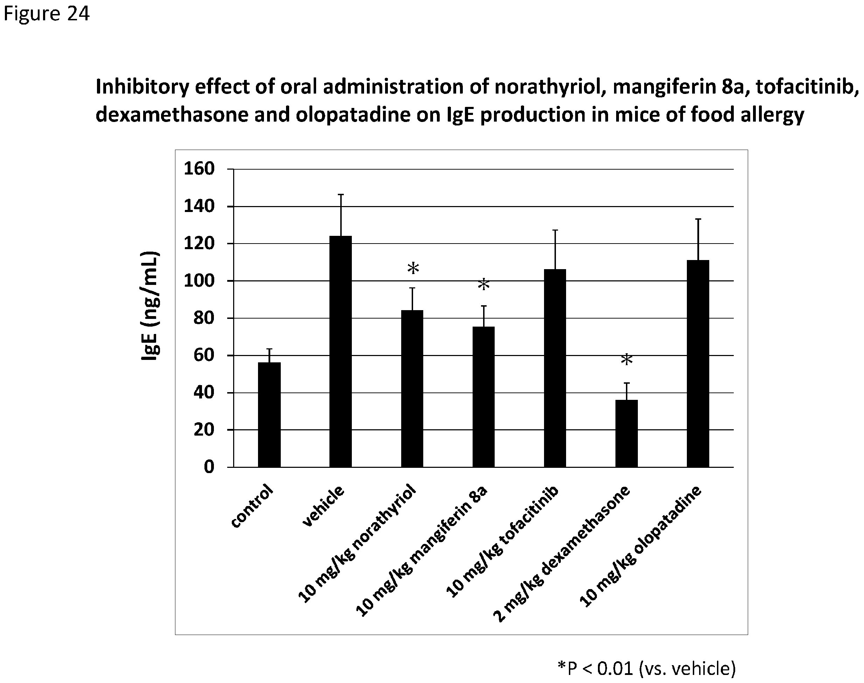

Results are shown in Figure 24. The horizontal axis represents the sample group and the vertical axis represents the amount of IgE antibody. The amount of IgE antibody in the OVA-immunized subject group (labeled "vehicle") was 124 ng/mL, compared to 56 ng/mL in the OVA-immunized control (labeled "control"), indicating that the subjects developed food allergy. The IgE antibody production was 84 ng/mL in the norathyriol group ("10 mg/kg norathyriol"), 75 ng/mL in the mangiferin 8a group ("10 mg/kg mangiferin 8a"), 106 ng/mL in the tofacitinib group ("10 mg/kg tofacitinib"), 36 ng/mL in the dexamethasone group ("2 mg/kg dexamethasone"), and 111 ng/mL in the olopatadine group ("10 mg/kg olopatadine"), indicating that norathyriol group, mangiferin 8a group and dexamethasone groups significantly decreased IgE antibody production compared to the vehicle group. The norathyriol and mangiferin 8a groups significantly suppressed IgE antibody production compared to the tofacitinib and olopatadine groups.

As described above, it was found that the norathyriol and mangiferin 8a treatment groups markedly suppressed IgE antibody production caused by food allergy. In other words, compositions containing norathyriol or mangiferin 8a as active ingredients can suppress IgE antibody production caused by food allergy.

### < Example 24: Inhibition of BAFF production >

Serum was collected from day 28 mice in Examples 21, and BAFF production was measured by enzyme-linked immunosorbent assay (ELISA). This assay was performed using a mouse BAFF ELISA kit (R&D).

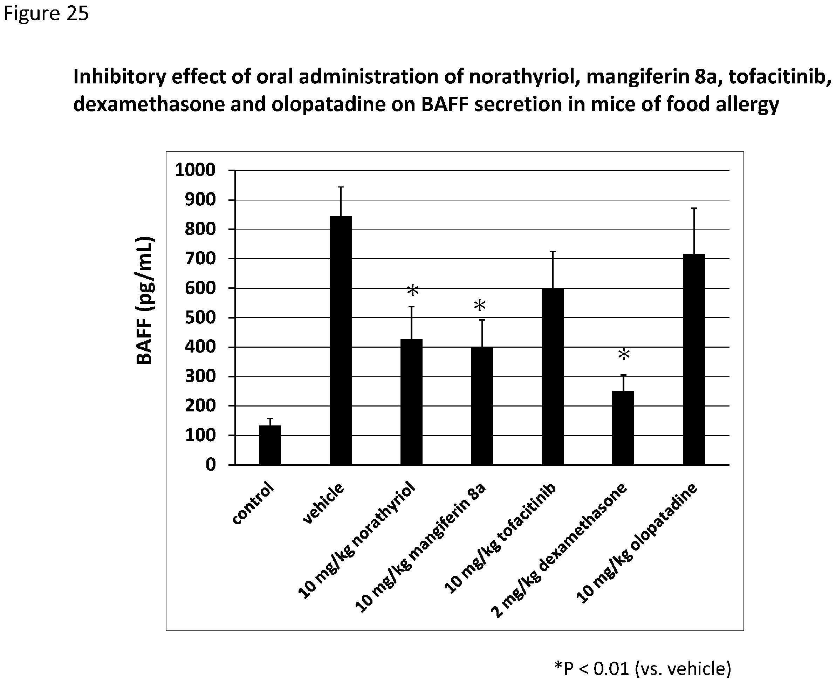

Results are shown in Figure 25. The horizontal axis represents the sample group and the vertical axis represents the amount of BAFF. The amount of BAFF in the OVA-immunized subject group (labeled "vehicle") was 845 pg/mL, compared to 134 pg/mL in the OVA-immunized control (labeled "control"), indicating that the subjects developed food allergy. The BAFF production was 426 pg/mL in the norathyriol group ("10 mg/kg norathyriol"), 398 pg/mL in the mangiferin 8a group ("10 mg/kg mangiferin 8a"), 598 pg/mL in the tofacitinib group ("10 mg/kg tofacitinib"), 251 pg/mL in the dexamethasone group ("2 mg/kg dexamethasone"), and 715 pg/mL in the olopatadine group ("10 mg/kg olopatadine"), indicating that norathyriol group, mangiferin 8a group and dexamethasone groups significantly decreased BAFF production compared to the vehicle group. The norathyriol and mangiferin 8a groups significantly suppressed BAFF production compared to the tofacitinib and olopatadine groups.

As described above, it was found that the norathyriol and mangiferin 8a treatment groups markedly suppressed BAFF production caused by food allergy. In other words, compositions containing norathyriol or mangiferin 8a as active ingredients can suppress BAFF production caused by food allergy.

### < Example 25: Inhibition of IL-4 production >

Serum was collected from day 28 mice in Examples 21, and IL-4 production was measured by enzyme-linked immunosorbent assay (ELISA). This assay was performed using a mouse IL-4 ELISA kit (R&D).

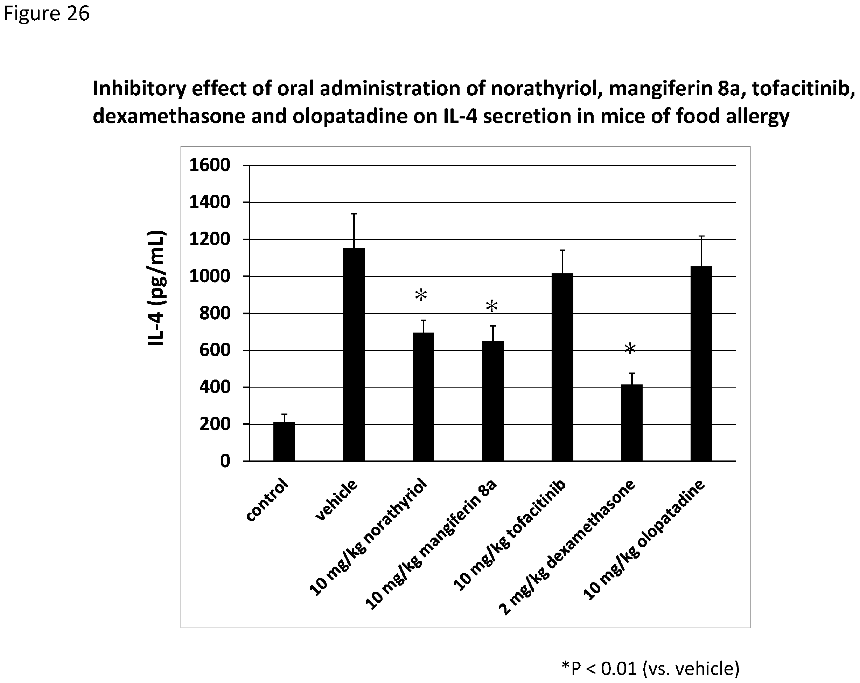

Results are shown in Figure 26. The horizontal axis represents the sample group and the vertical axis represents the amount of IL-4. The amount of IL-4 in the OVA-immunized subject group (labeled "vehicle") was 1154 pg/mL, compared to 210 pg/mL in the OVA-immunized control (labeled "control"), indicating that the subjects developed food allergy. The IL-4 production was 694 pg/mL in the norathyriol group ("10 mg/kg norathyriol"), 648 pg/mL in the mangiferin 8a group ("10 mg/kg mangiferin 8a"), 1015 pg/mL in the tofacitinib group ("10 mg/kg tofacitinib"), 415 pg/mL in the dexamethasone group ("2 mg/kg dexamethasone"), and 1054 pg/mL in the olopatadine group ("10 mg/kg olopatadine"), indicating that norathyriol group, mangiferin 8a group and dexamethasone groups significantly decreased IL-4 production compared to the vehicle group. The norathyriol and mangiferin 8a groups significantly suppressed IL-4 production compared to the tofacitinib and olopatadine groups.

As described above, it was found that the norathyriol and mangiferin 8a treatment groups markedly suppressed IL-4 production caused by food allergy. In other words, compositions containing norathyriol or mangiferin 8a as active ingredients can suppress IL-4 production caused by food allergy.

### < Example 26: Inhibition of IL-5 production >

Serum was collected from day 28 mice in Examples 21, and IL-5 production was measured by enzyme-linked immunosorbent assay (ELISA). This assay was performed using a mouse IL-5 ELISA kit (R&D).

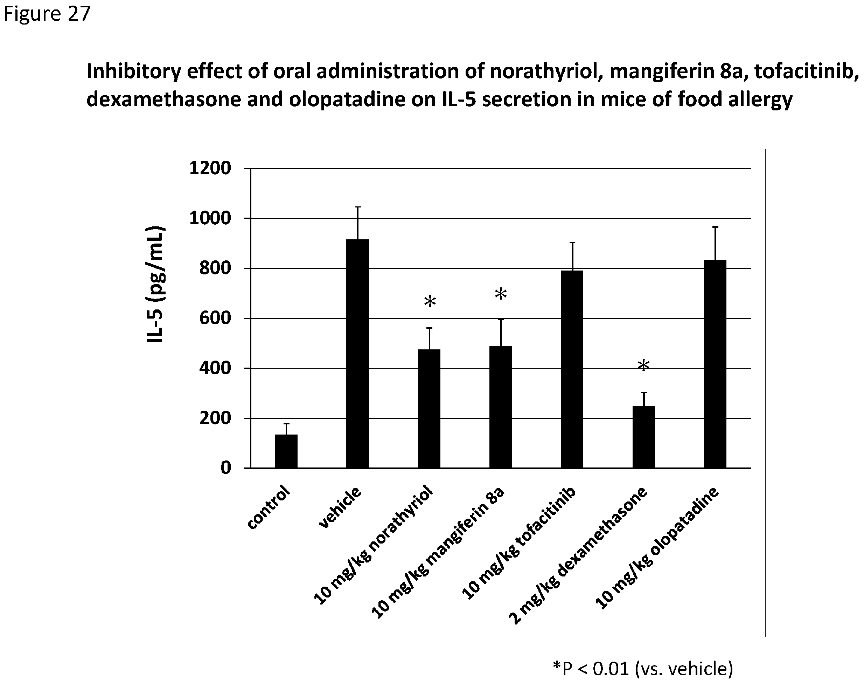

Results are shown in Figure 27. The horizontal axis represents the sample group and the vertical axis represents the amount of IL-5. The amount of IL-5 in the OVA-immunized subject group (labeled "vehicle") was 916 pg/mL, compared to 135 pg/mL in the OVA-immunized control (labeled "control"), indicating that the subjects developed food allergy. The IL-5 production was 475 pg/mL in the norathyriol group ("10 mg/kg norathyriol"), 487 pg/mL in the mangiferin 8a group ("10 mg/kg mangiferin 8a"), 791 pg/mL in the tofacitinib group ("10 mg/kg tofacitinib"), 249 pg/mL in the dexamethasone group ("2 mg/kg dexamethasone"), and 832 pg/mL in the olopatadine group ("10 mg/kg olopatadine"), indicating that norathyriol group, mangiferin 8a group and dexamethasone groups significantly decreased IL-5 production compared to the vehicle group. The norathyriol and mangiferin 8a groups significantly suppressed IL-5 production compared to the tofacitinib and olopatadine groups.

As described above, it was found that the norathyriol and mangiferin 8a treatment groups markedly suppressed IL-5 production caused by food allergy. In other words, compositions containing norathyriol or mangiferin 8a as active ingredients can suppress IL-5 production caused by food allergy.

### < Example 27: Inhibition of IL-17 production >

Serum was collected from day 28 mice in Examples 21, and IL-17 production was measured by enzyme-linked immunosorbent assay (ELISA). This assay was performed using a mouse IL-17 ELISA kit (R&D).

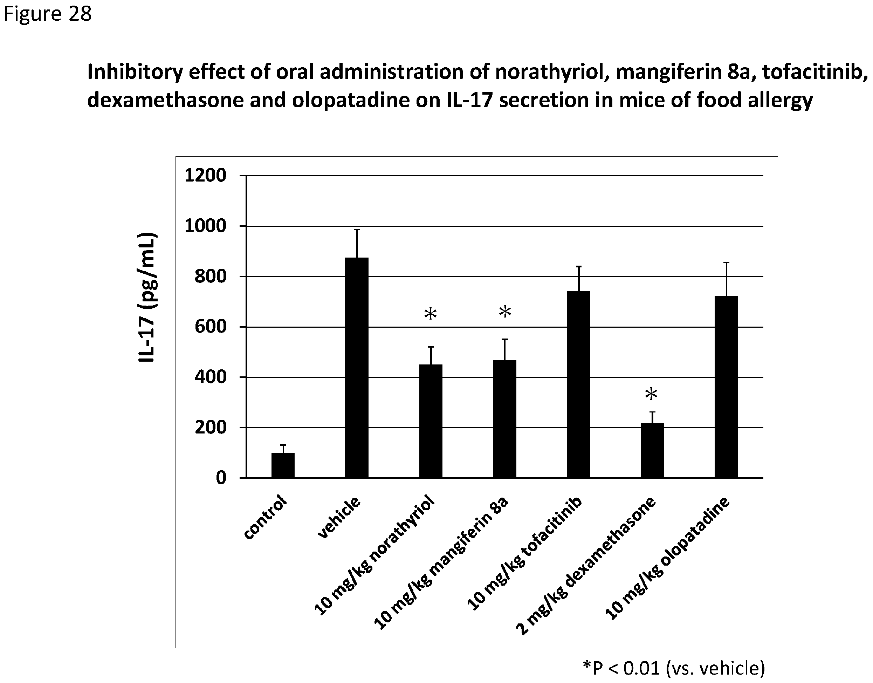

Results are shown in Figure 28. The horizontal axis represents the sample group and the vertical axis represents the amount of IL-17. The amount of IL-17 in the OVA-immunized subject group (labeled "vehicle") was 875 pg/mL, compared to 98 pg/mL in the OVA-immunized control (labeled "control"), indicating that the subjects developed food allergy. The IL-5 production was 451 pg/mL in the norathyriol group ("10 mg/kg norathyriol"), 467 pg/mL in the mangiferin 8a group ("10 mg/kg mangiferin 8a"), 741 pg/mL in the tofacitinib group ("10 mg/kg tofacitinib"), 216 pg/mL in the dexamethasone group ("2 mg/kg dexamethasone"), and 721 pg/mL in the olopatadine group ("10 mg/kg olopatadine"), indicating that norathyriol group, mangiferin 8a group and dexamethasone groups significantly decreased IL-17 production compared to the vehicle group. The norathyriol and mangiferin 8a groups significantly suppressed IL-17 production compared to the tofacitinib and olopatadine groups.

As described above, it was found that the norathyriol and mangiferin 8a treatment groups markedly suppressed IL-17 production caused by food allergy. In other words, compositions containing norathyriol or mangiferin 8a as active ingredients can suppress IL-17 production caused by food allergy.

In other words, the results of Examples 21-27 show that the subject group developed food allergy, while the norathyriol- and mangiferin 8a-treated groups suppressed food allergy. In other words, compositions with norathyriol or mangiferin 8a as active ingredients can inhibit food allergy.

### < Example 28: Suppression of rhinitis (nose scratching behavior) in cedar pollinosis model mice >

A 0.2 mL dose of 100 µg cedar pollen and 1 mg Alum mixture was administered to 6-week-old female Balb/c mice (Japan SLC Inc.) (day 0 of the example). Alum was used as an adjuvant. Seven days later and 14 days later, the Balb/c mice were immunized again with 0.2 mL injections of the above mixture. Starting on day 20, mice were orally treated with 10 mg/kg of norathyriol, 10 mg/kg of mangiferin 8a, 10 mg/kg of tofacitinib (JAK inhibitor), 2 mg/kg of dexamethasone (steroid) and 10 mg/kg of olopatadine (antihistamine) daily for 8 consecutive days. Starting on day 21, 500 µg cedar pollen was administered intranasally, and nose-scratching behavior was measured on day 27. Note that tofacitinib, dexamethasone, and olopatadine are currently in clinical use in cedar pollinosis and were used as control drugs in this example.

The group that was just immunized with cedar pollen is called the subject group, the one that received norathyriol is called the norathyriol group, the one that received mangiferin 8a is called the mangiferin 8a group, the one that received tofacitinib is called the tofacitinib group, the one that received dexamethasone is called the dexamethasone group, and the one that received olopatadine is called the olopatadine group. Each group consisted of five animals.

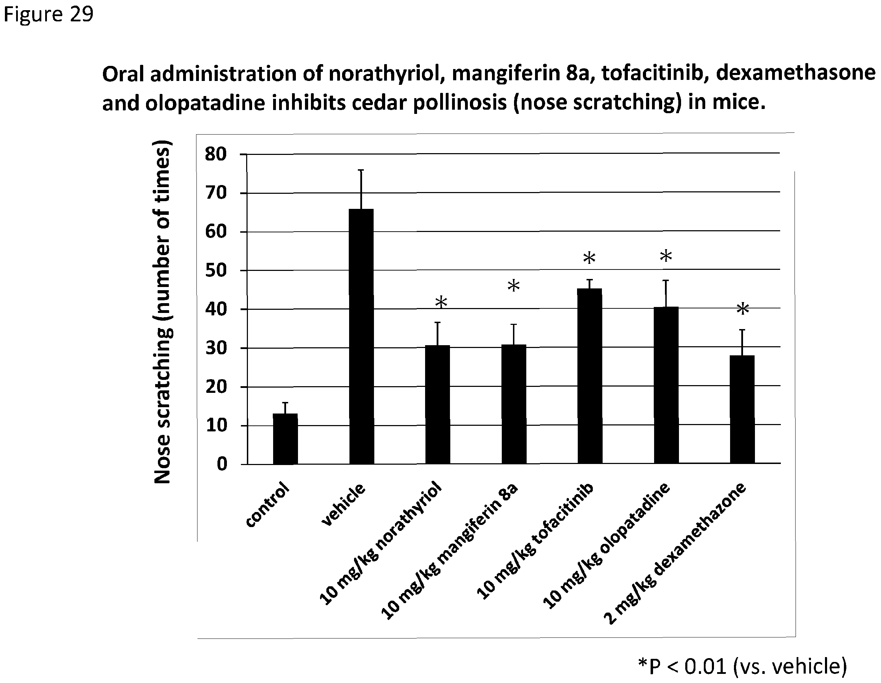

The results are shown in Figure 29. The horizontal axis represents the sample group and the vertical axis represents the number of nose scratches. Compared to the cedar pollen-immunized control (labeled "control"), the cedar pollen-immunized subject group (labeled "vehicle") showed a marked increase in nose-scratching behavior, indicating the development of cedar pollinosis. The norathyriol group ("10 mg/kg norathyriol"), mangiferin 8a group ("10 mg/kg mangiferin 8a"), tofacitinib group ("10 mg/kg tofacitinib"), dexamethasone group ("2 mg/kg dexamethasone"), and olopatadine group ("10 mg/kg olopatadine") significantly decreased the number of nose scratches compared to the vehicle group. The norathyriol and mangiferin 8a groups significantly suppressed nose-scratching behavior compared to the tofacitinib and olopatadine groups.

As described above, it was found that the norathyriol and mangiferin 8a treatment groups significantly suppressed nose-scratching behavior caused by cedar pollinosis. In other words, compositions containing norathyriol or mangiferin 8a as active ingredients can suppress nose-scratching behavior caused by cedar pollinosis.

### < Example 29: Inhibition of rhinitis ("sneezing") in cedar pollinosis model mice >

A 0.2 mL dose of 100 µg cedar pollen and 1 mg Alum mixture was administered to 6-week-old female Balb/c mice (Japan SLC Inc.) (day 0 of the example). Alum was used as an adjuvant. Seven days later and 14 days later, the Balb/c mice were immunized again with 0.2 mL injections of the above mixture. Starting on day 20, mice were orally treated with 10 mg/kg of norathyriol, 10 mg/kg of mangiferin 8a, 10 mg/kg of tofacitinib (JAK inhibitor), 2 mg/kg of dexamethasone (steroid) and 10 mg/kg of olopatadine (antihistamine) daily for 8 consecutive days. Starting on day 21, 500 µg cedar pollen was administered intranasally, and sneezing was measured on day 27. Note that tofacitinib, dexamethasone, and olopatadine are currently in clinical use in cedar pollinosis and were used as control drugs in this example.

The group that was just immunized with cedar pollen is called the subject group, the one that received norathyriol is called the norathyriol group, the one that received mangiferin 8a is called the mangiferin 8a group, the one that received tofacitinib is called the tofacitinib group, the one that received dexamethasone is called the dexamethasone group, and the one that received olopatadine is called the olopatadine group. Each group consisted of five animals.

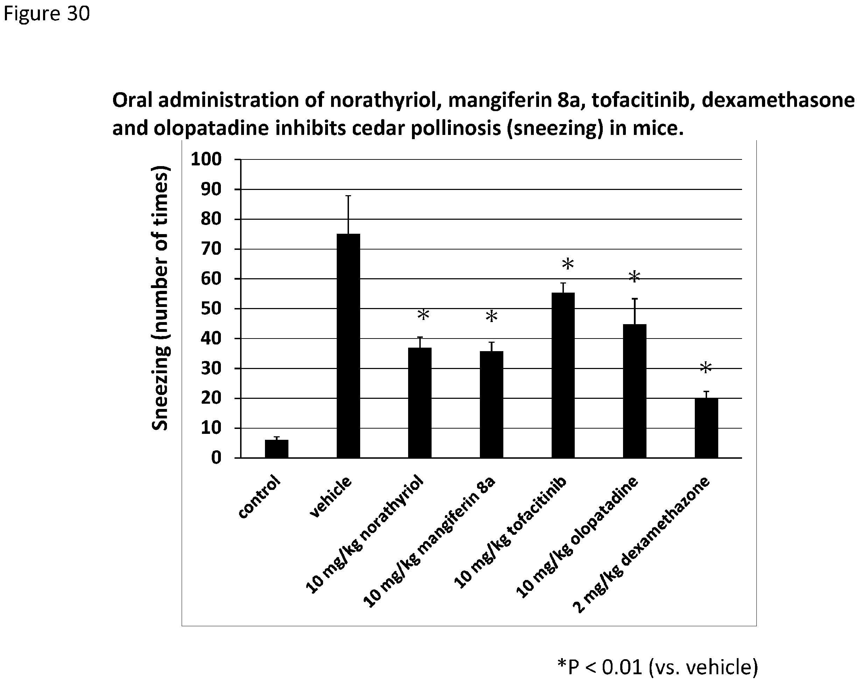

The results are shown in Figure 30. The horizontal axis represents the sample group and the vertical axis represents the number of sneezing. Compared to the cedar pollen-immunized control (labeled "control"), the cedar pollen-immunized subject group (labeled "vehicle") showed a marked increase in sneezing, indicating the development of cedar pollinosis. The norathyriol group ("10 mg/kg norathyriol"), mangiferin 8a group ("10 mg/kg mangiferin 8a"), tofacitinib group ("10 mg/kg tofacitinib"), dexamethasone group ("2 mg/kg dexamethasone"), and olopatadine group ("10 mg/kg olopatadine") significantly decreased the number of sneezing compared to the vehicle group. The norathyriol and mangiferin 8a groups significantly suppressed sneezing compared to the tofacitinib and olopatadine groups.

As described above, it was found that the norathyriol and mangiferin 8a treatment groups significantly suppressed sneezing caused by cedar pollinosis. In other words, compositions containing norathyriol or mangiferin 8a as active ingredients can suppress sneezing caused by cedar pollinosis.

### < Example 30: Inhibition of IgE antibody production >

Serum was collected from day 28 mice in Examples 28 and 29, and IgE antibody production was measured by enzyme-linked immunosorbent assay (ELISA). This assay was performed using a mouse IgE antibody ELISA kit (R&D).

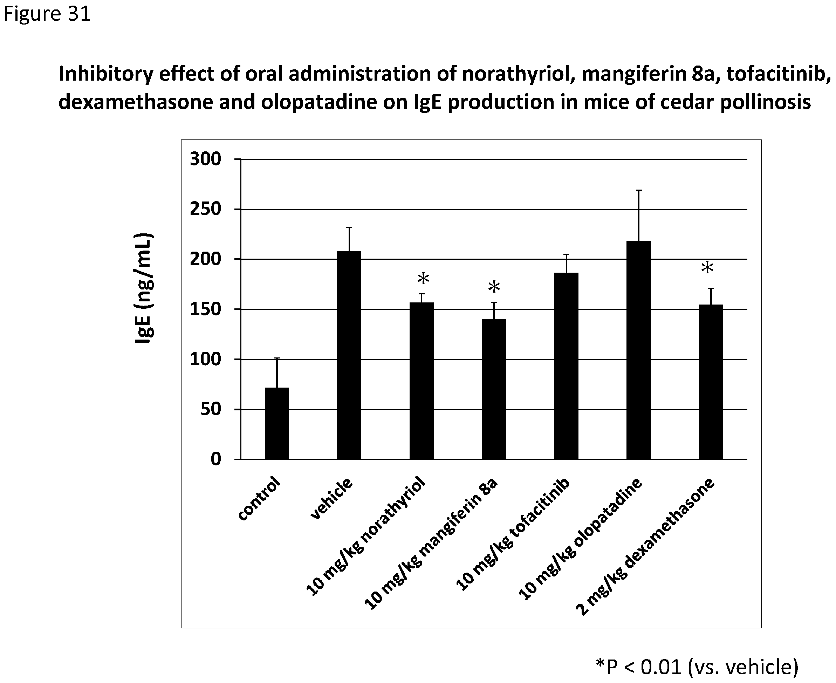

Results are shown in Figure 31. The horizontal axis represents the sample group and the vertical axis represents the amount of IgE antibody. The amount of IgE antibody in the cedar pollen-immunized subject group (labeled "vehicle") was 207 ng/mL, compared to 71 ng/mL in the cedar pollen-immunized control (labeled "control"), indicating that the subjects developed cedar pollinosis. The IgE antibody production was 156 ng/mL in the norathyriol group ("10 mg/kg norathyriol"), 139 ng/mL in the mangiferin 8a group ("10 mg/kg mangiferin 8a"), 185 ng/mL in the tofacitinib group ("10 mg/kg tofacitinib"), 154 ng/mL in the dexamethasone group ("2 mg/kg dexamethasone"), and 218 ng/mL in the olopatadine group ("10 mg/kg olopatadine"), indicating that norathyriol group, mangiferin 8a group and dexamethasone groups significantly decreased IgE antibody production compared to the vehicle group. The norathyriol and mangiferin 8a groups significantly suppressed IgE antibody production compared to the tofacitinib and olopatadine groups.

As described above, it was found that the norathyriol and mangiferin 8a treatment groups markedly suppressed IgE antibody production caused by cedar pollinosis. In other words, compositions containing norathyriol or mangiferin 8a as active ingredients can suppress IgE antibody production caused by cedar pollinosis.

### < Example 31: Inhibition of IL-4 production >

Serum was collected from day 28 mice in Examples 28 and 29, and IL-4 production was measured by enzyme-linked immunosorbent assay (ELISA). This assay was performed using a mouse IL-4 ELISA kit (R&D).

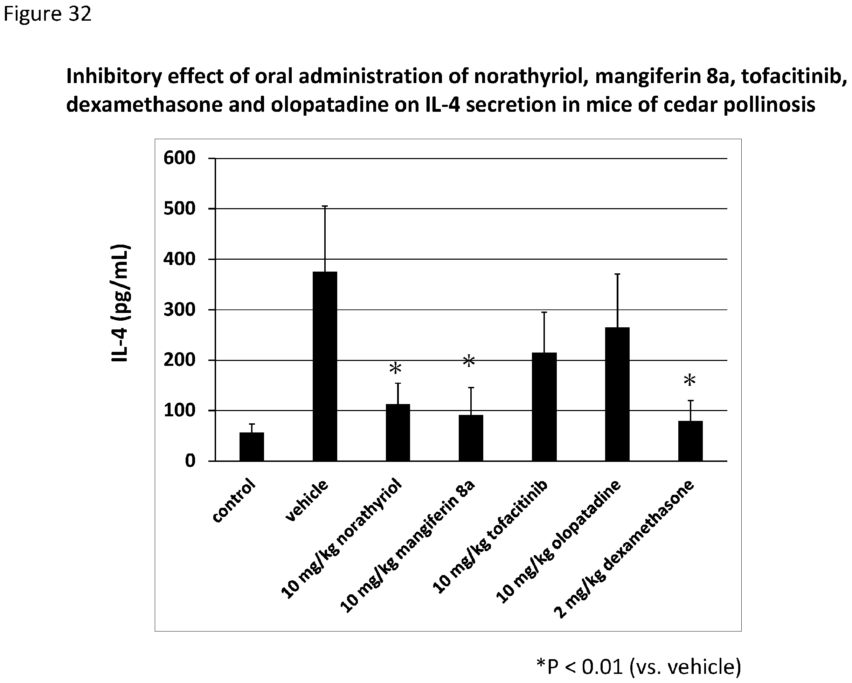

Results are shown in Figure 32. The horizontal axis represents the sample group and the vertical axis represents the amount of IL-4. The amount of IL-4 in the cedar pollen-immunized subject group (labeled "vehicle") was 374 pg/mL, compared to 55 pg/mL in the cedar pollen-immunized control (labeled "control"), indicating that the subjects developed cedar pollinosis. The IL-4 production was 111 pg/mL in the norathyriol group ("10 mg/kg norathyriol"), 90 pg/mL in the mangiferin 8a group ("10 mg/kg mangiferin 8a"), 213 pg/mL in the tofacitinib group ("10 mg/kg tofacitinib"), 78 pg/mL in the dexamethasone group ("2 mg/kg dexamethasone"), and 263 pg/mL in the olopatadine group ("10 mg/kg olopatadine"), indicating that norathyriol group, mangiferin 8a group and dexamethasone groups significantly decreased IL-4 production compared to the vehicle group. The norathyriol and mangiferin 8a groups significantly suppressed IL-4 production compared to the tofacitinib and olopatadine groups.

As described above, it was found that the norathyriol and mangiferin 8a treatment groups markedly suppressed IL-4 production caused by cedar pollinosis. In other words, compositions containing norathyriol or mangiferin 8a as active ingredients can suppress IL-4 production caused by cedar pollinosis.

### < Example 32: Inhibition of IL-5 production >

Serum was collected from day 28 mice in Examples 28 and 29, and IL-5 production was measured by enzyme-linked immunosorbent assay (ELISA). This assay was performed using a mouse IL-5 ELISA kit (R&D).

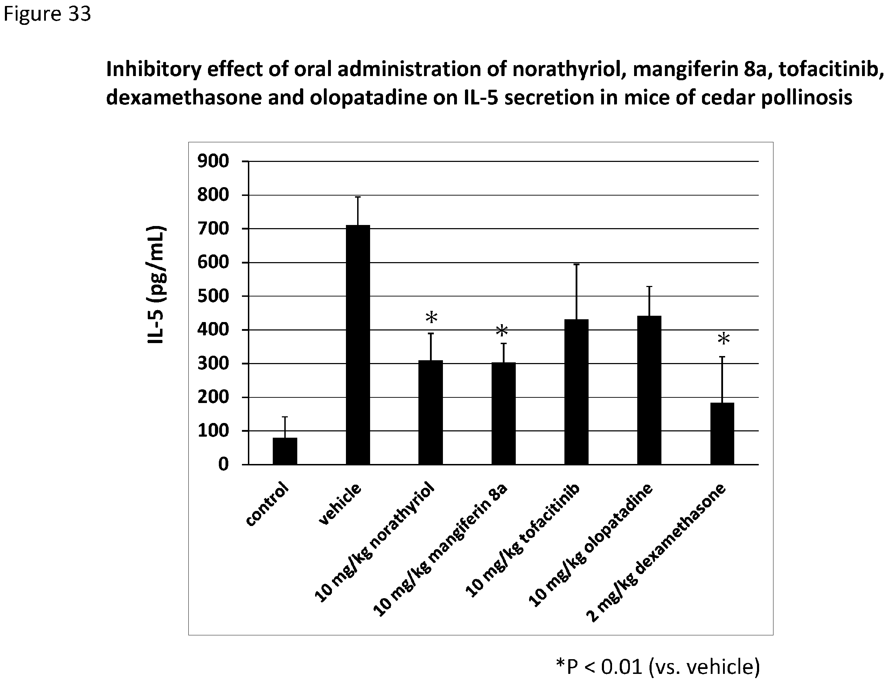

Results are shown in Figure 33. The horizontal axis represents the sample group and the vertical axis represents the amount of IL-5. The amount of IL-5 in the cedar pollen-immunized subject group (labeled "vehicle") was 708 pg/mL, compared to 77 pg/mL in the cedar pollen-immunized control (labeled "control"), indicating that the subjects developed cedar pollinosis. The IL-5 production was 307 pg/mL in the norathyriol group ("10 mg/kg norathyriol"), 300 pg/mL in the mangiferin 8a group ("10 mg/kg mangiferin 8a"), 428 pg/mL in the tofacitinib group ("10 mg/kg tofacitinib"), 182 pg/mL in the dexamethasone group ("2 mg/kg dexamethasone"), and 438 pg/mL in the olopatadine group ("10 mg/kg olopatadine"), indicating that norathyriol group, mangiferin 8a group and dexamethasone groups significantly decreased IL-5 production compared to the vehicle group. The norathyriol and mangiferin 8a groups significantly suppressed IL-5 production compared to the tofacitinib and olopatadine groups.

As described above, it was found that the norathyriol and mangiferin 8a treatment groups markedly suppressed IL-5 production caused by cedar pollinosis. In other words, compositions containing norathyriol or mangiferin 8a as active ingredients can suppress IL-5 production caused by cedar pollinosis.

### < Example 33: Inhibition of IL-17 production >

Serum was collected from day 28 mice in Examples 28 and 29, and IL-17 production was measured by enzyme-linked immunosorbent assay (ELISA). This assay was performed using a mouse IL-17 ELISA kit (R&D).

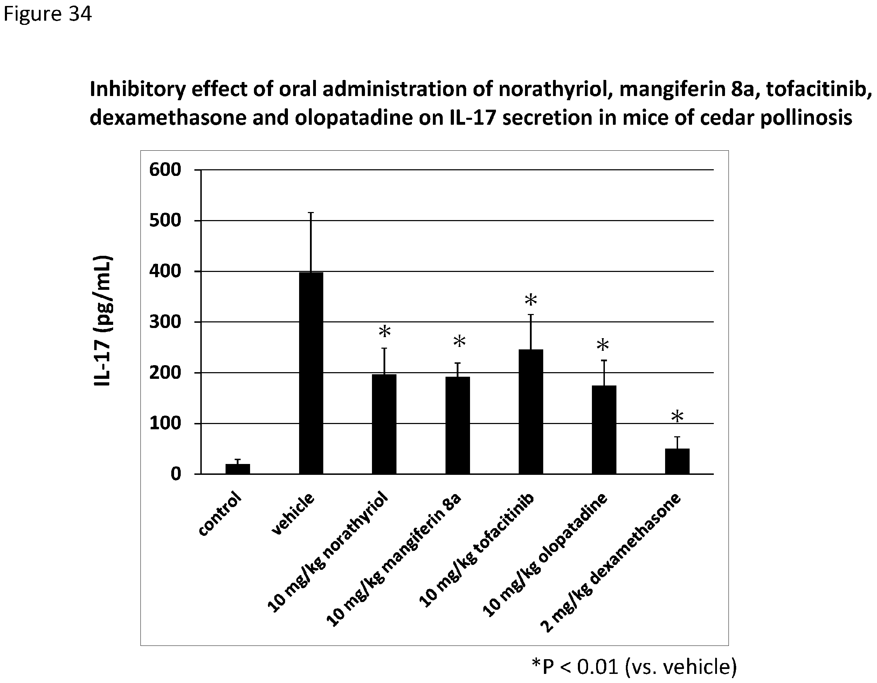

Results are shown in Figure 34. The horizontal axis represents the sample group and the vertical axis represents the amount of IL-17. The amount of IL-17 in the cedar pollen-immunized subject group (labeled "vehicle") was 397 pg/mL, compared to 18 pg/mL in the cedar pollen-immunized control (labeled "control"), indicating that the subjects developed cedar pollinosis. The IL-5 production was 195 pg/mL in the norathyriol group ("10 mg/kg norathyriol"), 190 pg/mL in the mangiferin 8a group ("10 mg/kg mangiferin 8a"), 244 pg/mL in the tofacitinib group ("10 mg/kg tofacitinib"), 49 pg/mL in the dexamethasone group ("2 mg/kg dexamethasone"), and 172 pg/mL in the olopatadine group ("10 mg/kg olopatadine"), indicating that norathyriol group, mangiferin 8a group and dexamethasone groups significantly decreased IL-17 production compared to the vehicle group. The norathyriol and mangiferin 8a groups significantly suppressed IL-17 production compared to the tofacitinib and olopatadine groups.

As described above, it was found that the norathyriol and mangiferin 8a treatment groups markedly suppressed IL-17 production caused by cedar pollinosis. In other words, compositions containing norathyriol or mangiferin 8a as active ingredients can suppress IL-17 production caused by cedar pollinosis.

In other words, the results of Examples 28-33 show that the subject group developed cedar pollinosis, while the norathyriol- and mangiferin 8a-treated groups suppressed cedar pollinosis. In other words, compositions with norathyriol or mangiferin 8a as active ingredients can inhibit cedar pollinosis.

### < Example 34: Inhibition of differentiation into mature B cells >

B cells collected from mice were cultured in RPMI 1640 medium for 1 day. Then, 100 µM mangiferin, 10 µM mangiferin 8a, 10 µM norathyriol, 20 µM 1,3,5,6-tetrahydroxyxanthone, 10 µM xanthohydrol, 10 µM α-mangostin, 10 µM of γ-mangostin were added to B cell, and after 1 day of culture, 100 ng/mL of BAFF was added, followed by 10 days of culture. After 10 days of culture, cells were stained with anti-IgM and anti-IgD antibodies, markers of B cells, and the expression of IgM and IgD was measured using BD LSRFortessa. Note that IgM negative and IgD negative indicates pre-B cells, IgM positive and IgD negative indicates immature B cells, and IgM positive and IgD positive indicates mature B cells.

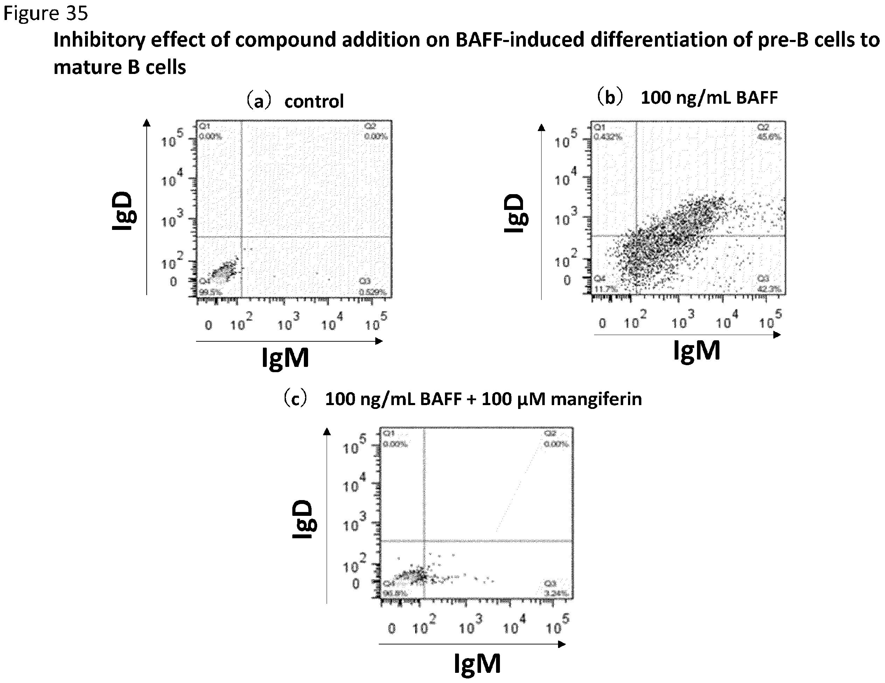

Results are shown in Figure 35, 36 and 37. In all figures, the horizontal axis represents the expression level of IgM and the vertical axis represents the expression level of IgD. In the control without compound and BAFF (Figure 35(a)), 99.5% of the population was IgM negative and IgD negative, 0.529% was IgM positive and IgD negative, and 0% was IgM positive and IgD positive. On the other hand, in the BAFF-added group (Fig. 35(b)), 11.7% of the population was IgM negative and IgD negative, 42.3% was IgM positive and IgD negative, and 45.6% was IgM positive and IgD positive, indicating that they were differentiated into mature B cells.

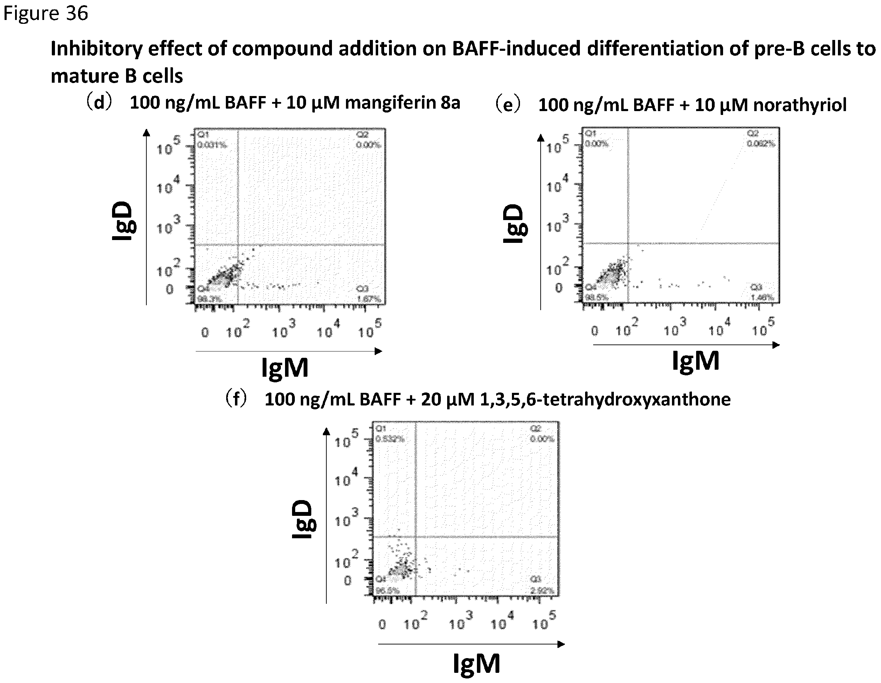

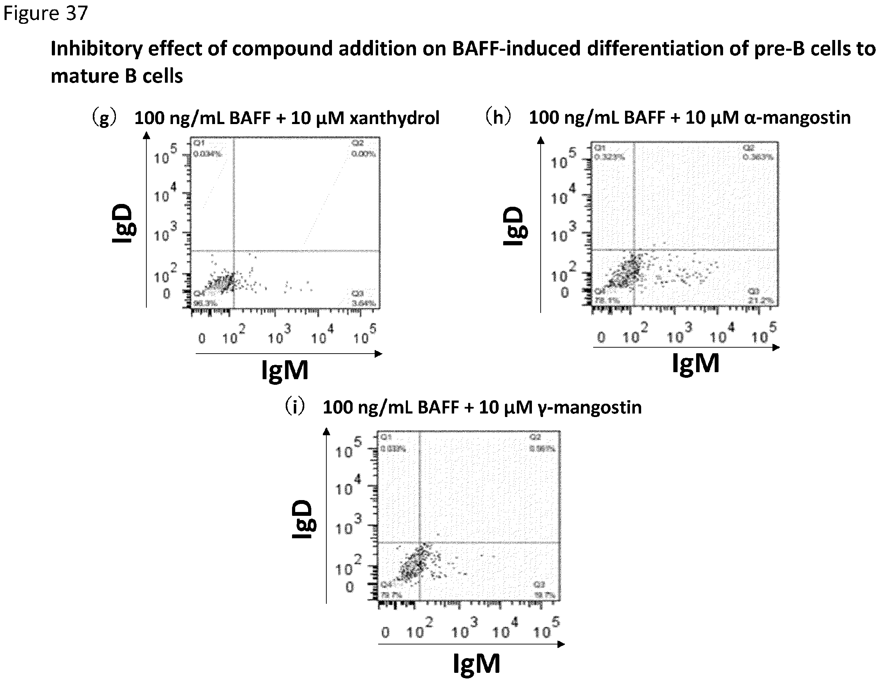

In the mangiferin group (Figure 35(c)), 95.8% of the population was IgM negative and IgD negative, 3.24% was IgM positive and IgD negative, 0% was IgM positive and IgD positive, in the mangiferin 8a group (Figure 36(d)), 98.3% of the population was IgM negative and IgD negative, 1.67% was IgM positive and IgD negative, 0% was IgM positive and IgD positive population, in the norathyriol group (Figure 36(e)), 98.5% of the population was IgM negative and IgD negative, 1.46% was IgM positive and IgD negative, 0.062% was IgM positive and IgD positive, in the 1,3,5,6-tetrahydroxyxanthone group (Figure 36(f)), 96.5% of the population was IgM negative and IgD negative, 2.92% was IgM positive and IgD negative, 0% was IgM positive and IgD positive, in the xanthohydrol group (Figure 37(g)), 96.3% of the population was IgM negative and IgD negative, 3.64% was IgM positive and IgD negative, 0% was IgM positive and IgD positive, in the α-mangostin group (Figure 37(h)), 78.1% of the population was IgM negative and IgD negative, 21.2% was IgM positive and IgD negative, 0.363% was IgM positive and IgD positive, and in the γ-mangostin group (Fig. 37(i)), 79.7% of the population was IgM negative and IgD negative, 19.7% was IgM positive and IgD negative, 0.561% was IgM positive and IgD positive, indicating that the percentage of mature B cells was significantly reduced in all compounds.

In other words, mangiferin, mangiferin 8a, norathyriol, 1,3,5,6-tetrahydroxyxanthone, xanthohydrol, α-mangostin, and γ-mangostin inhibited the differentiation of pre-B cells to mature B cells in BAFF-induced allergic inflammation.

### < Example 35: Inhibition of differentiation into plasma cells >

B cells collected from mice were cultured in RPMI 1640 medium for 1 day. Then, 100 µM mangiferin, 10 µM mangiferin 8a, 10 µM norathyriol, 20 µM 1,3,5,6-tetrahydroxyxanthone, 10 µM xanthohydrol, 10 µM α-mangostin, 10 µM of γ-mangostin were added to B cell, and after 1 day of culture, 100 ng/mL of BAFF was added, followed by 10 days of culture. After 10 days of culture, cells were stained with anti-CD138 antibody, a marker of plasma cells, and CD138 expression was measured using BD LSRFortessa.

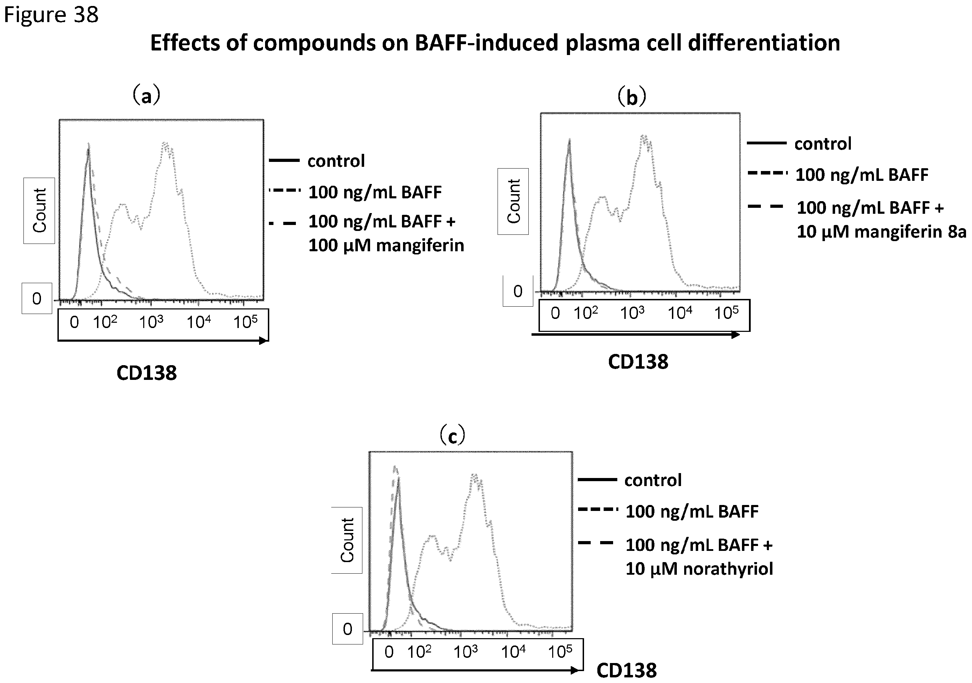

Results are shown in Figures 38, 39 and 40. In all figures, the horizontal axis represents the expression level of CD138 and the vertical axis represents the number of cells. The solid line in the panel shows control, which contains neither BAFF nor compound, the dotted line shows 100 ng/mL BAFF added (100 ng/mL BAFF was just added to B cells), and the dashed line shows 100 ng/mL BAFF and each compound added. CD138 expression was markedly increased in the BAFF-added group compared to the control without the compound and BAFF.

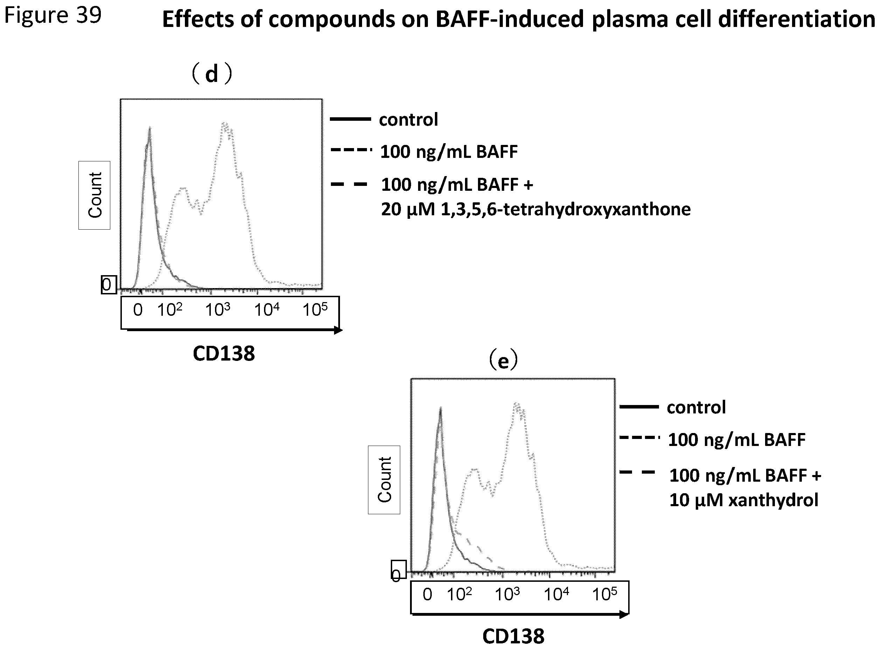

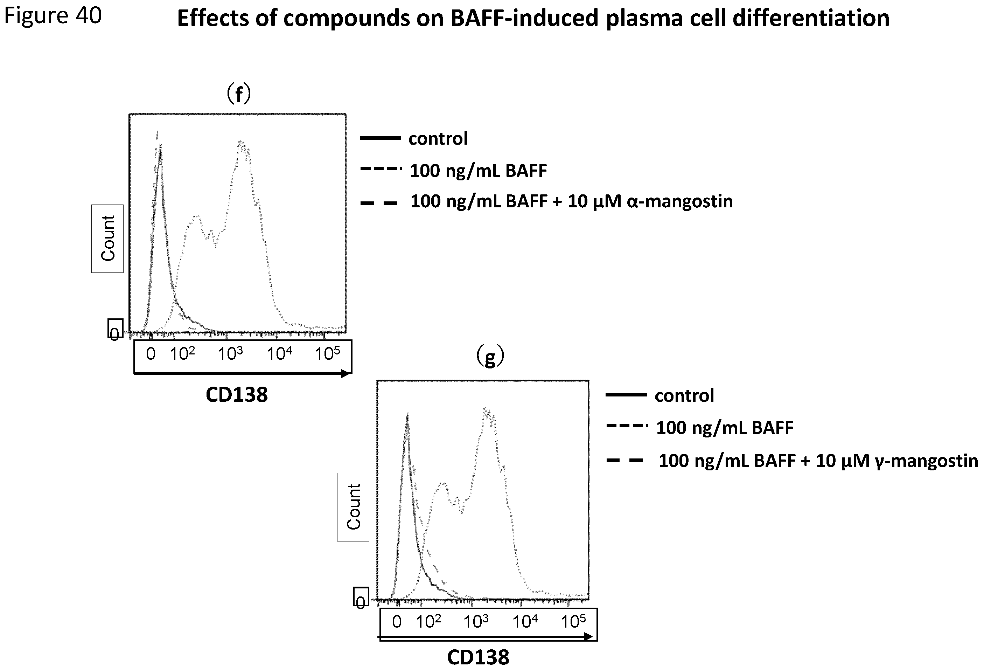

The mangiferin group (Fig. 38(a)), mangiferin 8a group (Fig. 38(b)), norathyriol group (Fig. 38(c)), 1,3,5,6-tetrahydroxyxanthone group (Fig. 39(d)), xanthohydrol group (Fig. 39(e)), α-mangostin group (Fig. 40(f)) and γ-mangostin group (Fig. 40(g)) showed significantly decreased CD138 expression compared to the BAFF-added group, which were almost at the same level as the control group. In other words, mangiferin, mangiferin 8a, norathyriol, 1,3,5,6-tetrahydroxyxanthone, xanthohydrol, α-mangostin, and γ-mangostin inhibited differentiation into plasma cells in BAFF-induced allergic inflammation.

### < Example 36: Inhibition of BAFF-induced NIK activation by compounds in B cells >

Spleens were harvested from 6-week-old male Balb/c mice and B cells were isolated. After isolation, the cells were seeded in 100 mm² dish and cultured in RPMI 1640 medium for 1 day and used as control. In addition, B cells were seeded into 100 mm² dish, and after 1 day of culture, 100 ng/mL BAFF was added and cultured for 1 h. Furthermore, B cells were seeded in 100 mm² dish, and after 1 day of culture, 100 µM mangiferin, 10 µM mangiferin 8a, 10 µM norathyriol, 20 µM 1,3,5,6-tetrahydroxyxanthone, 10 µM xanthohydrol, 10 µM of α-mangostin, and 10 µM of γ-mangostin were added to B cells, and after 23 h, 100 ng/mL BAFF was added to each, and the cells were incubated for 1 h. All were incubated at 37°C and 5% CO₂.

Proteins were extracted with cell lysate and used as samples. Each sample was transferred to a PVDF membrane after SDSPAGE and assayed with anti-phospho-NIK, anti-NIK, anti-NF-κB p52, anti-NF-κB p65 and anti-Lamin antibodies.

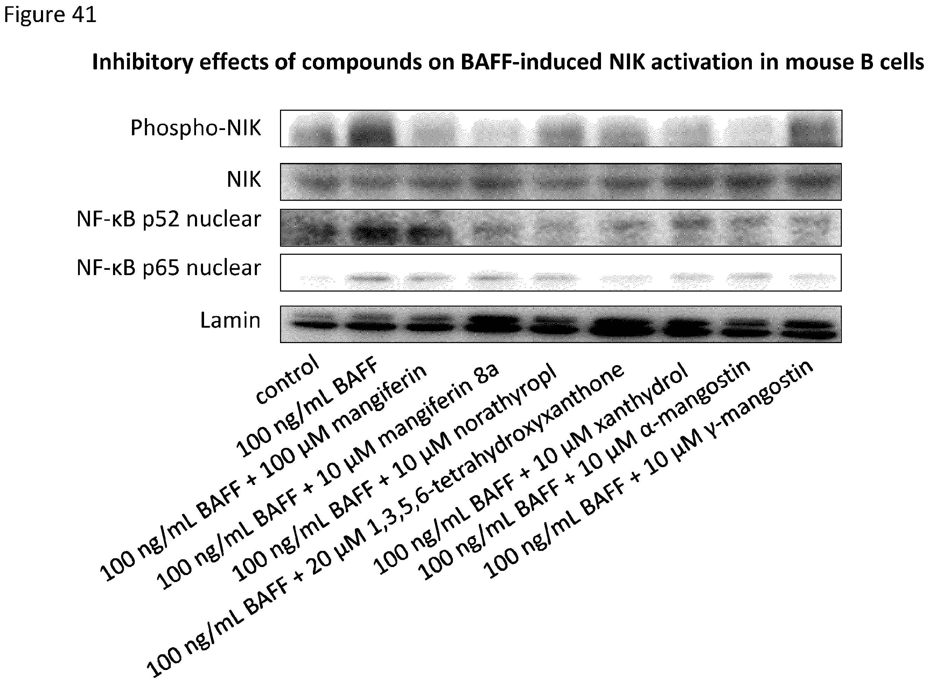

The results of immunoblotting are shown in Figure 41. Mangiferin, mangiferin 8a, norathyriol, 1,3,5,6-tetrahydroxyxanthone, xanthohydrol, α-mangostin, and γ-mangostin are shown as reagents (compounds) in the horizontal direction.

The antibody species is indicated in the vertical direction. Specifically, in the case of anti-phospho-NIK antibody (described as "Phospho-NIK"), anti-NIK antibody (described as "NIK"), anti-NF-κB p52 antibody (described as "NF-κB p52 nuclear"), anti-NF-κB p65 antibody (described as "NF-κB p65 nuclear") and anti-Lamin antibody (described as "Lamin").

Photographs of anti-NIK antibody showed no decrease in the density of the NIK band compared to the control. In the case of anti-phospho-NIK antibody, 100 ng/mL BAFF was increased compared to control. This indicates that the addition of BAFF activates NIK and enhances B cell differentiation and activation.

On the other hand, in the case of anti-phospho-NIK antibody, the immunoblotting results for mangiferin, mangiferin 8a, norathyriol, 1,3,5,6-tetrahydroxyxanthone, xanthohydrol, α-mangostin, and γ-mangostin samples were fainter. Therefore, it can be said that mangiferin, mangiferin 8a, norathyriol, 1,3,5,6-tetrahydroxyxanthone, xanthohydrol, α-mangostin, and γ-mangostin inhibit NIK phosphorylation.

Next, we refer to anti-NF-κB p52 (described as "NF-κB p52 nuclear"), anti-NF-κB p65 (described as "NF-κB p65 nuclear"), and anti-Lamin (described as "Lamin") antibodies against cellular nuclear material. Lamin was present regardless of all compounds, but nuclear NF-κB p52 was found to be fainter with mangiferin, mangiferin 8a, norathyriol, 1,3,5,6-tetrahydroxyxanthone, xanthohydrol, α-mangostin, and γ-mangostin treatments compared to 100 ng/mL BAFF. Nuclear NF-κB p65 was also reduced (shaded) for all compounds compared to 100 ng/mL BAFF.

Lamin, a fibrous protein that maintains structure and regulates transcription in the cell nucleus, is detected as an internal standard (loading control) for nuclear proteins. Therefore, with the nuclear protein loading control detected in all samples, the addition of each compound indicates that nuclear NF-κB p65 and NF-κB p52 are not present, or if present, very little. In other words, mangiferin, mangiferin 8a, norathyriol, 1,3,5,6-tetrahydroxyxanthone, xanthohydrol, α-mangostin, and γ-mangostin are a NIK inhibitor.

### < Example 37: Inhibition of BAFF-induced TNFα secretions >

Spleens were harvested from 6-week-old male DBA/1J mice and B cells were isolated. After isolation, the cells were cultured in RPMI 1640 medium for 1 day. Then, 10 µM mangiferin 8a and 10 µM norathyriol were added to B cells, and 100 ng/mL of BAFF was added to each 3 h later, and the cells were cultured for 7 days. After 7 days of culture, the culture supernatant was collected and TNFα secretion was measured by enzyme-linked immunosorbent assay (ELISA). This assay was performed using a mouse TNFα ELISA kit (R&D).

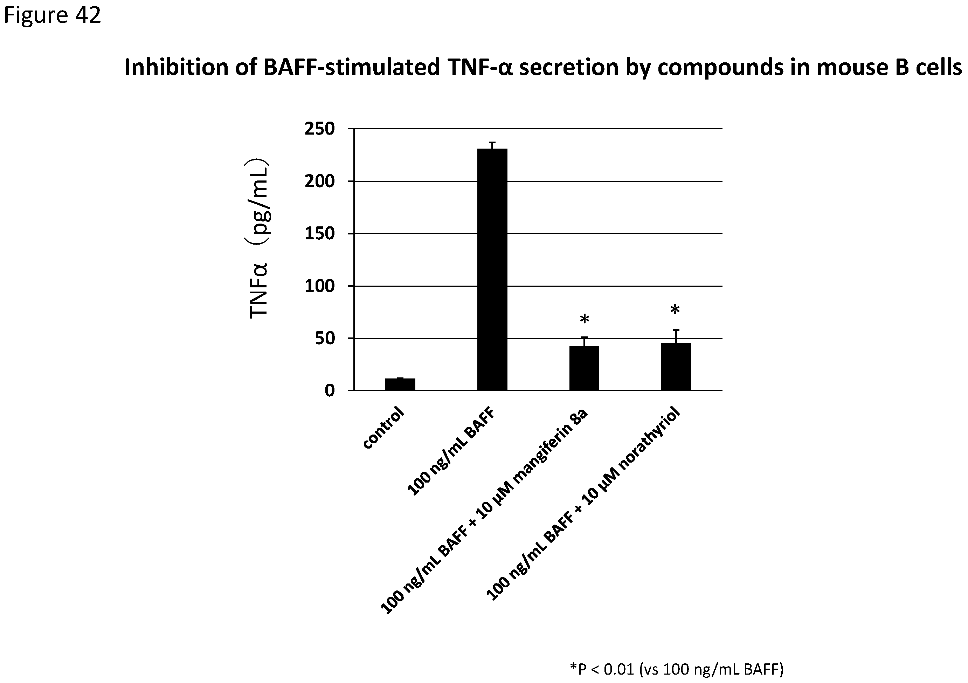

The results are shown in Figure 42. The horizontal axis represents the sample group and the vertical axis represents TNFα secretion (pg/mL). In control (no compound or BAFF added), TNFα was 11.34 pg/mL. On the other hand, at adding 100 ng/mL BAFF, TNFα secretion was 230.64 pg/mL, indicating that B cells are activated by BAFF stimulation and TNFα secretion is enhanced.

In contrast, the mangiferin 8a and norathyriol groups were 41.88 pg/mL and 45.07 pg/mL, respectively.

As described above, administration of mangiferin 8a and norathyriol markedly inhibited TNFα secretion in BAFF stimulation. As a result, we can say that excessive TNFα is suppressed and allergic diseases are improved.

### < Example 38: Inhibition of BAFF-induced IL-6 secretions >

Spleens were harvested from 6-week-old male DBA/1J mice and B cells were isolated. After isolation, the cells were cultured in RPMI 1640 medium for 1 day. Then, 10 µM mangiferin 8a and 10 µM norathyriol were added to B cells, and 100 ng/mL of BAFF was added to each 3 h later, and the cells were cultured for 7 days. After 7 days of culture, the culture supernatant was collected and IL-6 secretion was measured by enzyme-linked immunosorbent assay (ELISA). This assay was performed using a mouse IL-6 ELISA kit (R&D).

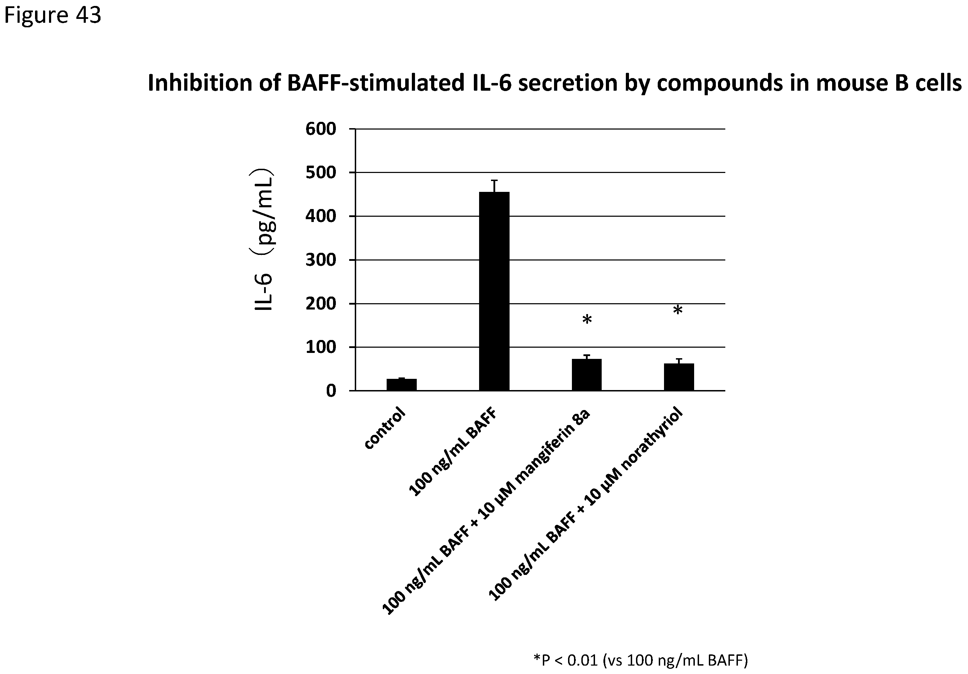

The results are shown in Figure 43. The horizontal axis represents the sample group and the vertical axis represents IL-6 secretion (pg/mL). In control (no compound or BAFF added), IL-6 was 26.06 pg/mL. On the other hand, at adding 100 ng/mL BAFF, IL-6 secretion was 454.47 pg/mL, indicating that B cells are activated by BAFF stimulation and IL-6 secretion is enhanced.

In contrast, the mangiferin 8a and norathyriol groups were 71.52 pg/mL and 61.39 pg/mL, respectively.

As described above, administration of mangiferin 8a and norathyriol markedly inhibited IL-6 secretion in BAFF stimulation. As a result, we can say that excessive IL-6 is suppressed and allergic diseases are improved.

### < Example 39: Inhibition of Th1 cell activation in an inflammation-induced mouse model >

Equal volume mixtures of Freund's complete adjuvant and Bovine type2 collagen were administered to 6-week-old male DBA/1J mice (Japan SLC Inc.) at 0.1 mL (day 0 of the study). 21 days later, DBA/1J mice were again injected with 0.1 mL of the above mixture for secondary immunization. After the second immunization, mice were orally treated with 10 mg/kg of mangiferin 8a and 10 mg/kg of norathyriol daily for 20 days. 20 days later, thymus glands were harvested from mice and stained with anti-CD4 and anti-IFNy antibodies, which are Th1 cell markers, and expression of IFNγ (CD4 positive) was measured using BD LSRFortessa.

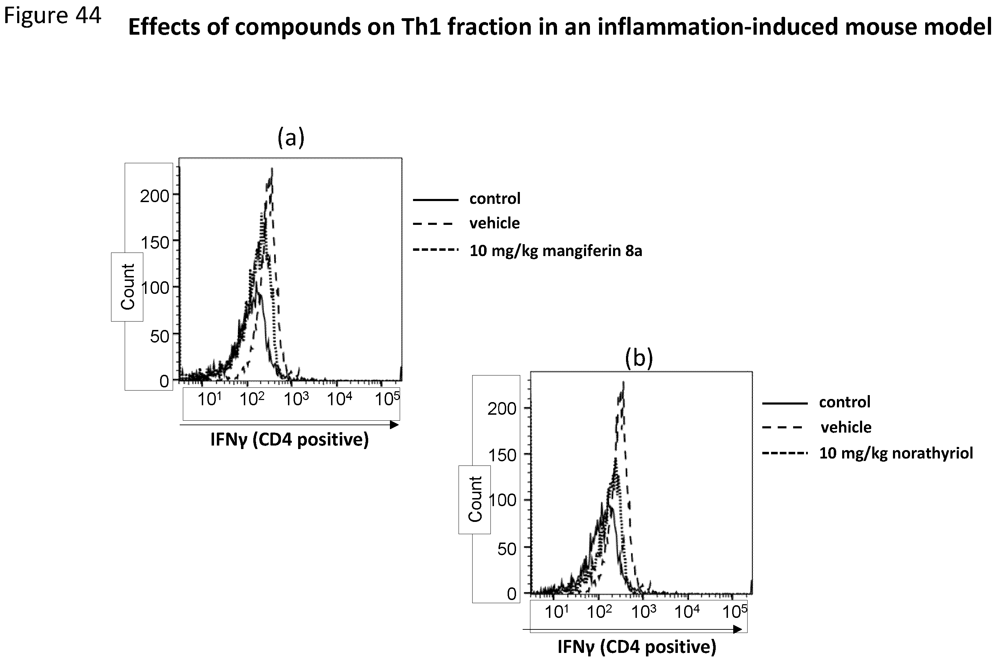

The results are shown in Figure 44. In all figures, the horizontal axis represents the expression level of IFNγ (CD4 positive) and the vertical axis represents the number of cells. The solid lines in the panels show control without induced inflammation, the dashed lines show those with induced inflammation (vehicle), and the dotted lines show those with induced inflammation treated with each compound. Compared to the control, in which no inflammation was induced, IFNγ expression was markedly increased in the vehicle group, indicating that Th1 cells were activated. Compared to the vehicle group, IFNγ expression was markedly reduced in the mangiferin 8a group (Fig. 44(a)) and norathyriol group (Fig. 44(b)), and mangiferin 8a group and norathyriol group were almost equal to that of the control. In other words, mangiferin 8a and norathyriol inhibited Th1 cell activation in a mouse model of inflammation.

### < Example 40: Inhibition of Th2 cell activation in an inflammation-induced mouse model >

Equal volume mixtures of Freund's complete adjuvant and Bovine type2 collagen were administered to 6-week-old male DBA/1J mice (Japan SLC Inc.) at 0.1 mL (day 0 of the study). 21 days later, DBA/1J mice were again injected with 0.1 mL of the above mixture for secondary immunization. After the second immunization, mice were orally treated with 10 mg/kg of mangiferin 8a and 10 mg/kg of norathyriol daily for 20 days. 20 days later, thymus glands were harvested from mice and stained with anti-CD4 and anti-IL-4 antibodies, which are Th2 cell markers, and expression of IL-4 (CD4 positive) was measured using BD LSRFortessa.

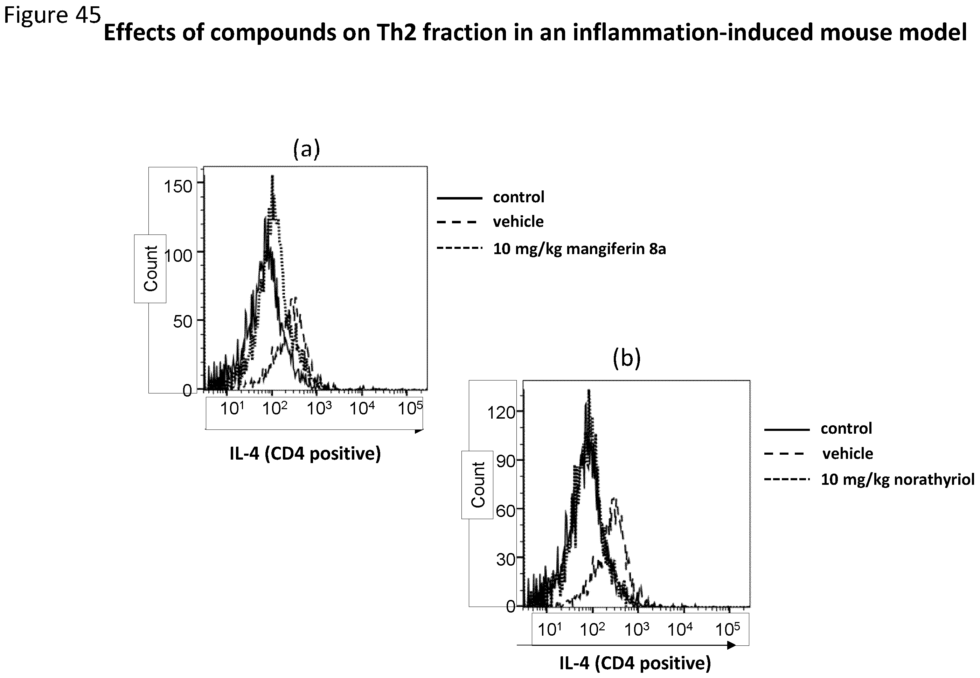

The results are shown in Figure 45. In all figures, the horizontal axis represents the expression level of IL-4 (CD4 positive) and the vertical axis represents the number of cells. The solid lines in the panels show control without induced inflammation, the dashed lines show those with induced inflammation (vehicle), and the dotted lines show those with induced inflammation treated with each compound. Compared to the control, in which no inflammation was induced, IL-4 expression was markedly increased in the vehicle group, indicating that Th2 cells were activated. Compared to the vehicle group, IL-4 expression was markedly reduced in the mangiferin 8a group (Fig. 45(a)) and norathyriol group (Fig. 45(b)), and mangiferin 8a group and norathyriol group were almost equal to that of the control. In other words, mangiferin 8a and norathyriol inhibited Th2 cell activation in a mouse model of inflammation.

### < Example 41: Inhibition of Th17 cell activation in an inflammation-induced mouse model >

Equal volume mixtures of Freund's complete adjuvant and Bovine type2 collagen were administered to 6-week-old male DBA/1J mice (Japan SLC Inc.) at 0.1 mL (day 0 of the study). 21 days later, DBA/1J mice were again injected with 0.1 mL of the above mixture for secondary immunization. After the second immunization, mice were orally treated with 10 mg/kg of mangiferin 8a and 10 mg/kg of norathyriol daily for 20 days. 20 days later, thymus glands were harvested from mice and stained with anti-CD4 and anti-IL-17A antibodies, which are Th17 cell markers, and expression of IL-17A (CD4 positive) was measured using BD LSRFortessa.

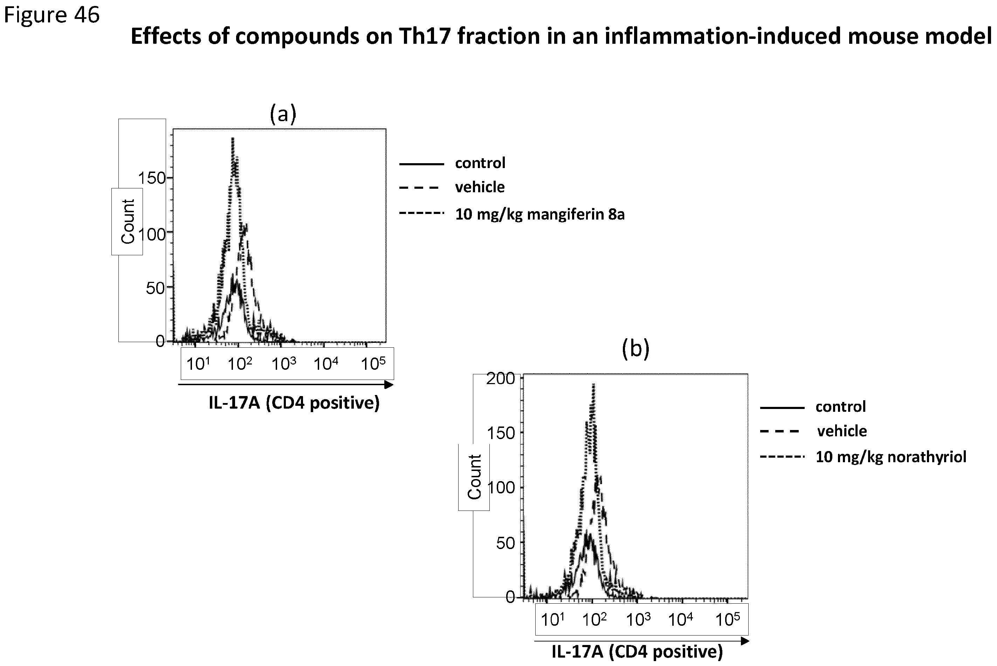

The results are shown in Figure 46. In all figures, the horizontal axis represents the expression level of IL-17A (CD4 positive) and the vertical axis represents the number of cells. The solid lines in the panels show control without induced inflammation, the dashed lines show those with induced inflammation (vehicle), and the dotted lines show those with induced inflammation treated with each compound. Compared to the control, in which no inflammation was induced, IL-17A expression was markedly increased in the vehicle group, indicating that Th17 cells were activated. Compared to the vehicle group, IL-17A expression was markedly reduced in the mangiferin 8a group (Fig. 46(a)) and norathyriol group (Fig. 46(b)), and mangiferin 8a group and norathyriol group were almost equal to that of the control. In other words, mangiferin 8a and norathyriol inhibited Th17 cell activation in a mouse model of inflammation.

### < Example 42: Promotes Treg cell activation in an inflammation-induced mouse model >

Equal volume mixtures of Freund's complete adjuvant and Bovine type2 collagen were administered to 6-week-old male DBA/1J mice (Japan SLC Inc.) at 0.1 mL (day 0 of the study). 21 days later, DBA/1J mice were again injected with 0.1 mL of the above mixture for secondary immunization. After the second immunization, mice were orally treated with 10 mg/kg of mangiferin 8a and 10 mg/kg of norathyriol daily for 20 days. 20 days later, thymus glands were harvested from mice and stained with anti-CD4, anti-CD25 and anti-Foxp3 antibodies, which are Treg cell markers, and expression of Foxp3 (CD4 positive and CD25 positive) was measured using BD LSRFortessa.

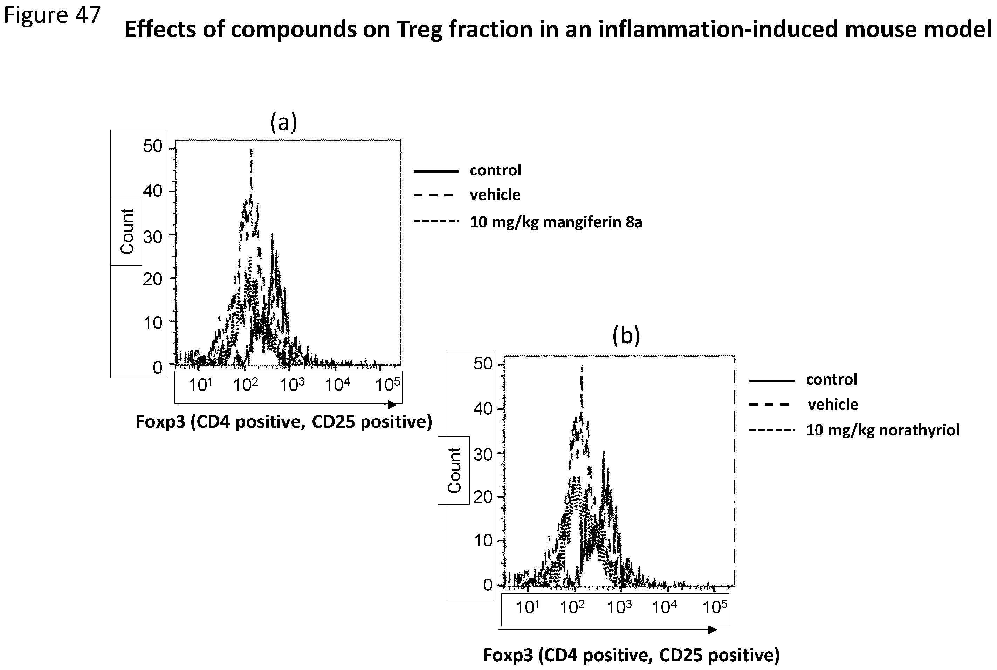

The results are shown in Figure 47. In all figures, the horizontal axis represents the expression level of Foxp3 (CD4 positive and CD2 -positive) and the vertical axis represents the number of cells. The solid lines in the panels show control without induced inflammation, the dashed lines show those with induced inflammation (vehicle), and the dotted lines show those with induced inflammation treated with each compound. Compared to the control, in which no inflammation is induced, Foxp3 expression is markedly decreased in the vehicle group, indicating that Treg cell activity is suppressed. Foxp3 expression was slightly increased in the mangiferin 8a group (Fig. 47(a)) and norathyriol group (Fig. 47(b)) compared to the vehicle group. In other words, mangiferin 8a and norathyriol promote Treg cell activation in a mouse model of inflammation.

### < Example 43: Inhibition of cell proliferation in B cells >

The inhibitory effect of the compound on B cell proliferation was examined using mouse B cells.

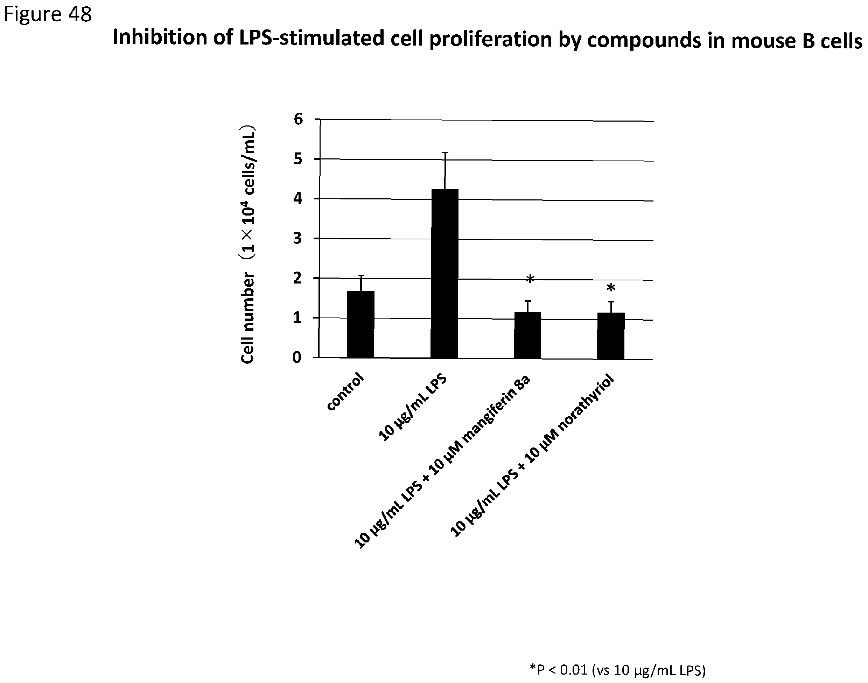

B cells collected from mice were cultured in RPMI 1640 medium for 1 day. Then, 10 µM mangiferin 8a and 10 µM norathyriol were added to B cells, and after 1 day of culture, 10 µg/mL of lipopolysaccharide (LPS) was added to each for 3 days. After 3 days of incubation, the number of viable cells was determined by the trypan blue dye method. The results are shown in Figure 48. Note that LPS was used as an allergic inflammation inducer.

Referring to Figure 48, the horizontal axis indicates treatment with each compound and the vertical axis indicates cell count (1 × 10⁴ cells/mL). Only 0.5% DMSO in PBS, which was used to dissolve the reagent (compound), was added as control. The LPS group is referred to as the LPS group with 10 µg/mL LPS, the mangiferin 8a group is referred to as the mangiferin 8a group with LPS and mangiferin 8a, and the norathyriol group is referred to as the norathyriol group with LPS and norathyriol.

The number of cells in the control group was 1.66 × 10⁴ cells/mL, whereas the LPS group showed 4.25 × 10⁴ cells/mL, indicating that B cells were activated by LPS stimulation and cell proliferation was enhanced. 1.16 × 10⁴ cells/mL and 1.16 × 10⁴ cells/mL in the mangiferin 8a and norathyriol groups, respectively.

As described above, administration of mangiferin 8a and norathyriol markedly inhibited B cell proliferation in LPS stimulation.

### < Example 44: Inhibition of cell proliferation in macrophage >

The inhibitory effect of the compound on macrophage cell proliferation was examined using mouse macrophages.

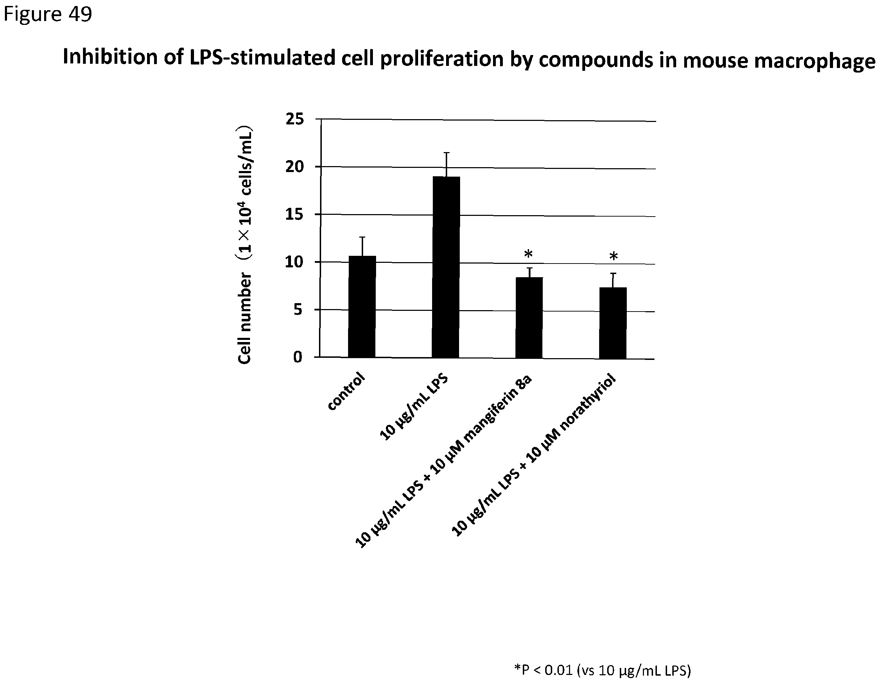

Macrophages collected from mice were cultured in RPMI 1640 medium for 1 day. Then, 10 µM mangiferin 8a and 10 µM norathyriol were added to macrophages, and after 1 day of culture, 10 µg/mL of lipopolysaccharide (LPS) was added to each for 3 days. After 3 days of incubation, the number of viable cells was determined by the trypan blue dye method. The results are shown in Figure 49. Note that LPS was used as an allergic inflammation inducer.

Referring to Figure 49, the horizontal axis indicates treatment with each compound and the vertical axis indicates cell count (1 × 10⁴ cells/mL). Only 0.5% DMSO in PBS, which was used to dissolve the reagent (compound), was added as control. The LPS group is referred to as the LPS group with 10 µg/mL LPS, the mangiferin 8a group is referred to as the mangiferin 8a group with LPS and mangiferin 8a, and the norathyriol group is referred to as the norathyriol group with LPS and norathyriol.

The number of cells in the control group was 10.65 × 10⁴ cells/mL, whereas the LPS group showed 19 × 10⁴ cells/mL, indicating that B cells were activated by LPS stimulation and cell proliferation was enhanced. 8.5 × 10⁴ cells/mL and 7.5 × 10⁴ cells/mL in the mangiferin 8a and norathyriol groups, respectively.

As described above, administration of mangiferin 8a and norathyriol markedly inhibited macrophage cell proliferation in LPS stimulation.

### < Example 45: Inhibition of differentiation into mature B cells >

B cells collected from mice were cultured in RPMI 1640 medium for 1 day. Then, 10 µM mangiferin 8a and 10 µM norathyriol were added to B cell, and after 1 day of culture, 10 µg/mL of LPS was added, followed by 10 days of culture. After 10 days of culture, cells were stained with anti-IgM and anti-IgD antibodies, markers of B cells, and the expression of IgM and IgD was measured using BD LSRFortessa. Note that IgM negative and IgD negative indicates pre-B cells, IgM positive and IgD negative indicates immature B cells, and IgM positive and IgD positive indicates mature B cells.

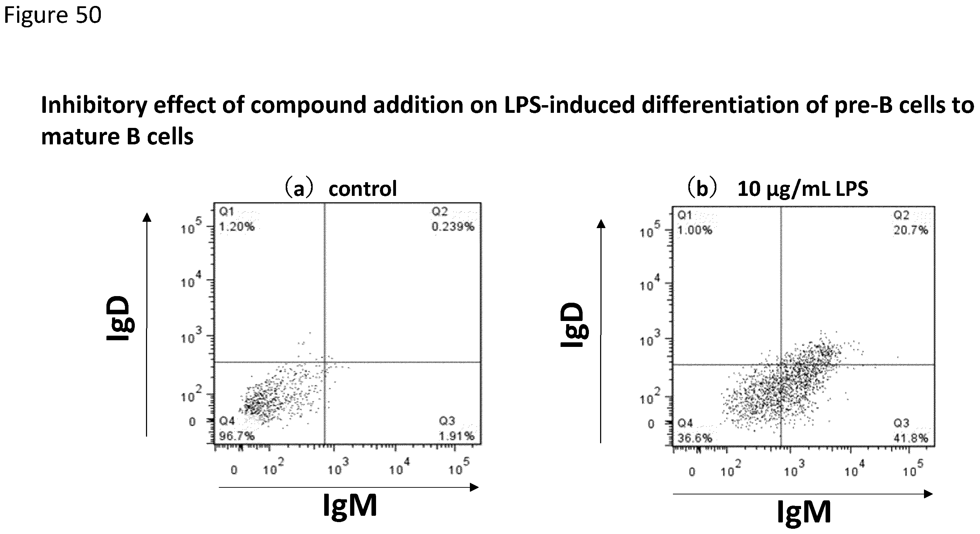

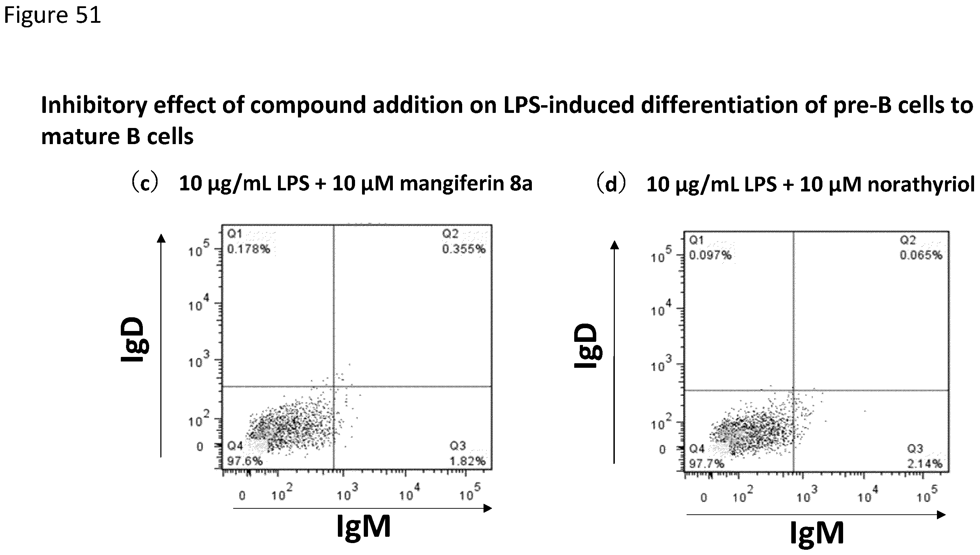

Results are shown in Figure 50 and 51. In all figures, the horizontal axis represents the expression level of IgM and the vertical axis represents the expression level of IgD. In the control without compound and BAFF (Figure 50(a)), 96.7% of the population was IgM negative and IgD negative, 1.91% was IgM positive and IgD negative, and 0.239% was IgM positive and IgD positive. On the other hand, in the LPS-added group (Fig. 50(b)), 36.6% of the population was IgM negative and IgD negative, 41.8% was IgM positive and IgD negative, and 20.7% was IgM positive and IgD positive, indicating that they were differentiated into mature B cells.

In the mangiferin 8a group (Figure 51(c)), 97.6% of the population was IgM negative and IgD negative, 1.82% was IgM positive and IgD negative, 0.355% was IgM positive and IgD positive population, and in the norathyriol group (Figure 51(d)), 97.7% of the population was IgM negative and IgD negative, 2.14% was IgM positive and IgD negative, 0.065% was IgM positive and IgD positive, indicating that the percentage of mature B cells was significantly reduced in all compounds.

In other words, mangiferin 8a and norathyriol inhibited the differentiation of pre-B cells to mature B cells in LPS-induced allergic inflammation.

### < Example 46: Inhibition of differentiation into plasma cells >

B cells collected from mice were cultured in RPMI 1640 medium for 1 day. Then, 10 µM mangiferin 8a and 10 µM norathyriol were added to B cell, and after 1 day of culture, 10 µg/mL of LPS was added, followed by 10 days of culture. After 10 days of culture, cells were stained with anti-CD138 antibody, a marker of plasma cells, and CD138 expression was measured using BD LSRFortessa.

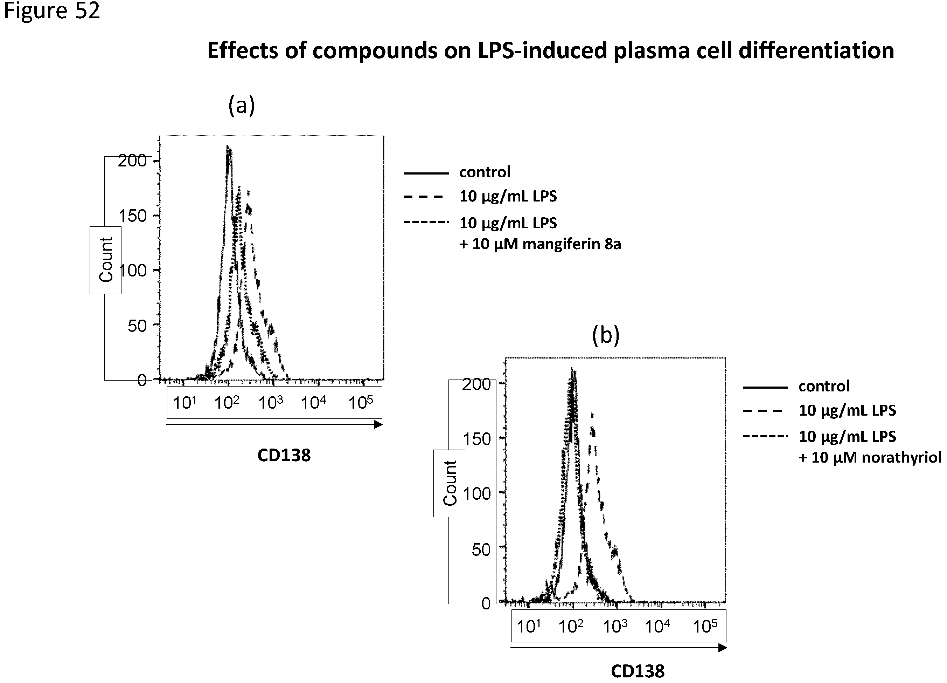

Results are shown in Figures 52. In all figures, the horizontal axis represents the expression level of CD138 and the vertical axis represents the number of cells. The solid line in the panel shows control, which contains neither LPS nor compound, the dotted line shows 10 µg/mL LPS added, and the dashed line shows 10 µg/mL LPS and each compound added. CD138 expression was markedly increased in the LPS-added group compared to the control without the compound and BAFF. Mangiferin 8a group (Fig. 52(a)) and norathyriol group (Fig. 52(b)) showed significantly decreased CD138 expression compared to the LPS-added group, which were almost at the same level as the control group. In other words, mangiferin 8a and norathyriol inhibited differentiation into plasma cells in LPS-induced allergic inflammation.

### < Example 47: Inhibition of macrophage activation >

Monocytes collected from mice were cultured in RPMI 1640 medium for 1 day. Then, 10 µM mangiferin 8a and 10 µM norathyriol were added to monocytes, and after 1 day of culture, 10 µg/mL of LPS was added, followed by 10 days of culture. After 10 days of culture, cells were stained with anti-CD11b and anti-F4/80 antibodies, markers of macrophages, and expression of CD11b and F4/80 was measured using BD LSRFortessa. Note that CD11b positive and F4/80 positive indicate activated macrophages.

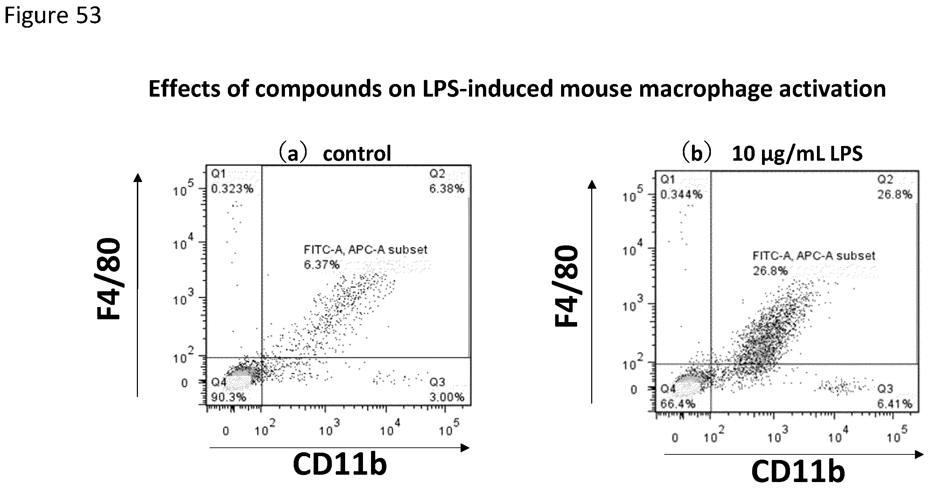

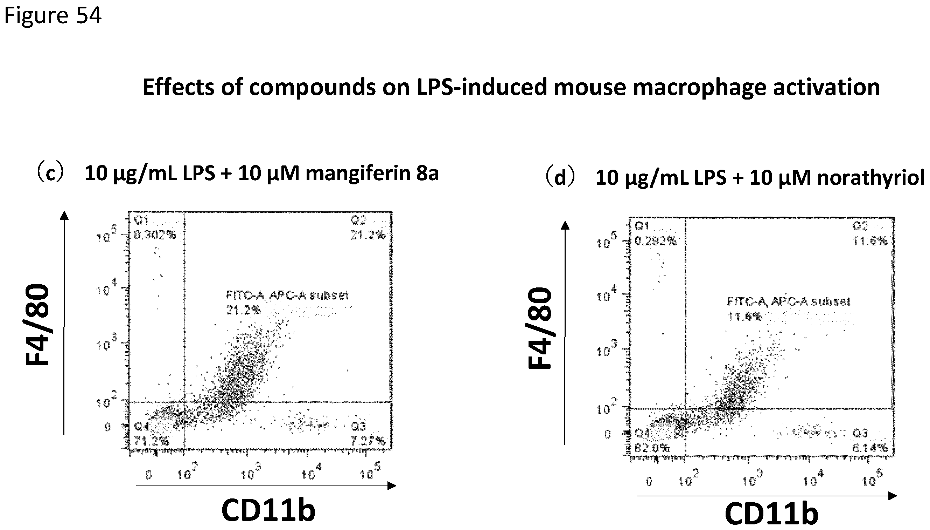

Results are shown in Figures 53 and 54. In all figures, the horizontal axis represents the expression level of CD11b, and the vertical axis represents the expression level of F4/80. In the control (Fig. 53(a)) without compounds (mangiferin 8a or norathyriol) and LPS, the CD11b positive and F4/80 positive population was 6.38%, while in the LPS-added group (Fig. 53(b)), the CD11b positive and F4/80 positive population was 26.8%, indicating an increase in activated macrophages.

The percentage of activated macrophages was significantly reduced in both compounds, with 21.2% of the population being CD11b positive and F4/80 positive in the mangiferin 8a group (Fig. 54(c)) and 11.6% of the population being CD11b positive and F4/80 positive in the norathyriol group (Fig. 54(d)).

In other words, administration of mangiferin 8a and norathyriol inhibited macrophage activation in LPS-induced allergic inflammation.

### < Example 48: Inhibition of M1 macrophage activation >

Monocytes collected from mice were cultured in RPMI 1640 medium for 1 day. Then, 10 µM mangiferin 8a and 10 µM norathyriol were added to Monocytes, and after 1 day of culture, 10 µg/mL of LPS was added, followed by 10 days of culture. After 10 days of culture, cells were stained with anti-CD11b and anti-F4/80 antibodies, markers of macrophages, and anti-CD80 antibody, marker of M1 macrophages, and CD80 (CD11b positive and F4/80 positive) expression was measured using BD LSRFortessa.

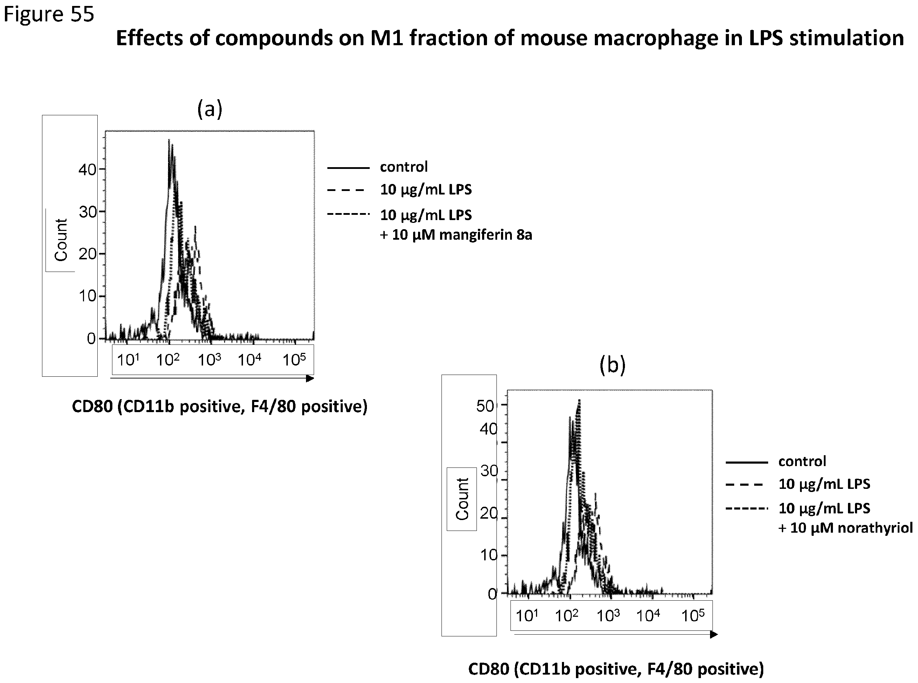

The results are shown in Figure 55. In all figures, the horizontal axis represents the expression level of CD80 and the vertical axis represents the number of cells. The solid line in the panel shows control, which contains neither LPS nor compound, the dotted line shows 10 µg/mL LPS added, and the dashed line shows 10 µg/mL LPS and each compound added. CD80 expression was markedly increased in the LPS-added group compared to the control without compound or LPS. CD80 expression was markedly reduced in the mangiferin 8a (Fig. 55(a)) and norathyriol (Fig. 55(b)) groups compared to the LPS-added group, and were almost at the same level as in the control group. In other words, administration of mangiferin 8a and norathyriol inhibited M1 macrophage activation in LPS-induced allergic inflammation.

### < Example 49: Inhibition of LPS-induced NIK activation by compounds in B cells >

Spleens were harvested from 6-week-old male Balb/c mice and B cells were isolated. After isolation, the cells were seeded in 100 mm² dish and cultured in RPMI 1640 medium for 1 day and used as control. In addition, B cells were seeded into 100 mm² dish, and after 1 day of culture, 10 µg/mL LPS was added and cultured for 1 h. Furthermore, B cells were seeded in 100 mm² dish, and after 1 day of culture, 10 µM mangiferin 8a and 10 µM norathyriol were added to B cells, and after 23 h, 10 µg/mL LPS was added to each, and the cells were incubated for 1 h. All were incubated at 37°C and 5% CO₂.

Proteins were extracted with cell lysate and used as samples. Each sample was transferred to a PVDF membrane after SDSPAGE and assayed with anti-phospho-NIK, anti-NIK, anti-NF-κB p52, anti-NF-κB p65 and anti-Lamin antibodies.

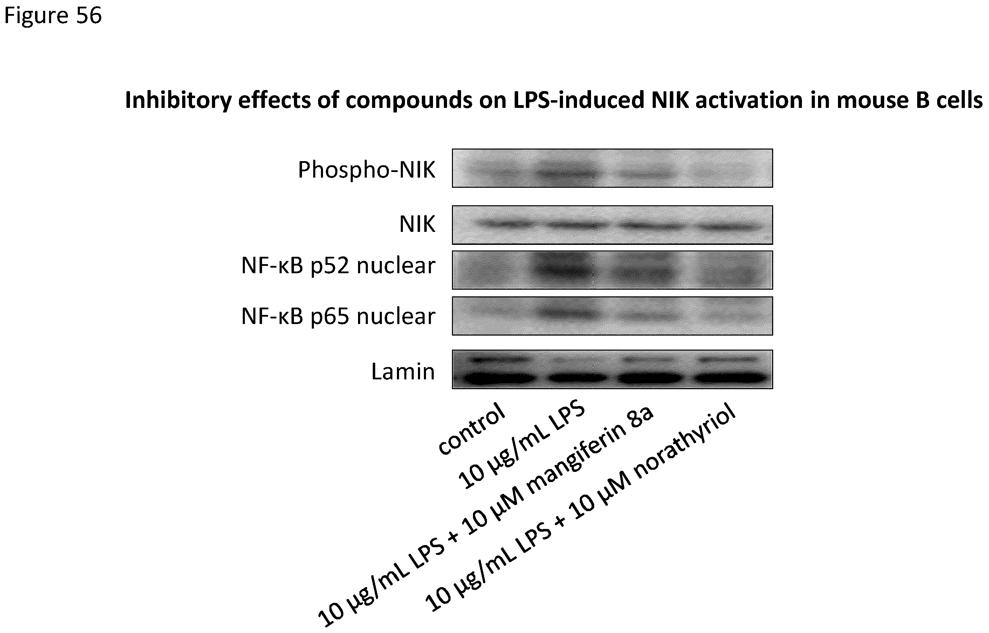

The results of immunoblotting are shown in Figure 56. Control, 10 µg/mL LPS, 10 µg/mL LPS + 10 µM mangiferin 8a and 10 µg/mL LPS + 10 µM norathyriol are shown as reagents (compounds) in the horizontal direction.

The antibody species is indicated in the vertical direction. Specifically, in the case of anti-phospho-NIK antibody (described as "Phospho-NIK"), anti-NIK antibody (described as "NIK"), anti-NF-κB p52 antibody (described as "NF-κB p52 nuclear"), anti-NF-κB p65 antibody (described as "NF-κB p65 nuclear") and anti-Lamin antibody (described as "Lamin").

Photographs of anti-NIK antibody showed no decrease in the density of the NIK band compared to the control. In the case of anti-phospho-NIK antibody, 10 µg/mL LPS was increased compared to control. This indicates that the addition of LPS activates NIK and enhances B cell differentiation and activation. On the other hand, in the case of anti-phospho-NIK antibodies, the immunoblotting results for mangiferin 8a and norathyriol samples were fainter. Therefore, it can be said that mangiferin 8a and norathyriol inhibit NIK phosphorylation.

Next, we refer to anti-NF-κB p52 (described as "NF-κB p52 nuclear"), anti-NF-κB p65 (described as "NF-κB p65 nuclear"), and anti-Lamin (described as "Lamin") antibodies against cellular nuclear material. Lamin was present regardless of all compounds, but nuclear NF-κB p52 was found to be fainter with mangiferin 8a and norathyriol treatments compared to 10 µg/mL LPS. Nuclear NF-κB p65 was also reduced (shaded) for all compounds compared to 10 µg/mL LPS.

Lamin, a fibrous protein that maintains structure and regulates transcription in the cell nucleus, is detected as an internal standard (loading control) for nuclear proteins. Therefore, with the nuclear protein loading control detected in all samples, the addition of each compound indicates that nuclear NF-κB p65 and NF-κB p52 are not present, or if present, very little. In other words, mangiferin 8a and norathyriol are a NIK inhibitor.

### < Example 50: Inhibition of TNFα secretion in B cells >

Mouse B cells were used to examine the inhibition of compounds on TNFα secretion in LPS stimulation.

Mouse B cells were cultured in RPMI 1640 medium for 1 day. Then, 10 µM mangiferin 8a and 10 µM norathyriol were added to B cells, and after 1 day of culture, 10 µg/mL of LPS was added to each, followed by 2 days of culture.

Thus, the samples were divided into "control" with no treatment, "10 µg/mL LPS + 10 µM mangiferin 8a" to which 10 µg/mL LPS and 10 µM mangiferin 8a were added, and "10 µg/mL LPS + 10 µ M norathyriol" to which 10 µg/mL LPS and 10 µM norathyriol were added and only 10 µg/mL LPS was added ("10 µg/L LPS").

After these samples were incubated for 2 days, the culture supernatant was collected and TNFα secretion was measured by enzyme-linked immunosorbent assay (ELISA). The mouse TNFα ELISA kit (R&D) was used for this measurement.

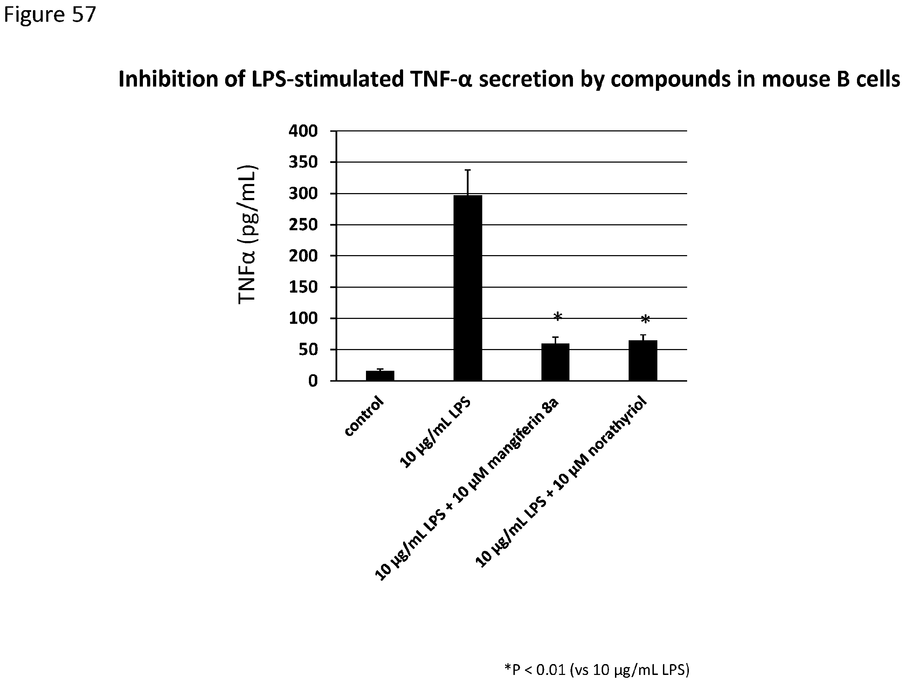

Results are shown in Figure 57. The horizontal axis represents the sample group and the vertical axis represents TNFα secretion. In control, TNFα was 15.13 pg/mL, whereas in 10 µg/mL LPS, TNFα was 296.31 pg/mL, indicating that LPS stimulation activated B cells and enhanced TNFα secretion. 59.31 pg/mL and 64.13 pg/mL in the mangiferin 8a and norathyriol groups, respectively.

As described above, administration of mangiferin 8a and norathyriol markedly inhibited TNFα secretion in LPS stimulation.

### < Example 51: Inhibition of IL-6 secretion in B cells >

Mouse B cells were used to examine the inhibition of compounds on IL-6 secretion in LPS stimulation.

Mouse B cells were cultured in RPMI 1640 medium for 1 day. Then, 10 µM mangiferin 8a and 10 µM norathyriol were added to B cells, and after 1 day of culture, 10 µg/mL of LPS was added to each, followed by 2 days of culture.

Thus, the samples were divided into "control" with no treatment, "10 µg/mL LPS + 10 µM mangiferin 8a" to which 10 µg/mL LPS and 10 µM mangiferin 8a were added, and "10 µg/mL LPS + 10 µ M norathyriol" to which 10 µg/mL LPS and 10 µM norathyriol were added and only 10 µg/mL LPS was added ("10 µg/L LPS").

After these samples were incubated for 2 days, the culture supernatant was collected and IL-6 secretion was measured by enzyme-linked immunosorbent assay (ELISA). The mouse IL-6 ELISA kit (R&D) was used for this measurement.

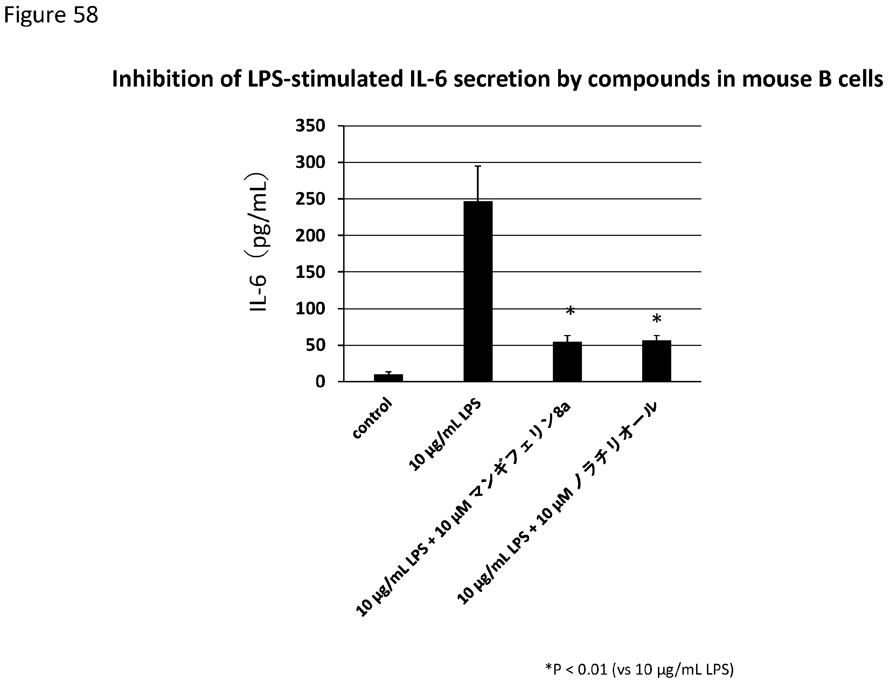

Results are shown in Figure 58. The horizontal axis represents the sample group and the vertical axis represents IL-6 secretion. In control, IL-6 was 9.46 pg/mL, whereas in 10 µg/mL LPS, IL-6 was 246.13 pg/mL, indicating that LPS stimulation activated B cells and enhanced IL-6 secretion. 54.13 pg/mL and 56.13 pg/mL in the mangiferin 8a and norathyriol groups, respectively.

As described above, administration of mangiferin 8a and norathyriol markedly inhibited IL-6 secretion in LPS stimulation.

### < Example 52: Inhibition of TNFα secretion in macrophage >

Mouse macrophages were used to examine the inhibition of compounds on TNFα secretion in LPS stimulation.

Mouse macrophages were cultured in RPMI 1640 medium for 1 day. Then, 10 µM mangiferin 8a and 10 µM norathyriol were added to macrophages, and after 1 day of culture, 10 µg/mL of LPS was added to each, followed by 2 days of culture.

Thus, the samples were divided into "control" with no treatment, "10 µg/mL LPS + 10 µM mangiferin 8a" to which 10 µg/mL LPS and 10 µM mangiferin 8a were added, and "10 µg/mL LPS + 10 µ M norathyriol" to which 10 µg/mL LPS and 10 µM norathyriol were added and only 10 µg/mL LPS was added ("10 µg/L LPS").

After these samples were incubated for 2 days, the culture supernatant was collected and TNFα secretion was measured by enzyme-linked immunosorbent assay (ELISA). The mouse TNFα ELISA kit (R&D) was used for this measurement.

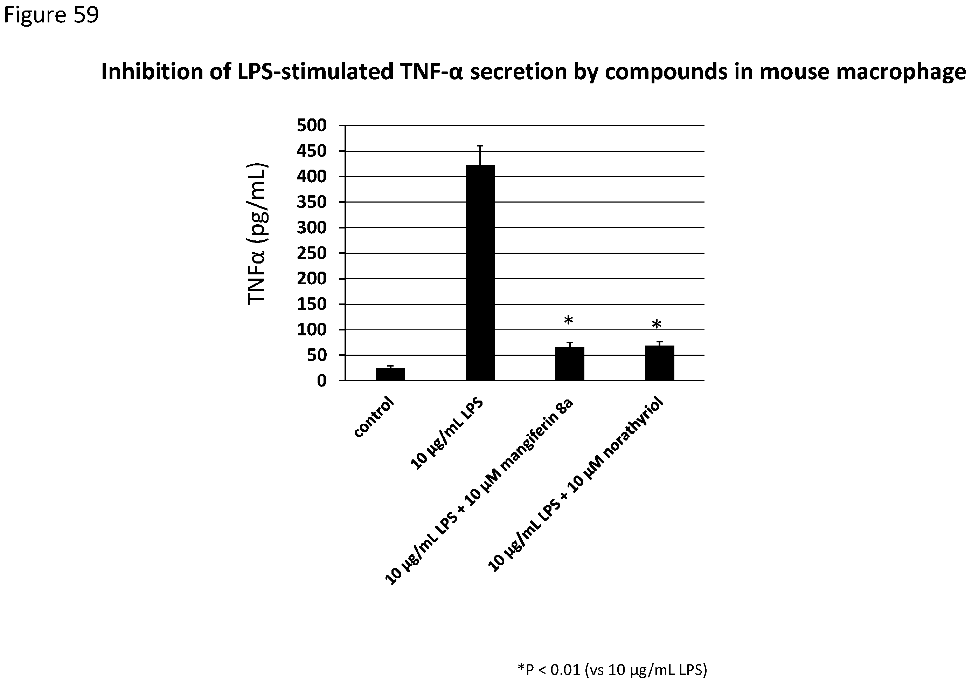

Results are shown in Figure 59. The horizontal axis represents the sample group and the vertical axis represents TNFα secretion. In control, TNFα was 24.11 pg/mL, whereas in 10 µg/mL LPS, TNFα was 421.13 pg/mL, indicating that LPS stimulation activated macrophages and enhanced TNFα secretion. 65.13 pg/mL and 68.13 pg/mL in the mangiferin 8a and norathyriol groups, respectively.

As described above, administration of mangiferin 8a and norathyriol markedly inhibited TNFα secretion in LPS stimulation.

### < Example 53: Inhibition of IL-6 secretion in macrophages >

Mouse macrophages were used to examine the inhibition of compounds on IL-6 secretion in LPS stimulation.

Mouse macrophages were cultured in RPMI 1640 medium for 1 day. Then, 10 µM mangiferin 8a and 10 µM norathyriol were added to macrophages, and after 1 day of culture, 10 µg/mL of LPS was added to each, followed by 2 days of culture. After these samples were incubated for 2 days, the culture supernatant was collected and IL-6 secretion was measured by enzyme-linked immunosorbent assay (ELISA). The mouse IL-6 ELISA kit (R&D) was used for this measurement.

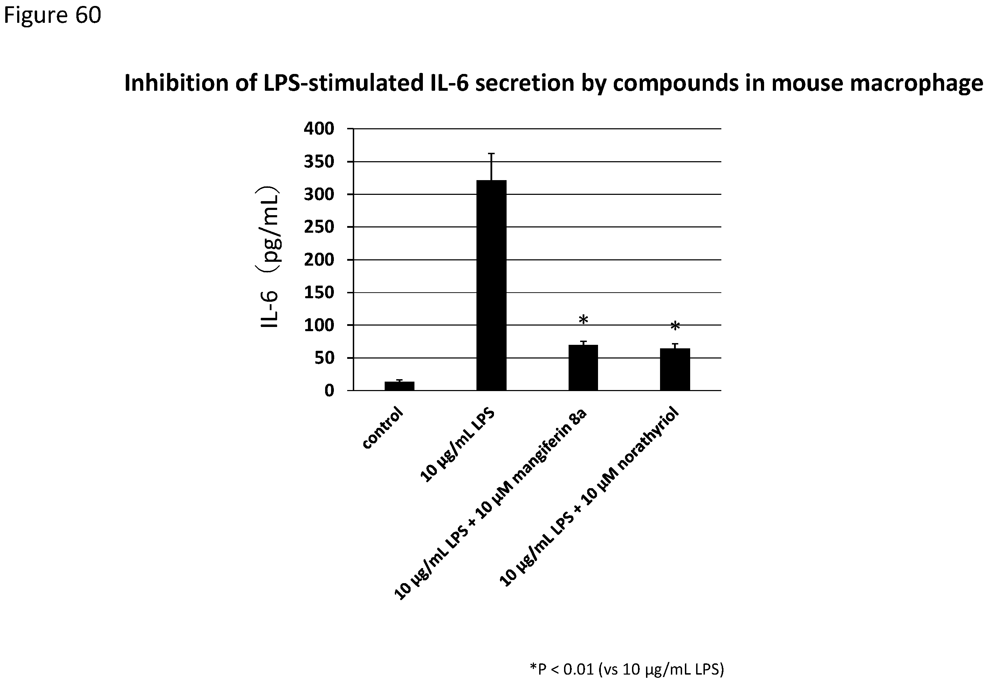

Results are shown in Figure 60. The horizontal axis represents the sample group and the vertical axis represents IL-6 secretion. In control, IL-6 was 13.13 pg/mL, whereas in 10 µg/mL LPS, IL-6 was 321.13 pg/mL, indicating that LPS stimulation activated macrophages and enhanced IL-6 secretion. 69.16 pg/mL and 64.13 pg/mL in the mangiferin 8a and norathyriol groups, respectively.

As described above, administration of mangiferin 8a and norathyriol markedly inhibited IL-6 secretion in LPS stimulation.

p52 and p65 are inflammatory signals, and their translocation into the nucleus is believed to regulate the expression of pro- or anti-inflammatory proteins. Thus, the inhibition of p52 nuclear translocation is thought to reduce inflammation, which is also consistent with the results of the examples shown in Figure 1-Figure 60. In addition, activation of the NIK/NF-κB p52 and NIK/NF-κB p65 pathways by BAFF and others has been associated with B cell differentiation, B cell activation and antibody production, T cell activation, and macrophage activation in allergic diseases. In other words, inhibition of these pathways may prevent B cell differentiation, antibody production, activation of T cell and macrophage, and promote de-differentiation of plasma cells to immature B cells, which are also consistent with the results of the examples shown in Figure 1-Figure 60.

### [Industrial applicability]

The pharmaceutical compositions for improvement of allergic diseases can be useful for allergic diseases caused by activation of NF-κB p52 and NF-κB p65 as selective NIK inhibitors.

## Claims

1. The pharmaceutical composition for the improvement of allergic diseases, containing as active ingredients at least one compound of mangiferin 8a (formula (1)), norathyriol (formula (2)), mangiferin (formula (3)), 1,3,5,6-tetrahydroxyxanthone (formula (4)), xanthohydrol (formula (5)), α-mangostin (formula (6)), and γ-mangostin (formula (7)) having a xanthone structure.

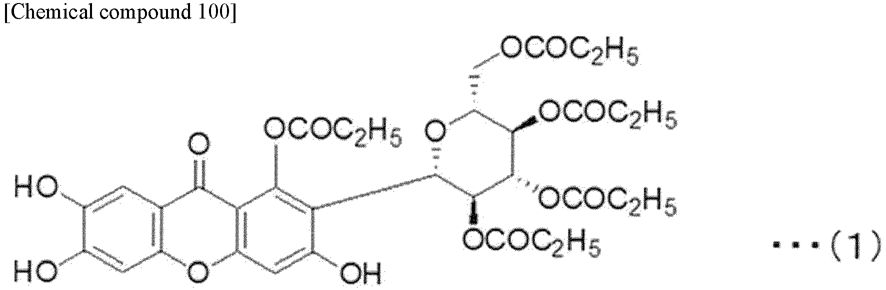

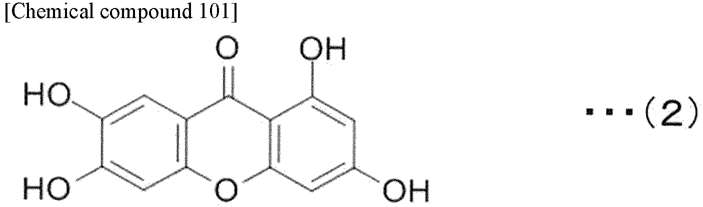

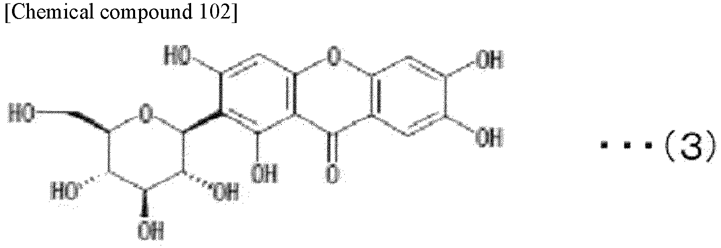

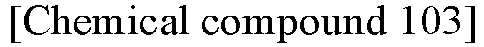

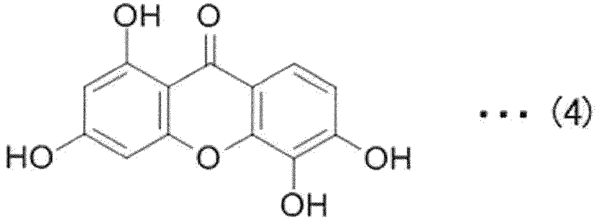

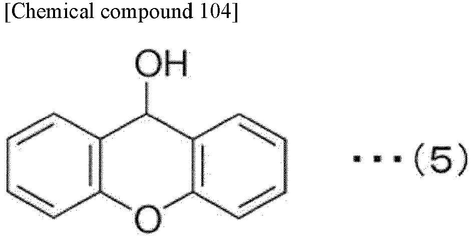

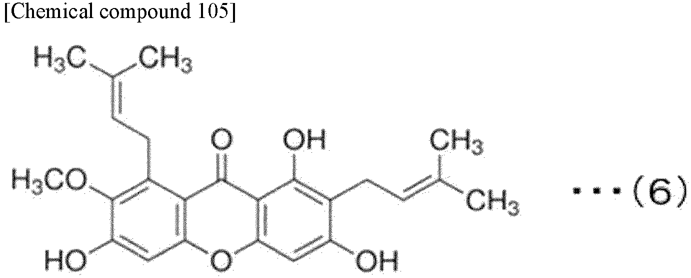

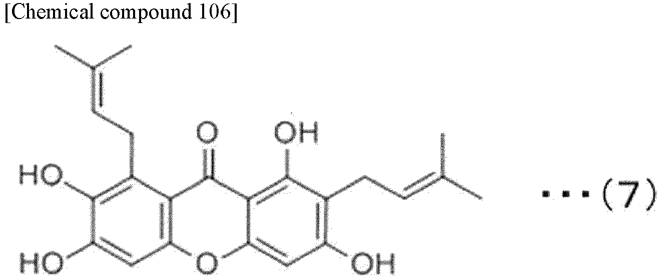

2. The pharmaceutical composition for improvement of allergic disease according to claim 1, wherein
the allergic disease is an allergic disease involving BAFF overexpression.

3. The pharmaceutical composition for improvement of allergic disease according to claim 1, wherein
the allergic disease is an allergic disease involving NF-κB activation.

4. The pharmaceutical composition for amelioration of allergic disease according to any one of claims 1 to 3, wherein the allergic disease is at least one allergic disease of bronchial asthma, atopic dermatitis, pollinosis, allergic rhinitis, food allergy, metal allergy, drug allergy, anaphylaxis, urticaria, allergic conjunctivitis, allergic bronchitis, and contact dermatitis.

5. NIK inhibitors with at least one of the following compounds as active ingredients: mangiferin 8a (formula (1)), norathyriol (formula (2)), mangiferin (formula (3)), 1,3,5,6-tetrahydroxyxanthone (formula (4)), xanthohydrol (formula (5)), α-mangostin (formula (6)), and γ-mangostin (formula (7)) having a xanthone structure.

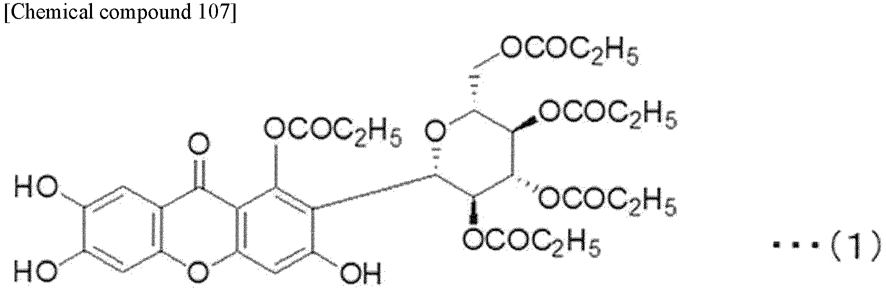

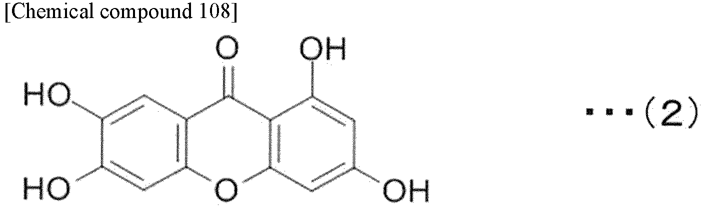

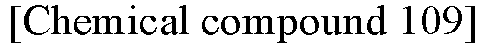

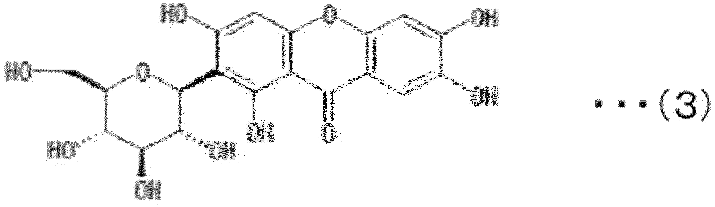

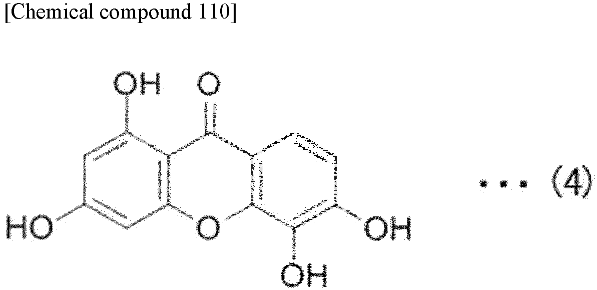

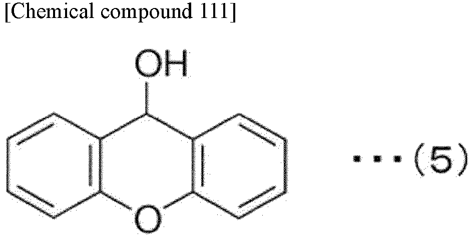

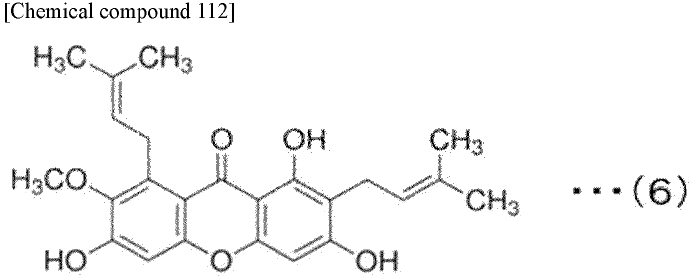

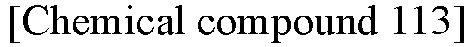

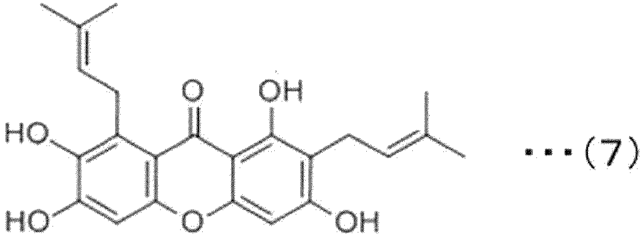

6. The NIK inhibitor described in claim 5, which inhibits plasma cell differentiation.

7. The NIK inhibitor described in claim 5 that de-differentiates plasma cells into B cells.

8. The NIK inhibitor described in claim 5, which inhibits B cell proliferation.

9. The NIK inhibitor described in claim 5, which inhibits proliferation of macrophage.

10. The NIK inhibitor described in claim 5, which inhibits activation of macrophage.

11. The NIK inhibitor as described in claim 5 that inhibits activation of Th1 cells, Th2 cells, and Th17 cells.

12. The NIK inhibitor described in claim 5 that modulates Treg cell activation.

13. The NIK inhibitor as described in claim 5 that inhibits TNFα, IL-6, BAFF, IL-4, IL-5, IL-17 and TSLP production.

14. Processed food products containing at least one of the following compounds: mangiferin 8a (formula (1)), norathyriol (formula (2)), mangiferin (formula (3)), 1,3,5,6-tetrahydroxyxanthone (formula (4)), xanthohydrol (formula (5)), α-mangostin (formula (6)), and γ-mangostin (formula (7)) having a xanthone structure.

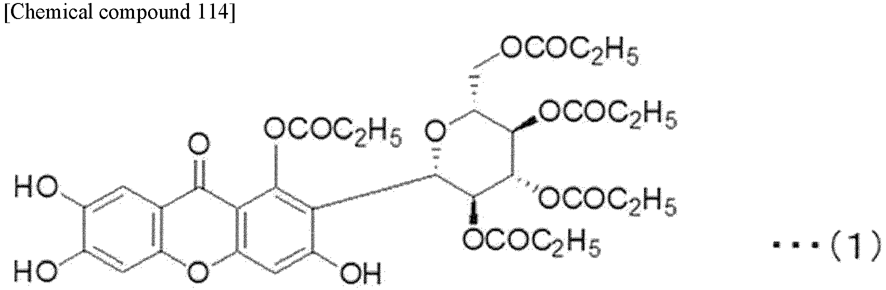

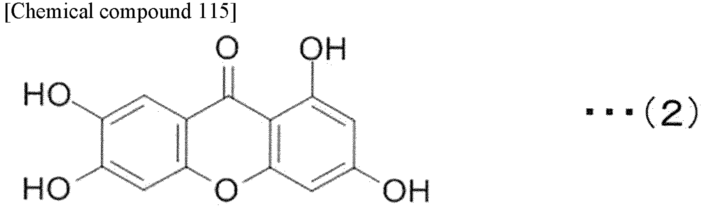

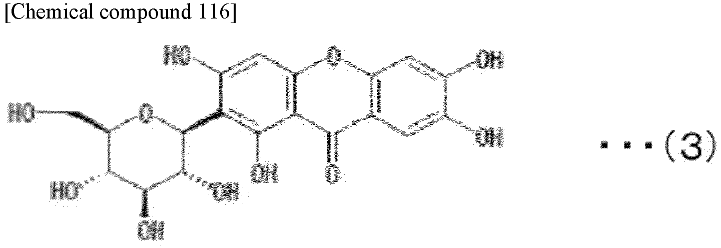

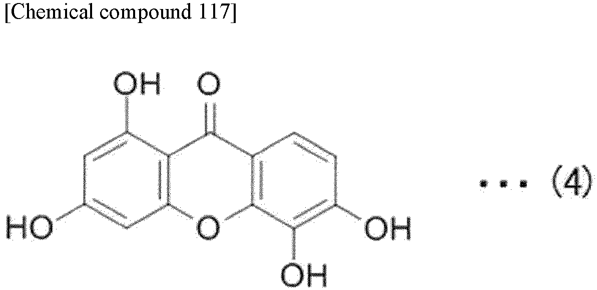

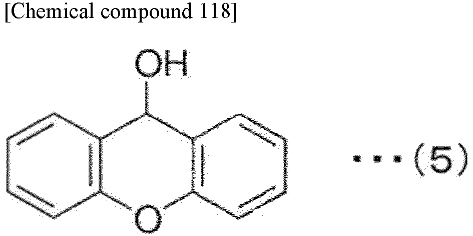

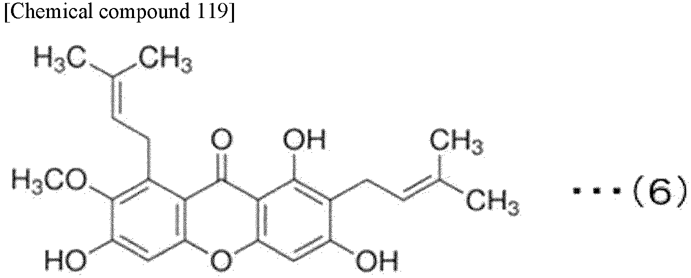

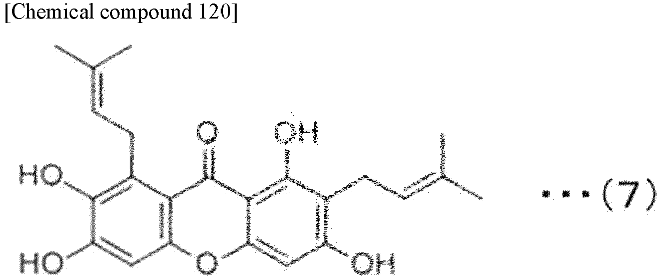

15. The pharmaceutical composition for improvement of allergic diseases according to claim 1, wherein
the active ingredient is norathyriol and the allergic disease is atopic dermatitis.

16. The pharmaceutical composition for improvement of allergic disease according to claim 1, wherein
said active ingredient is norathyriol or mangiferin 8a and the allergic disease is allergic rhinitis.

17. The pharmaceutical composition for improvement of allergic disease according to claim 1, wherein
said active ingredient is norathyriol or mangiferin 8a and the allergic disease is bronchial asthma.

18. The pharmaceutical composition for improvement of allergic disease according to claim 1, wherein
said active ingredient is norathyriol or mangiferin 8a and the allergic disease is food allergy.

19. The pharmaceutical composition for improvement of allergic disease according to claim 1, wherein
said active ingredient is norathyriol or mangiferin 8a and the allergic disease is pollinosis.
